(19) Europäisches Patentamt
European Patent Office
Office européen des brevets

(11) EP 2 013 222 B1

(12) **EUROPEAN PATENT SPECIFICATION**

(45) Date of publication and mention of the grant of the patent:
**13.02.2013 Bulletin 2013/07**

(21) Application number: **07761452.7**

(22) Date of filing: **27.04.2007**

(51) Int Cl.:
***C07H 21/04*** *(2006.01)*

(86) International application number:
**PCT/US2007/067628**

(87) International publication number:
**WO 2007/127919 (08.11.2007 Gazette 2007/45)**

(54) **COMPOSITIONS AND METHODS FOR INHIBITING EXPRESSION OF A GENE FROM THE JC VIRUS**

ZUSAMMENSETZUNGEN UND VERFAHREN ZUR HEMMUNG DER EXPRESSION EINES GENS AUS DEM JC-VIRUS

COMPOSITIONS ET PROCÉDÉS D'INHIBITION DE L'EXPRESSION D'UN GÈNE DU VIRUS JC

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **28.04.2006 US 795765 P**

(43) Date of publication of application:
**14.01.2009 Bulletin 2009/03**

(60) Divisional application:
**12155219.4**

(73) Proprietor: **Alnylam Pharmaceuticals Inc.**
**Cambridge MA 02142 (US)**

(72) Inventors:
- **TAN, Pamela**
  **95326 Kulmbach (DE)**
- **SAH, Dinah**
  **Boston, MA 02114 (US)**
- **BRAMLAGE, Birgit**
  **95326 Kulmbach (DE)**

(74) Representative: **Wachenfeld, Joachim et al**
**Vossius & Partner**
**Siebertstrasse 4**
**81675 München (DE)**

(56) References cited:
**WO-A-2005/112636** **WO-A-2005/113014**
**US-A1- 2004 259 247**

- **ORBA YASUKO ET AL: "SUPPRESSION OF JC VIRUS INFECTION BY SMALL INTERFERING RNA (SIRNA)" INTERNET CITATION, September 2003 (2003-09), XP009082878**
- **RADHAKRISHAN S ET AL: "Intracellular approach for blocking JC virus gene expression by using RNA interference during viral infection" JOURNAL OF VIROLOGY, THE AMERICAN SOCIETY FOR MICROBIOLOGY, US, vol. 78, no. 13, 1 January 2004 (2004-01-01), pages 7264-7269, XP002997069 ISSN: 0022-538X**
- **KUMAR PRITI ET AL: "A single siRNA suppresses fatal encephalitis induced by two different flaviviruses." PLOS MEDICINE APR 2006, vol. 3, no. 4, February 2006 (2006-02), page e96, XP002544652 ISSN: 1549-1676**
- **CHANG L -J ET AL: "Lentiviral siRNAs targeting multiple highly conserved RNA sequences of human immunodeficiency virus type 1" GENE THERAPY, vol. 12, no. 14, July 2005 (2005-07), pages 1133-1144, XP002544653 ISSN: 0969-7128**
- **ORBA Y. ET AL.: 'Inhibition of Virus Production in JC Virus-Infected Cells by Postinfection RNA Interference' J. VIROL. vol. 78, no. 13, July 2004, pages 7270 - 7273, XP002997068**
- **TUSCHL T. ET AL.: 'The siRNA user guide', [Online] 16 April 2003, page 6 Retrieved from the Internet: <URL:http://www.rockefeller.edu/ labheads/tu schl/sima.html>**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 013 222 B1

## Description

### Field of the Invention

This invention relates to double-stranded ribonucleic acid (dsRNA), and its use in mediating RNA interference to inhibit the expression of one of the genes of the JC virus and the use of the dsRNA to treat pathological processes mediated by JC virus infection, such as PML.

### Background of the Invention

Progressive multifocal leukoencephalopathy (PML) is a fatal demyelinating disease of the central nervous system which results from reactivation of the latent polyomavirus JC virus (JCV) and its productive replication in glial cells of the human brain (Berger, J. R. (1995) J. Neurovirol. 1:5-18). Once a rare disease primarily seen in patients with impaired immune systems due to lymphoproliferative and myeloproliferative disorders, PML has become one of the major neurologic problems among patients with AIDS (Cinque, P., (2003). J. Neurovirol. 9(Suppl. 1):88-92).

It has been reported that between 4 and 8% of AIDS patients exhibit signs of PML, and JCV has been detected in the cerebrospinal fluid of affected patients, suggesting that there is active replication of the virus in the brain (Berger, J. R. (1995) J. Neurovirol. 1:5-18, Clifford, D. B., (2001) J. Neurovirol. 4:279). In addition, PML has recently been seen in patients undergoing experimental treatment with Tsybari, an anti VLA4 antobody, in combination with interferon. The histological hallmarks of PML include multifocal demyelinated lesions with enlarged eosinophilic nuclei in oligodendrocytes and enlarged bizarre astrocytes with lobulated hyperchromatic nuclei within white matter tracts of the brain (Cinque, P., (2003). J. Neurovirol. 9(Suppl. 1):88-92), although in some instances atypical features that include a unifocal pattern of demyelination and involvement of the gray matter have been reported (Sweeney, B. J., (1994). J. Neurol. Neurosurg. Psychiatry 57:994-997). Earlier observations from in vitro cell culture studies and an in vivo evaluation of JCV in clinical samples led to early assumptions that oligodendrocytes and astrocytes are the only cells which support productive viral infections (Gordon, J. (1998) Int. J. Mol. Med. 1:647-655). Accordingly, molecular studies have provided evidence for cell-type-specific transcription of the viral early genome in cells derived. from the central nervous system (Raj, G. V., (1995) Virology 10:283-291). However, subsequent studies have shown low, but detectable, levels of JCV gene expression in nonneural cells, including B cells, and noticeably high levels of production of the viral eady protein in several neural and nonneural tumor cells in humans (Gordon, J. (1998) Int. J. Mol. Med. 1:647-653, Khalil, K., 2003. Oncogene 22:5181-5191).

Like the other polyomavinises, JCV is a small DNA virus whose genome can be divided into three regions that encompass the transcription control region; the genes responsible for the expression of the viral early protein, T antigen; and the genes encoding the viral late proteins, VP1, VP2, and VP3. In addition, the late genome is also responsible for production of an auxiliary viral protein, agnoprotein. T-antigen expression is pivotal for initiation of the viral lytic cycle, as this protein stimulates transcription of the late genes and induces the process of viral DNA replication. Recent studies have ascribed an important role for agnoprotein in the transcription and replication of JCV, as inhibition of its production significantly reduced viral gene expression and replication (M. Safak et al., unpublished observations). Furthermore, the agnoprotein dysregulates the cell cycle by altering the expression of several cyclins and their associated kinases (Darbinyan, A., (2002) Oncogene 21:5574-5581).

Thus far, there are no effective therapies for the suppression of JCV replication and the treatment of PML. Cytosine arabinoside (AraC) has been tested for the treatment of PML patients, and the outcome in some instances revealed a remission of JCV-associated demyelination (Aksamit, A. (2001) J. Neurovirol. 7:386-390). Reports from the AIDS Clinical Trial Group Organized Trial 243, however, have suggested that there is no difference in the survival of human immunodeficiency virus type 1 (HIV-1)-infected patients with PML and that of the control population, although in other reports it has been suggested that the failure of AraC in the AIDS Clinical Trial Group trial may have been due to insufficient delivery of the AraC via the intravenous and intrathecal routes (Levy, R. M.,(2001) J. Neurovirol. 7:382-385). Based on in vitro studies showing the ability of inhibitors of topoisomerase to suppress JCV DNA replication, the topoisomerase inhibitor topotecan was used for the treatment of AIDS-PML patients, and the results suggested that topotecan treatment may be associated with a decreased lesion size and prolonged survival (Royal, W., III, (2003) J. Neurovirol. 9:411-419).

Double-stranded RNA molecules (dsRNA) have been shown to block gene expression in a highly conserved regulatory mechanism known as RNA interference (RNAi). WO 99/32619 (Fire et al.) discloses the use of a dsRNA of at least 25 nucleotides in length to inhibit the expression of genes in C. *elegans.* dsRNA has also been shown to degrade target RNA in other organisms, including plants (see, e.g., WO 99/53050, Waterhouse et al.; and WO 99/61631, Heifetz et al.), *Drosophila* (see, e.g., Yang, D., et al., Curr. Biol. (2000) 10:1191-1200), and mammals (see WO 00/44895, Limmer; and DE 101 00 586.5, Kreutzer et al.). This natural mechanism has now become the focus for the development of a new class of pharmaceutical agents for treating disorders that are caused by the aberrant or unwanted regulation of a gene.

Recent reports have indicated that in vitro, RNAi may show some promise in reducing JC virus replication (Radhakrishnan, S. (2004) J. Vir. 78:7264-7269, Orba, Y. (2004) J. Vir. 78:7270-7273). For example, WO 2005/112636 describes small interfering RNAs which are specific for mRNA produced from the JCV agnoprotein and large T antigen genes and inhibit the expression of these and other polyamavirus genes. Furthermore, WO 2005/113014 is intended to provide a medicinal composition for treating JC virus-infected cells by RNA interference using a siRNA directed to a gene encoding VP-1 of JC virus. Orba et al. (2003), Journal of NeuroVirology, 9(Supp. 3):128 relates to the suppression of JC Virus infection by small interfering RNA (siRNA) directed to the genes encoding the agnoprotein, T antigen and VP-1 of JCV. Radhakrishnan et al. (2004); Journal of Virology, 78(13):7264-7269 show the blocking of JCV gene expression, in particular expression of the T antigen and agnoprotein of JCV, by using RNA interference. However, the RNAi agents examined were not designed against all know JC Virus strains and were not selected for stability and other properties need for in vivo therapeutic RNAi agents. Accordingly, despite significant advances in the field of RNAi, there remains a heed for an agent that can selectively and efficiently silence a gene in the JC virus using the cell's own RNAi machinery that has both high biological activity and in vivo stability, and that can effectively inhibit replication of the JC virus for use in treating pathological processes mediated by JC virus infection.

## Summary of the Invention

The present invention is defined by the claims. The invention provides double-stranded ribonucleic acid (dsRNA) as defined in the claims, as well as compositions. The invention also provides compositions and methods for treating pathological conditions and diseases caused by JC viral infection, such as PML. The dsRNA of the invention comprises an RNA strand (the antisense strand) having a region which is less than 30 nucleotides in length, generally 19-24 nucleotides in length, and is substantially complementary to at least part of an mRNA transcript of a gene from the JC Virus.

In one embodiment, the invention provides double-stranded ribonucleic acid (dsRNA) molecules for inhibiting the expression one of the genes of the human JC virus and viral replication. The dsRNA comprises at least two sequences that are complementary to each other. The dsRNA comprises a sense strand comprising a first sequence and an antisense strand comprising a second sequence. The antisense strand comprises a nucleotide sequence which is substantially complementary to at least part of an mRNA encoded by a gene from the JC Virus, and the region of complementarity is less than 30 nucleotides in length, generally 19-24 nucleotides in length. The dsRNA, upon contacting with a cell expressing infected with the JC virus, inhibits the expression of a gene from the JC Virus by at least 40%.

The dsRNA molecules of the invention can be comprised of naturally occurring nucleotides or can be comprised of at least one modified nucleotide, such as a 2'-O-methyl modified nucleotide, a nucleotide comprising a 5'-phosphorothioate group, and a terminal nucleotide linked to a cholesteryl derivative. Alternatively, the modified nucleotide may be chosen from the group of: a 2'-deoxy-2'-fluoro modified nucleotide, a 2'-deoxy-modified nucleotide, a locked nucleotide, an abasic nucleotide, 2'-amino-modified nucleotide, 2'-alkyl-modified nucleotide, morpholino nucleotide, a phosphoramidate, and a non-natural base comprising nucleotide. Generally, such modified sequence will be based on a first sequence of said dsRNA selected from the group consisting of the sense sequences of Tables 1a and b and a second sequence selected from the group consisting of the antisense sequences of Tables 1a and b.

In another embodiment, the invention provides a cell comprising one of the dsRNAs as defined by the claims. The cell is generally a mamnialian cell, such as a human cell.

In another embodiment, the invention provides a pharmaceutical composition for inhibiting the replication of the JC virus in an organism, generally a human subject, comprising one or more of the dsRNAs as defined by the claims and a pharmaceutically acceptable carrier or delivery vehicle.

In another embodiment, the invention provides a method for inhibiting the expression of a gene in the JC Virus in a cell, comprising the following steps:

(a) introducing into the cell a double-stranded ribonucleic acid (dsRNA), wherein the dsRNA comprises at least two sequences that are complementary to each other. The dsRNA comprises a sense strand comprising a first sequence and an antisense strand comprising a second a sequence comprising a region of complementarity which is substantially complementary to at least a part of a mRNA encoding JC virus, and wherein said region of complementarity is less than 30 nucleotides in length and wherein said dsRNA, upon contact with a cell expressing said JC virus, inhibits expression of said JC virus genome, wherein said second sequence is a partial sequence of at least 15 contiguous nucleotides of the sequence of SEQ ID NO: 132, and wherein the dsRNA differs in its ability to inhibit the expression of a gene from the JC Virus in a FACS assay by no more than 30 % inhibition from a dsRNA comprising the full sequence of SEQ ID NO: 132 complementarity which is substantially complementary to at least a part of a mRNA encoded by the JC virus, and wherein the region of complementarity is less than 30 nucleotides in length, generally 19-24 nucleotides in length, and wherein the dsRNA, upon contact with a cell infected with the JC virus, inhibits; and

(b) maintaining the cell produced in step (a) for a time sufficient to obtain degradation of the mRNA transcript of a JC virus gene, thereby inhibiting expression of a gene from the JC Virus in the cell wherein any such method practiced as a method for treatment of the human or animal body by therapy or as a diagnostic method practiced on the human or animal body is excluded.

Also described herein are methods for treating, preventing or managing pathological processes mediated by JC virus infection, e.g. such as PML, comprising administering to a patient in need of such treatment, prevention or management a therapeutically or prophylactically effective amount of one or more of the dsRNAs as defined by the claims .

In another embodiment, the invention provides vectors as defined by the claims for inhibiting the expression of a gene of the JC virus in a cell, comprising a regulatory sequence operably linked to a nucleotide sequence that encodes at least one strand of one of the dsRNA of the invention.

In another embodiment, the invention provides a cell comprising a vector as defined by the claims for inhibiting the expression of a gene of the JC virus in a cell. The vector comprises a regulatory sequence operably linked to a nucleotide sequence that encodes at least one strand of one of the dsRNA of the invention.

## Brief Description of the Figures

No Figures are presented.

## Detailed Description of the Invention

The invention provides double-stranded ribonucleic acid (dsRNA) as defined by the claims, as well as compositions and methods for inhibiting the expression of a gene from the JC Virus in a cell or mammal using the dsRNA. The invention also provides compositions and methods for treating pathological conditions and diseases in a mammal caused by JC virus infection using dsRNA. dsRNA directs the sequence-specific degradation of mRNA through a process known as RNA interference (RNAi).

The dsRNA as defined by the claims comprises an RNA strand (the antisense strand) having a region which is less than 30 nucleotides in length, generally 19-24 nucleotides in length, and is substantially complementary to at least part of an mRNA transcript of a gene from the JC Virus. The use of these dsRNAs enables the targeted degradation of mRNAs of genes that are implicated in replication and or maintenance of JC virus infection and the occurance of PML in a subject infected with the JC virus. Using cell-based and animal assays, the present inventors have demonstrated that very low dosages of these dsRNA can specifically and efficiently mediate RNAi, resulting in significant inhibition of expression of a gene from the JC Virus. Thus, the methods and compositions of the invention comprising these dsRNAs are useful for treating pathological processes mediated by JC viral infection, e.g. cancer, by targeting a gene involved in JC virus relication and/or maintainance in a cell.

The following detailed description discloses how to make and use the dsRNA and compositions containing dsRNA to inhibit the expression of a gene from the JC virus, as well as compositions and methods for treating diseases and disorders caused by the infection with the JC virus, such as PML. The pharmaceutical compositions as defined by the claims comprise a dsRNA having an antisense strand comprising a region of complementarity which is less than 30 nucleotides in length, generally 19-24 nucleotides in length, and is substantially complementary to at least part of an RNA transcript of a gene from the JC Virus, together with a pharmaceutically acceptable carrier.

Accordingly, certain aspects of the invention provide pharmaceutical compositions comprising the dsRNA as defined by the claims together with a pharmaceutically acceptable carrier, use of the compositions to inhibit expression of a gene in a gene from the JC Virus, and use of the pharmaceutical compositions to treat diseases caused by infection with the JC virus.

### I. Definitions

For convenience, the meaning of certain terms and phrases used in the specification, examples, and appended claims, are provided below. If there is an apparent discrepancy between the usage of a term in other parts of this specification and its definition provided in this section, the definition in this section shall prevail.

"G," "C," "A" and "U" each generally stand for a nucleotide that contains guanine, cytosine, adenine, and uracil as a base, respectively. However, it will be understood that the term "ribonucleotide" or "nucleotide" can also refer to a modified nucleotide, as further detailed below, or a surrogate replacement moiety. The skilled person is well aware that guanine, cytosine, adenine, and uracil may be replaced by other moieties without substantially altering the base pairing properties of an oligonucleotide comprising a nucleotide bearing such replacement moiety. For example, without limitation, a nucleotide comprising inosine as its base may base pair with nucleotides containing adenine, cytosine, or uracil. Hence, nucleotides containing uracil, guanine, or adenine may be replaced in the nucleotide sequences of the invention by a nucleotide containing, for example, inosine. Sequences comprising such replacement moieties are embodiments of the invention.

As used herein, "JC virus" refers to the latent polyomavirus JC Virus that has a reference sequence NC_001699. In addition, further accession numbers of various JCVirus sequences are AB038249.1-AB038255.1, AB048545.1 - AB048582.1, AB074575.1 - AB074591.1, AB077855.1 - AB077879.1, AB081005.1 - AB0810301, AB081600.1 - AB081618.1, AB081654.1, AB092578.1 - AB092587.1, AB103387.1, AB103402.1 - AB103423.1, AB104487.1, AB113118.1 - AB113145.1, AB118651.1 - AB118659.1, AB126981.1 - AB127027.1, AB127342.1, AB127344.1, AB127346.1 AB127349.1, AB127352.1 - AB127353.1, AB198940.1 - AB198954.1, AB220939.1 - AB220943.1, AP004349.1 - AF004350.1, AF015526.1 - AF015537.1, AF015684.1, AF030085.1, AF281599.1 - AF281626.1, AF295731.1 - AF295739.1, AF300945.1 - AF300967.1, AF363830.1 - AF363834.1, AF396422.1 - AF396435.1, AY121907.1 - AY121915.1, NC_001699.1, U61771.1, AF363S34.1, AF396422.1 - AF396435.1, AY121907.1 - AY121915.1, NC_001699.1, U61771.1, U73500.1 - U73502.1.

As used herein, "target sequence" refers to a contiguous portion of the nucleotide sequence of an mRNA molecule formed during the transcription of a gene from the JC Virus, including mRNA that is a product of RNA processing of a primary transcription product.

As used herein, the term "strand comprising a sequence" refers to an oligonucleotide comprising a chain of nucleotides that is described by the sequence referred to using the standard nucleotide nomenclature.

As used herein, and unless otherwise indicated, the term "complementary," when used to describe a first nucleotide sequence in relation to a second nucleotide sequence, refers to the ability of an oligonucleotide or polynucleotide comprising the first nucleotide sequence to hybridize and form a duplex structure under certain conditions with an oligonucleotide or polynucleotide comprising the second nucleotide sequence, as will be understood by the skilled person. Such conditions can, for example, be stringent conditions, where stringent conditions may include: 400 mM NaCl, 40 mM PIPES pH 6.4, 1 mM EDTA, 50°C or 70°C for 12-16 hours followed by washing. Other conditions, such as physiologically relevant conditions as may be encountered inside an organism, can apply. The skilled person will be able to determine the set of conditions most appropriate for a test of complementarity of two sequences in accordance with the ultimate application of the hybridized nucleotides.

This includes base-pairing of the oligonucleotide or polynucleotide comprising the first nucleotide sequence to the oligonucleotide or polynucleotide comprising the second nucleotide sequence over the entire length of the first and second nucleotide sequence. Such sequences can be referred to as "fully complementary" with respect to each other herein. However, where a first sequence is referred to as "substantially complementary" with respect, to a second sequence herein, the two sequences can be fully complementary, or they may form one or more, but generally not more than 4, 3 or 2 mismatched base pairs upon hybridization, while retaining the ability to hybridize under the conditions most relevant, to their ultimate application. However, where two oligonucleotides are designed to form, upon hybridization, one or more single stranded overhangs, such overhangs shall not be regarded as mismatches with regard to the determination of complementarity. For example, a dsRNA comprising one oligonudeotide 21 nuclotides in length and another oligonucleotide 23 nucleotides in length, wherein the longer oligonucleotide comprises a sequence or 21 nucleotides that is fully complementary to the shorter oligonucleotide, may yet be referred to as "fully complementary" for the purposes of the invention.

"Complementary" sequences, as used herein, may also include, or be formed entirely from, non-Watson-Crick base pairs and/or base pairs formed from non-natural and modified nucleotides, in as far as the above requirements with respect to their ability to hybridize are fulfilled.

The terms "complementary", "fullly complementary" and "substantially complementary" herein may be used with respect to the base matching between the sense strand and the antisense strand of a dsRNA, or between the antisense strand of a dsRNA and a target sequence, as will be understood from the context of their use.

As used herein, a polynucleotide which is "substantially complementary to at least part of a messenger RNA (mRNA) refers to a polynucleotide which is substantially complementary to a contiguous portion of the mRNA of interest (e.g., encoding JC virus). For example, a polynucleotide is complementary to at least a part of a JC virus mRNA if the sequence is substantially complementary to a non-interrupted portion of a mRNA encoding JC virus.

The term "double-stranded RNA" or "dsRNA", as used herein, refers to a complex of ribonucleic acid molecules, having a duplex structure comprising two anti-parallel and substantially complementary, as defined above, nucleic acid strands,. The two strands forming the duplex structure may be different portions of one larger RNA molecule or they may be separate RNA molecules. Where the two strands are part of one larger molecule, and therefore are connected by an uninterrupted chain of nucleotides between the 3'-end of one strand and the 5'end of the respective other strand forming the duplex structure, the connecting RNA chain is referred to as a "hairpin loop". Where the two strands are connected covalently by means other than an uninterrupted chain of nucleotides between the 3'-end of one strand and the 5'end of the respective other strand forming the duplex structure, the connecting structure is referred to as a "linker". The RNA strands may have the same or a different number of nucleotides. The maximum number of base pairs a the number of nucleotides in the shortest strand of the dsRNA minus any overhangs that are present in the duplex. In addition to the duplex structure, a dsRNA may comprise one or more nucleotide overhangs.

As used herein, a "nucleotide overhang" refers to the unpaired nucleotide or nucleotides that protrude from the duplex structure of a dsRNA when a 3'end of one strand of the dsRNAs extends beyond the 5'-end of the other strand, or vice versa. "Blunt" or "blunt end" means that there are no unpaired nucleotides at that end of the dsRNA, i.e., no nucleotide overhang. A "blunt ended" dsRNA is a dsRNAs that is double-stranded over its entire length, i.e., no nucleotide overhang at either end of the molecule.

The term "antisense strand" refers to the strand of a dsRNA which includes a region that: is substantially complementary to a target sequence. As used herein, the term "region of complementarity" refers to the region on the antisense strand that is substantially complementary to a sequence, for example a target sequence, as defined herein. Where the region of complementanty is not fully complementary to the target sequence, the mismatches are most tolerated in the terminal regions and, if present, are generally in a terminal region or regions, e.g., within 6, 5, 4, 3, or 2 nucleotides of the 5' and/or 3' terminus.

The term "sense strand," as used herein, refers to the strand of a dsRNA that includes a region that is substantially complementary to a region of the antisense strand.

"Introducing into a cell", when referring to a dsRNA, means facilitating uptake or absorption into the cell, as is understood by those skilled in the art. Absorption or uptake of dsRNA can occur through unaided diffusive or active cellular processes, or by auxiliary agents or dsRNA can occur through unaided diffusive or active cellular processes, or by auxiliary y agents or devices. The meaning of this term is not limited to cells in vitro; a dsRNA may also be "introduced into a cell", wherein the cell is part of a living. In such instance, introduction into the cell will include the delivery to the organism. For example, for in vivo delivery, dsRNA can be injected into a tissue site or administered systemically. In vitro introduction into a cell includes methods known in the art such as electroporation and lipofection.

The terms "silence and "inhibit the expression of", in as far as they refer to a gene from the JC Virus, herein refer to the at least partial suppression of the expression of a gene from the JC Virus, as manifested by a reduction of the amount of mRNA transcribed from a gene from the JC Virus which may be isolated from a first cell or group of cells in which a gene from the JC Virus is transcribed and which has or have been treated such that the expression of a gene from the JC Virus is inhibited, as compared to a second cell or group of cells substantially identical to the first cell or group of cells but which has or have not been so treated (control cells). The degree of inhibition is usually expressed in terms of

$$\frac{(\text{mRNA in control cells}) - (\text{mRNA in treated cells})}{(\text{mRNA in control cells})} \bullet 100\%$$

Alternatively, the degree of inhibition may be given in terms of a reduction of a parameter that is functionally linked to JC virus genome transcription, e.g. the amount of protein encoded by a gene from the JC Virus, or the number of cells displaying a certain phenotype, e.g infection with the JC Virus. In principle, JC virus genome silencing may be determined in any cell expressing the target, either constitutively or by genomic engineering, and by any appropriate assay. However, when a reference is needed in order to determine whether a given dsRNA inhibits the expression of a gene from the JC Virus by a certain degree and therefore is encompassed by the instant invention, the assay provided in the Examples below serve as such reference.

For example, in certain instances, expression of a gene from the JC Virus is suppressed by at least about 20%, 23%, 35%, or 50% by administration of the double-stranded oligonucleotide of the invention. In some embodiment, a gene from the JC Virus is suppressed by at least about 60%, 70%, or 80% by administration of the double-stranded oligonudeolide of the invention. In some embodiments, a gene from the JC Virus is suppressed by at least about. 85%, 90%, or 95% by administration of the double-stranded oligonucleotide of the invention.

As used herein in the context or virus expression, the terms "treat", "treatment", and the like, refer to relief from or alleviation of pathological processes mediated by JC virus infection. In the context of the present invention insofar as it relates to any of the other conditions recited herein below (other than pathological processes mediated by JC virus expression), the terms "treat", "tratment", and the like mean to relieve or alleviate at least one symptom associated with such condition, or to slow or reverse the progression of such condition.

As used herein, the phrases "therapeutically effective amount" and "prophylactically effective amount" refer to an amount that provides a therapeutic benefit in the treatment, prevention, or management of pathological processes mediated by JC virus infection or an overt symptom of pathological processes mediated by JC virus expression. The specific amount that is therapeutically effective can be readily determined by ordinary medical practitioner, and may vary depending en factors known in the art, such as, e.g. the type of pathological processes mediated by JC virus infection, the patient's history and age, the stage of pathological processes mediated by JC virus infection, and the administration of other anti-pathological processes mediated by JC virus infection.

As used herein, a "pharmaceutical composition" comprises a pharmacologically effective amount of a dsRNA and a pharmaceutically acceptable carrier. As used herein, "pharmacologically effective amount," "therapeutically effective amount" or simply "effective amount" refers to that amount of an RNA effective to produce the intended pharmacological, therapeutic or preventive result. For example, if a given clinical treatment is considered effective when there is at least a 25% reduction in a measurable parameter associated with a disease or disorder, a therapeutically effective amount of a drug for the treatment of that disease or disorder is the amount necessary to effect at least a 25% reduction in that parameter.

The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture - medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

As used herein, a "transformed cell" is a cell into which a vector has been introduced from which a dsRNA molecule may be expressed.

II. Double-stranded ribonucleic acid (dsRNA)

In one embodiment, the invention provides double-stranded ribonucleic acid (dsRNA) as defined by the claims molecules for inhibiting the expression of a gene from the JC Virus in a cell or mammal, wherein the dsRNA comprises an antisense strand comprising a region of complementarity which is complementary to at least a part of an mRNA formed in the expression of a gene from the JC Virus, and wherein the region of complementarity is less than 30 nucleotides in length, generally 19-24 nucleotides in length, and wherein said dsRNA, upon contact with a cell expressing the gene from the JC virus, inhibits the expression of the JC virus gene by at least 40%.

The dsRNA comprises two RNA strands that are sufficiently complementary to hybridize to form a duplex structure. One strand of the dsRNA (the antisense strand) comprises a region of complementarity that is substantially complementary, and generally fully complementary, to a target sequence, derived from the sequence of an mRNA formed during the expression of a gene from the JC Virus, the other strand (the sense strand) comprises a region which is complementary to the antisense strand, such that the two strands hybridize and form a duplex structure when combined under suitable conditions. Generally, the duplex structure is between 15 and 30, more generally between 18 and 25, yet more generally between 19 and 24, and most generally between 19 and 21 base pairs in length. Similarly, the region of complementarity to the target sequence is between 15 and 30, more generally between 18 and 25, yet more generally between 19 and 24, and most generally between 19 and 21 nucleotides in length. The dsRNA as defined by the claims may further comprise one or more single-stranded nucleotide overhang(s).

The dsRNA can be synthesized by standard methods known in the art as further discussed below, e.g., by use of an automated DNA synthesizer, such as are commercially available from, for example, Biosearch, Applied Biosystems, Inc. In accordance with the invention a gene from the JC Virus is the human JC virus genome.

The skilled person is well aware that dsRNAs comprising a duplex structure of between 20 and 23, but specifically 21, base pairs have been hailed as particularly effective in inducing RNA interference (Elbashir et al., EMBO 2001, 20: 6877-6888). However, others have found that shorter or longer dsRNAs can be effective as well. In the embodiments described above, the dsRNAs as defined by the claims can comprise at least one strand of a length of minimally 21 nt. It can be reasonably expected that shorter dsRNAs comprising one of the sequences in accordance with the claims minus only a few nucleotides on one or both ends may be similarly effective as compared to the dsRNAs described above. Hence, dsRNAs comprising a partial sequence of at least 15, 16, 17, 18, 19, 20, or more contiguous nucleotides from one of the sequences in accordance with the claims and differing in their ability to inhibit the expression of a gene from the JC Virus in a FACS assay as described herein below by not more than 5, 10, 15, 20, 25, or 30 % inhibition from a dsRNA comprising the full sequence, are contemplated by the invention. Further dsRNAs that cleave within the target sequence as defined by the claims can readily be made using the JC virus sequence and the target sequence provided.

In addition, the RNAi agents provided the claims identify a site in the JC virus mRNA that is susceptible to RNAi based cleavage. As such the present invention further includes RNAi agents that target within the sequence targeted by one of the agents of the present invention. As used herein a second RNAi agent is said to target within the sequence of a first RNAi agent if the second RNAi agent cleaves the message anywhere within the mRNA that is complementary to the antisense strand of the first RNAi agent. Such a second agent will generally consist of at least 15 contiguous nucleotides from one of the sequences provided in Tables 1a and b coupled to additional nucleotide sequences taken from the region contiguous to the selected sequence in a gene from the JC Virus. For example, the last 15 nucleotides of SEQ ID NO:1 combined with the next 6 nucleotides from the target JC virus genome produces a single strand agent of 21 nucleotides that is based on one of the sequences provided in Tables 1a and b.

The dsRNA of the invention can contain one or more mismatches to the target sequence. In a preferred embodiment, the dsRNA of the invention contains no more than 3 mismatches. If the antisense strand of the dsRNA contains mismatches to a target sequence, it is preferable that the area of mismatch not be located in the center of the region of complementarity. If the antisense strand of the dsRNA contains mismatches to the target sequence, it is preferable that the mismatch be restricted to 5 nucleotides from either end, for example 5, 4, 3, 2, or 1 nucleotide from either the 5' or 3' end of the region of complementarity. For example, for a 23 nucleotide dsRNA strand which is complementary to a region of a gene from the JC Virus, the dsRNA generally does not contain any mismatch within the central 13 nucleotides. The methods described within the invention can be used to determine whether a dsRNA containing a mismatch to a target sequence is effective in inhibiting the expression of a gene from the JC Virus. Consideration of the efficacy of dsRNAs with mismatches in inhibiting expressing of a gene from the JC Virus is important especially if the particular region of complementary in a gene from the JC Virus is known to have polymorphic sequence variation within the population.

In one embodiment, at least one end of the dsRNA has a single-stranded nucleotide overhang of 1 to 4, generally 1 or 2 nucleotides. dsRNAs having at least one nucleotide overhang have unexpectedly superior inhibitory properties than their blunt-ended counterparts. Moreover, the present inventors have discovered that the presence of only one nucleotide overhang strengthens the interference activity of the dsRNA, without affecting is overall stability. dsRNA having only one overhang has proven particularly stable and effective in vivo, as well has in a variety of cells, cell culture mediums, blood, and serum. Generally, the single-stranded overhang is located at the 3'-terminal end of the antisense strand or, alternatively, at the 3'-teminal end of the sense strand. The dsRNA may also have a blunt end, generally located at the 5'-end of the antisense strand. Such dsRNAs have improved stability and inhibitory activity, thus allowing administration at low dosages. i.e., less than 5 mg/kg body weight of the recipient per day. Generally, the antisense strand of the dsRNA has a nucleotide overhang at the 3'-end, and the 5'-end is blunt. In another embodiment, one or more of the nucleotides in the overhang is replaced with a nucleoside thiophosphate.

In yet another embodiment, the dsRNA is chemically modified to enhance stability. The nucleic acids of the invention may be synthesized and/or modified by methods well established in the art, such as those described in "Current protocols in nucleic acid chemistry", Beaucage, S.L. et al. (Edrs.), John Wiley & Sons. Inc., New York NY, USA, which is hereby incorporated herein by reference. Specific examples of preferred dsRNAs compounds useful in this invention include dsRNAs containing modified backbones or no natural internucleoside images. As defined in this specification, dsRNAs having modified backbones include those that retain a phosphorus atom in the backbone and those that do not have a phosphorus atom in the backbone. For the purposes of this specification, and as sometimes referenced in the art, modified dsRNAs that do not have a phosphorus atom in their internucleoside backbone can also be considered to be oligonucleosides.

Preferred modified dsRNA backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those) having inverted polarity wherein the adjacent pairs of nucleoside units are linked 3'-5' to 5'-3' or 2'-5' to 5'-2'. Various salts, mixed salts and free acid forms are also included.

Representative U.S. patents that teach the preparation of the above phosphorus-containing linkages include, but are not limited to, U.S. Pat. Nos. 3,687,808; 4,469,863; 4,476,301; 5,023,243; 5,177,195; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,316; 5,550,111; 5,563,253; 5,571,799; 5,587,361; and 5,625,050.

Preferred modified dsRNA backbones that do not include a phosphorus atom therein have backbones that are formed by short chain alkyl or cycloalkyl internucleoside linkages, mixed heteroatoms and alkyl or cycloalkyl internucleoside linkages, or ore or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts.

Representative U.S. patents that teach the preparation of the above oligonucleosides include, but are not limited to, U.S. Pat. Nos. 5,034,506; 5,166,315; 5,185,444; 5,214,134; 5,216,141; 5,235,033; 5,64,562; 5,264,564; 5,405,938; 5,434,257; 5,466,677; 5,470,967; 5,489,677; 5,541,307; 5,561,225; 5,596,086; 5,602,240; 5,608,046; 5,610,289; 5,618,704; 5,623,070; 5,663,312; 5,633,360; 5,677,437; and, 5,677,439.

In other preferred dsRNA mimetics, both the sugar and the internucleoside linkage, i.e., the backbone, of the nucleotide units are replaced with novel groups. The base units are maintained for hybridization with an appropriate nucleic acid target compound. One such oligomeric compound, an dsRNA mimetic that has been shown to have excellent hybridization properties, is referred to as a peptide nucleic acid (PNA). In PNA compounds, the sugar backbone of an dsRNA is replaced with an amide containing backbone, in particular an aminoethylglycine backbone. The nucleobases are retained and are bound directly or indirectly to aza nitrogen atoms of the amide portion of the backbone. Representative U.S. patents that teach the preparation of PNA compounds include, but are not limited to, U.S. Pat. Nos. 5,539,082; 5,714,331; and 5,719,262. Further teaching of PNA compounds can be found in Nielsen et al., Science, 1991, 254, 1497-1500.

Most preferred embodiments of the invention are dsRNAs with phosphorothioate backbones and oligonucleosides with heteroatom backbones, and in particular --CH.sub.2--NH-CH.sub.2--, --CH.sub.2--N(CH.sub.3)--O--CH.sub.2--[known as a methylene (methylimino) or MMI backbone], --CH.sub.2--O--N(CH.sub.3)--CH.sub.2--, --CH.sub.2--N(CH.sub.3)-N(CH.sub.3)--CH.sub.2-- and --N(CH.sub.3)--CH.sub.2--CH.sub.2--[wherein the native phosphodiester backbone is represented as --O--P--O--CH.sub.2--] of the above-referenced U.S. Pat. No. 5,489,677, and the amide backbones of the above-referenced U.S. Pat. No. 5,602,240. Also preferred are dsRNAs having morpholino backbone structures of the above-referenced U.S. Pat. No. 5,034,506.

Modified dsRNAs may also contain one or more substituted sugar moieties. Preferred dsRNAs comprise one of the following at the 2'-position: OH; F; O-, S-, or N-alkyl; O-, S-, or N-alkenyl; O-, S- or N-alkylnyl; or O-alkyl-O-alkyl, wherein the alkyl, alkenyl and alkynyl may be substituted or unsubstituted C.sub.1 to C.sub.10 alkyl or C.sub.2 to C.sub.10 alkenyl and alkynyl. Particularly preferred are O[(CH.sub.2).sub.mO].sub.mCH.sub.3,O(CH.sub.2).sub.nOCH.sub.3, O(CH.sub.2).sub.nON[(CH.Sub.2).sub.nCH.su-b.3)].sub.2, where n and m are from 1 to about 10. Other preferred dsRNAs comprise one of the following at the 2' position: C.sub.1 to C.sub.10 lower alkyl, substituted lower alkyl, alkaryl, aralkyl, O-alkaryl or O-aralkyl, SH, SCH.sub.3, OCN, Cl, Br, CN, CF.suh.3, OCF.sub.3, SOCH.sub.3, SO.sub.2CH.sub.3, ONO.sub.2, NO.sub.2, N.suh.3, NH.sub.2, heterocycloalkyl, heterocycloalkyl, aminoalkylamino, polyalkylamino, substituted silyl, an RNA cleaving group, a reporter group, an intercalator, a group for improving the pharmacokinetic properties of an dsRNA, or a group for improving the pharmacodynamic properties of an dsRNA, and other substituents having similar properties. A preferred modification includes 2'-methoxyethoxy (2'-O--CH.sub.2CH.sub.20CH.sub.3, also known as 2'-O-(2-methoxyethyl) or 2'-MOE) (Martin et al., Helv. Chim. Acta, 1995, 78, 486-504) i.e., an alkoxy-alkoxy group. A further preferred modification includes 2'-dimethylaminooxyethoxy, i.e.,. a O(CH.sub.2).sub.2ON(CH,sub.3); sub.2 group, also known as 2'-DMAOE, as described in examples hereinbelow, and 2'-dimethylaminoethoxyethoxy (also known in the art as 2'-O-dimethylaminoethoxyethyl or 2'-DMAEOE), i.e., 2'-O-CH.sub.2--O-CH.sub.2--N(CH.sub.2).sub.2, also described in examples hereinbelow.

Other preferred modifications include 2'-methoxy (2'-OCH.sub.3), 2'-aminopropoxy (2'-OCH.3ub.2CH.sub.2CH.sub.2NH .sub.2) and 2'-fluor-o (2'-F). Similar modifications may also be made at other positions on the dsRNA, particularly the 3' position of the sugar on the 3' terminal nucleotide or in 2'-5' linked dsRNAs and the 5' position of 5' terminal nucleotide, DsRNAs may also have sugar mimetics such as cyclobutyl moieties in place of the pentofuranosyl sugar. Representative U.S. patents that teach the preparation of such modified sugar structures include, but are not limited to, U.S. Pat. Nos. 4,981,957; 5,118,800; 5,319,080; 5,359,044; 5,393,878; 5,446,137; 3,466,786, 5,514,785; 5,519,134; 5,567,811; 5,576,412; 5,591,722; 5,597,909; 5,610,300; 5,627,053; 5,639,873; 5,646,265; 5,658,873; 5,670,633; and 5,700,920, certain of which are commonly owned with the instant application.

dsRNAs may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl anal other 8-substituted adenines and guanines, 5-hallo, particularly 5-bromo, 5-trifluoromethyl and other 5-substituted uracils and cytosines, 7-methylguanine and 7-methyladenine, 8-azaguanine and 8-azaadenine, 7-deazaguanine and 7-daazaadenine and 3-deazaguanine and 3-deazaadenine. Further nucleobases include those disclosed in U.S. Pat. No. 3,687,808, those disclosed in The Concise Encyclopedia Of Polymer Science And Engineering, pages 858-859, Kroschwitz, J. L, ed. John Wiley & Sons, 1990, these disclosed by Englisch et al., Angewandte Chemie, International Edition, 1991, 30, 613, and those disclosed by Sanghvi, Y S., Chapter 15, DsRNA Research and Applications, pages 289-302, Crooke, S. T. and Lebleu, B., Ed., CRC Press, 1993. Certain of these nucleobases are particularly useful for increasing the binding affinity of the oligomeric compounds of the invention. These include 5-substituted pyrimidines, 6-azapyrimidines and N-2, N-6 and 0-6 substituted purines, including 2-aminopropyladenine, 5-propynyluracil and 5-propynylcytosine. 5-methylcytosine substitutions have been shown to increase nucleic acid duplex stability by 0.6-1.2.degree. C. (Sanghvi, Y. S., Crooke, S. T. and Lebleu, B., Eds., DsRNA Research and Applications, CRC Press, Boca Raton, 1993, pp. 276-278) and are presently preferred base substitutions, even more particularly when combined with 2'-O-methoxyethyl sugar modifications.

Representative U.S. patents that teach the preparation of certain of the above noted modified nucleobases as well as other modified nucleobases include, but are not limited to, the above noted U.S. Pat. No. 3,687,808, as well as U.S. Pat. Nos. 4,845,205; 5,130,30; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941, each of which is herein incorporated by reference, and U.S. Pat. No. 5,750,692.

Another modification of the dsRNAs of the invention involves chemically linking to the dsRNA one or more moieties or conjugates which enhance the activity, cellular distribution or cellular uptake of the dsRNA. Such moieties include but are not limited to lipid moieties such as a cholesterol moiety (Letsinger et al., Proc. Natl. Acid. Sci. USA, 199, 86, 6553-6556), cholic acid (Manoharan et al., Biorg. Med. Chem. Let., 1994 4 1053-1060), a thioether, e.g., beryl-S-tritylthiol (Manoharan et al., Ann. N.Y. Acad. Sci., 1992, 660, 306-309; Manoharan et al., Biorg. Med. Chem. Let., 1993, 3, 2765-2770), a thiocholesterol (Oberhauser et al., Nucl. Acids Res., 1992, 20, 533-538), an aliphatic chain, e.g., dodecandiol or undecyl residues (Saison-Behmoaras et al., EMBO J, 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-Hphosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyloxycholesterol moiety (Crooke et al., J. Pharmacol. Exp. Ther., 1996, 277, 923-937).

Representative U.S. patents that teach the preparation of such dsRNA conjugates include, but are not limited to, U.S. Pat. Nos. 4,828,979; 4,948,882; 5,218,105; 5,525,465; 5,541,313; 5,545,730; 5,552,538; 5,578,717, 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

It is not necessary for all positions in a given compound to be uniformly modified, and in fact more than one of the aforementioned modifications may be incorporated in a single compound or even at a single nucleoside within an dsRNA. The present invention also includes dsRNA compounds which are chimeric compounds. "Chimeric" dsRNA compounds or "chimeras," in the context of this invention, are dsRNA compounds, particularly dsRNAs, which contain two or more chemically distinct regions, each made up of at least one monomer unit, i.e., a nucleotide in the case of an dsRNA compound. These dsRNAs typically contain at least one region wherein the dsRNA is modified so as to confer upon the dsRNA increased resistance to nuclease degradation, increased cellular uptake, and/or increased binding affinity for the target nucleic acid. An additional region of the dsRNA may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of dsRNA inhibition of gene expression. Consequently, comparable results can often be obtained with shorter dsRNAs when chimeric dsRNAs are used, compared to phosphorothioate deoxydsRNAs hybridizing to the same target region. Cleavage of the RNA target can be routinely detected by gel electrophoresis and, if necessary, associated nucleic acid hybridization techniques known in the art.

In certain instances, the dsRNA may be modified by a non-ligand group. A number of non-ligand molecules have been conjugated to dsRNAs in order to enhance the activity, cellular distribution or cellular uptake of the dsRNA, and procedures for performing such conjugations are available in the scientific literature. Such non-ligand moieties have included lipid moieties, such as cholesterol (Letsinger et al., Proc. Natl. Acad. Sci. USA, 1989, 86:6553), cholic acid (Manoharan et al., Bioorg. Med. Chem. Lett., 1994, 4:1053), a thioether, e.g., hexyl-S-tritylthiol (Manoharan et al., Ann. N.Y. Acad. Sci., 1992, 660:306; Manoharan et al., Biorg. Med. Chem. Let., 1993, 3:2765), a thiocholesterol (Oberhauser et al., Nucl. Acids Res., 1992, 20:533, an aliphatic chain, e.g., dodecandiol or undecyl residues (Saison-Behmoaras et al., EMBO J., 1991, 10: 111: Kabanov et al., FEBS Lett., 1990, 259:327; Svinarchuk et al., Biochimie, 1993, 75:49), a phospholipid, e.g., di-hexadecyl-rac-glycerol or triethylammonium 1,2-di-O-hexadecyl-rac-glycero-3-H-phosphonate (Manoharan et al., Tetrahedron Lett., 1995, 36:3651; Shea et al., Nucl. Acids Res., 1990, 18:3777), a polyamine or a polyethylene glycol chain (Manoharan et al., Nucleosides & Nucleotides, 1995, 14:969), or adamantane acetic acid (Manoharam et al., Tetrahedron Lett., 1995, 36:3651), a palmityl moiety (Mishra et al., Biochim. Biophys. Acta, 1995, 1264:229), or an octadecylamine or hexylamino-carbonyl-oxycholesterol moiety (Crooke et al., J. Pharmacol. Exp. Ther., 1996, 277:923). Representative United States patents that teach the preparation of such dsRNA conjugates have been listed above. Typical conjugation protocols involve the synthesis of dsRNAs bearing an aminolinker at one or more positions of the sequence. The amino group is then reacted with the molecule being conjugated using appropriate coupling or activating reagents. The conjugation reaction may be performed either with the dsRNA still bound to the solid support or following cleavage of the dsRNA in solution phase. Purification of the dsRNA conjugate by HPLC typically affords the pure conjugate.

Vector encoded RNAi agents

The dsRNA of the invention can also be expressed from recombinant viral vectors intracellularly in vivo. The recombinant viral vectors of the invention comprise sequences encoding the dsRNA of the invention and any suitable promoter for expressing the dsRNA sequences. Suitable promoters include, for example, the U6 or H1 RNA pol III promoter sequences and the cytomegalovirus promoter. Selection of other suitable promoters is within the skill in the art. The recombinant viral vectors of the invention can also comprise inducible or regulatable promoters for expression of the dsRNA in a particular tissue or in a particular intracellular environment. The use of recombinant viral vectors to deliver dsRNA of the invention to cells in vivo is discussed in more detail below.

dsRNA as defined by the claims can be expressed from a recombinant viral vector either as two separate, complementary RNA molecules, or as a single RNA molecule with two complementary regions.

Any viral vector capable of accepting the coding sequences for the dsRNA molecule(s) to be expressed can be used, for example vectors derived from adenovirus (AV); adeno-associated virus (AAV); retroviruses (e.g, lentiviruses (LV), Rhabdoviruses, murine leukemia virus); herpes virus, and the like. The tropism of viral vectors can be modified by pseudotyping the vectors with envelope proteins or other surface antigens from other viruses, or by substituting different viral capsid proteins, as appropriate.

For example, lentiviral vectors of the invention can be pseudotyped with surface proteins from vesicular stomatitis virus (VSV), rabies, Ebola, Mokola, and the like. AAV vectors of the invention can be made to target different cells by engineering the vectors to express different capsid protein serotypes. For example, an AAV vector expressing a serotype 2 capsid on a serotype 2 genome is called AAV 2/2. This serotype 2 capsid gene in the AAV 2/2 vector can be replaced by a serotype 5 capsid gene to produce an AAV 2/5 vector. Techniques for constructing AAV vectors which express different capsid protein serotypes are within the skill in the art; see, e.g., Rabinowitz J E et al. (2002), J Virol 76:791-801.

Selection of recombinant viral vectors suitable for use in the invention, methods for inserting nucleic acid sequences for expressing the dsRNA into the vector, and methods of delivering the viral vector to the cells of interest are within the skill in the art. See, for example, Dornburg R (1995), Gene Therap. 2: 301-310; Eglitis M A (1988), Biotechniques 6: 608-614; Miller A D (1990), Hum Gene Therap. 1: 5-14; Anderson W F (1998), Nature 392: 25-30; and Rubinson D A et al., Nat. Genet. 33: 401-406.

Preferred viral vectors are those derived from AV and AAV. In a particularly preferred embodiment, the dsRNA of the invention is expressed as two separate, complementary single-stranded RNA molecules from a recombinant AAV vector comprising, for example, either the U6 or H1 RNA promoters, or the cytomegalovirus (CMV) promoter.

A suitable AV vector for expressing the dsRNA of the invention, a method for constructing the recombinant AV vector, and a method for delivering the vector into target cells, are described in Xia H et al. (2002), Nat. Biotech. 20: 1006-1010.

Suitable AAV vectors for expressing the dsRNA of the invention, methods for constructing the recombinant AV vector, and methods for delivering the vectors into target cells are described in Samulski R et al. (1987), J. Virol. 61: 3096-3101; Fisher K J et al. (1996), J. Virol, 70: 520-532; Samulski R et al. (1989), J. Virol. 63: 3822-3826; U.S. Pat. No. 5,252,479; U.S. Pat. No. 5,139,941; International Patent Application No. WO 94/13788; and International Patent Application No. WO 93/24641.

III. Pharmaceutical compositions comprising dsRNA

In one embodiment the invention provides pharmaceutical compositions comprising a dsRNA, as defined by the claims and a pharmaceutically acceptable carrier. The pharmaceutical composition comprising the dsRNA is useful for treating a disease or disorder associated with the expression or activity of a gene from the JC Virus and/or viral infection, such as PML. Such pharmaceutical compositions are formulated based on the mode of delivery. One example is compositions that are formulated for systemic administration via parenteral delivery.

The pharmaceutical compositions of the invention are administered in dosages sufficient to inhibit expression of a gene from the JC Virus. The present inventors have found that, because of their improved efficiency, compositions comprising the dsRNA of the invention can be administered at surprisingly low dosages. A maximum dosage of 5 mg dsRNA per kilogram body weight of recipient per day is sufficient to inhibit or completely suppress expression of a gene from the JC Virus.

In general, a suitable dose of dsRNA will be in the range of 0.01 to 5.0 milligrams per kilogram body weight of the recipient per day, generally in the range of 1 microgram to 1 mg per kilogram body weight per day. The pharmaceutical composition may be administered once daily, or the dsRNA may be administered as two, three, or more sub-doses at appropriate intervals throughout the day or even using continuous infusion or delivery through a controlled release formulation. In that case, the dsRNA contained in each sub-dose must be correspondingly smaller in order to achieve the total daily dosage. The dosage unit can also be compounded for delivery over several days, e.g., using a conventional sustained release formulation which provides sustained release of the dsRNA over a several day period. In this embodiment, the dosage unit contains a corresponding multiple of the daily dose.

The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments. Estimates of effective dosages and in vivo half-lives for the individual dsRNAs encompassed by the invention can be made using conventional methodologies or on the basis of in vivo testing using an appropriate animal model, as described elsewhere herein.

Advances in mouse genetics have generated a number of mouse models for the study of various human diseases, such as pathological processes mediated by JC virus expression. Such models are used for in vivo testing of dsRNA, as well as for determining a therapeutically effective dose.

The present invention also includes pharmaceutical compositions and formulations which include the dsRNA compounds as defined by the claims. The pharmaceutical compositions as defined by the claims may be administered in a number of ways depending upon whether local or systemic treatment is desired and upon the area to be treated. Administration may be topical, pulmonary, e.g., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, e.g., intrathecal or intraventricular, administration.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable.

Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients.

Pharmaceutical compositions of the present invention include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations as defined by the claims which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

Liposomes

There are many organized surfactant structures besides microemulsions that have been studied and used for the formulation of drugs. These include monolayers, micelles, bilayers and vesicles. Vesicles, such as liposomes, have attracted great interest because of their specificity and the duration of action they offer from the standpoint of drug delivery. As used in the present invention, the term "liposome" means a vesicle composed of amphiphilic lipids arranged in a spherical bilayer or bilayers.

Liposomes are unilamellar or multilamellar vesicles which have a membrane formed from a lipophilic material and an aqueous interior. The aqueous portion contains the composition to be delivered. Cationic liposomes possess the advantage of being able to fuse to the cell wall. Non-cationic liposomes, although not able to fuse as efficiently with the cell wall, are taken up by macrophages in vivo.

In order to cross intact mammalian skin, lipid vesicles must pass through a series of fine pores, each with a diameter less than 50 nm, under the influence of a suitable transdermal gradient. Therefore, it is desirable to use a liposome which is highly deformable and able to pass through such fine pores.

Further advantages of liposomes include; liposomes obtained from natural phospholipids are biocompatible and biodegradable; liposomes can incorporate a wide range of water and lipid soluble drugs; liposomes can protect encapsulated drugs in their internal compartments from metabolism and degradation (Rosoff, in Pharmaceutical Dosage Forms, Lieberman, Rieger and Banker (Eds.), 1988, Marcel Dekker, Inc., New York. N.Y., volume 1, p. 245), important considerations in the preparation of liposome formulations are the lipid surface charge, vesicle size and the aqueous volume of the liposomes.

Liposomes are useful for the transfer and delivery of active ingredients to the site of action. Because the liposomal membrane is structurally similar to biological membranes, when liposomes are applied to a tissue, the liposomes start to merge with the cellular membranes and as the merging of the liposome and cell progresses, the liposomal contents are emptied into the cell where the active agent may act.

Liposomal formulations have been the focus of extensive investigation as the mode of delivery for many drugs. There is growing evidence that for topical administration, liposomes present several advantages over other formulations.

Such advantages include reduced side-effects related to high systemic absorption of the administered drug, increased accumulation of the administered drug at the desired target, and the ability to administer a wide variety of drugs, both hydrophilic and hydrophobic, into the skin.

Several reports have detailed the ability of liposomes to deliver agents including high-molecular weight DNA into the skin. Compounds including analgesics, antibodies, hormones and high-molecular weight DNAs have been administered to the skin. The majority of applications resulted in the targeting of the upper epidermis

Liposomes fall into two broad classes. Cationic liposomes are positively charged liposomes which interact with the negatively charged DNA molecules to form a stable complex. The positively charged DNA/liposome complex binds to the negatively charged cell surface and is internalized in an endosome. Due to the acidic pH within the endosome, the liposomes are ruptured, releasing their contents into the cell cytoplasm (Wang et al., Biochem. Biophys. Res. Comman., 1987, 147, 980-985).

Liposomes which are pH-sensitive or negatively-charged, entrap DNA rather than complex with it. Since both the DNA and the lipid are similarly charged, repulsion rather than complex formation occurs. Nevertheless, some DNA is entrapped within the aqueous interior of these liposomes. pH-sensitive liposomes have been used to deliver DNA encoding the thymidine kinase gene to cell monolayers in culture. Expression of the exogenous gene was detected in the target cells (Zhou et al., Journal of Controlled Release, 1992, 19, 269-274).

One major type of liposomal composition includes phospholipids other than naturally-derived phosphatidylcholine. Neutral liposome compositions, for example, can be formed from dimyristoyl phosphatidylcholine (DMPC) or dipalmitoyl phosphatidylcholine (DPPC). Anionic liposome compositions generally are formed from dimyristoyl phosphatidylglycerol, while anionic fusogenic liposomes are formed primarily from dioleoyl phosphatidylethanolamine (DOPE). Another type of liposomal composition is formed from phosphatidylcholine (PC) such as, for example, soybean PC, and egg PC. Another type is formed from mixtures of phospholipid and/or phosphatidylcholine and/or cholesterol.

Several studies have assessed the topical delivery of liposomal drug formulations to the skin. Application of liposomes containing interferon to guinea pig skin resulted in a reduction of skin herpes sores white delivery of interferon via other means (e.g. as a solution or as an emulsion) were ineffective (Weiner et al., Journal of Drug Targeting, 1992, 2, 405-410). Further, an additional study tested the efficacy of interferon administered was part of a liposomal formulation to the administration of interferon using an aqueous system, and concluded that the liposomal formulation was superior to aqueous administration (du Plessis et al., Antiviral Research, 1992, 18, 259-265).

Non-ionic liposomal systems have also been examined to determine their utility in the delivery of drugs to the skin, in particular systems comprising non-ionic surfactant and cholesterol. Non-ionic liposomal formulations comprising Novasome.TM. I (glyceryl dilaurate/cholesterol/po- lyoxyetylene-10-stearyl ether) and Novasome.TM. II (glyceryl distearate/cholesterol/polyoxyethylene-10-stearyl ether) were used to deliver cyclosporin-A into the dermis of mouse sikin. Results indicated that such non-ionic liposomal systems were effective in facilitating the deposition of cyclosporin-A into different layers of the skin (Hu et al. S.T.P.Pharma. Sci., 1994, 4, 6, 466).

Liposomes also include "sterically stabilized" liposomes, a a term which, as used herein, refers to liposomes comprising one or more specialized lipids that, when incorporated into liposomes, result in enhanced circulation lifetimes relative to liposomes lacking such specialized lipids. Examples of sterically stabilized liposomes are those in which part of the vesicle-forming lipid portion of the liposome (A) comprises one or more glycolipids, such as monosialoganglioside G.sub.MI, or (B) is derivatized with one or more hydrophilic polymers, such as a polyethylene glycol (PEG) moiety. While not wishing to be bound by any particular theory, it is thought in the art that, at least for sterically stabilized liposomes containing gangliosides, sphingomyelin, or PEG-derivatized lipids, the enhanced circulation half-life of these sterically stabilized liposomes derives from a reduced uptake into cells of the reticuloendothelial system (RES) (Allen et al., FEBS Letters, 1987, 223, 42; Wu et al., Cancer Research, 1993, 53, 3765).

Various liposomes: comprising one or more glycolipids are known in the art. Papahadjopoulos et al. (Ann. N.Y. Acad. Sci., 1987, 507, 64) reported the ability of monosialoganglioside G.sub.MI, galactocerebroside sulfate and phosphatidylinositol to improve blood half-lives of liposomes. These findings were expounded upon by Gabizon al. (Proc. Nail . Acad. Sci. U.S.A., 1988, 85, 6949). U.S. Pat. No. 4,837,028 and WO 88/04924, both to Allen et al., disclose liposomes comprising (1) sphingomyelin and (2) the ganglioside G.sub.MI or a galactocerebroside sulfate ester. U.S. Pat. No. 5,543.152 (Webb et al.) discloses liposomes comprising sphingomyelin. Liposomes comprising 1,2-sn-dimyristoylphosphat- idylcholine are disclosed in WO 97/13499 (Lim et al).

Many liposomes comprising lipids derivatized with one or more hydrophilic polymers, and methods of preparation thereof, are known in the art. Sunamoto et al. (Bull. Chem. Soc. Jpn., 1980, 511, 2778) described liposomes comprising a nonionic detergent, 2C.sub. 121G, that contains a PEG moiety. Illum et al. (FEBS Lett., 1984, 167, 79) noted that hydrophilic coating of polystyrene particles with polymeric glycols results in significantly enhanced blood half-lives. Synthetic phospholipids modified by the attachment of carboxylic groups of polyalkylene glycols (e.g., PEG) are described by Sears (U.S. Pat. Nos. 4,426,330 and 4,534,899). Klibanov et al. (FEBS Lett., 1990, 268, 235) described experiments demonstrating that liposomes comprising phosphatidylethanolamine (PE) derivatized with PEG or PEG stearate have significant increases in blood circulation half-lives. Blume et al. (Biochimica et Biophysica Acta, 1990, 1029, 91) extended such observations to other PEG-derivatized phospholipids, e.g., DSPE-PEG, formed from the combination of distearoylphosphatidylethanolamine (DSPE) and PEG. Liposomes having covalently bound PEG moieties on their external surface are described in European Patent No. EP 0 445 131 B1 and WO 90/04384 to Fisher. Liposome compositions containing 1-20 mole percent of PE derivatized with PEG, and methods of use thereof, are described by Woodle et al. (U.S. Pat. Nos. 5,013,556 and 5,356,633) and Martin et al. (U.S. Pat. No. 3,213,804 and European Patent No. EP 0 496 813 B1). Liposomes comprising a number of other lipid-polymer conjugates are disclosed in WO 91/05545 and U.S. Pat. No. 5,225,212 (both to Martin et al.) and in WO 94/20073 (Zalipsky et al.) Liposomes comprising PEG-modified ceramide lipids are described in WO 96/10391 (Choi et al). U.S. Pat. No. 5,540,935 (Miyazaki et al.) and U.S. Pat. No. 5,556,948 (Tagawa et. al.) describe PEG-containing liposomes that can be further derivatized with functional moieties on their surfaces.

A limited number of liposomes comprising nucleic acids are known in the art. WO 96/40062 to Thierry et al. discloses methods for encapsulating high molecular weight nucleic acids in liposomes. U.S. Pat. No. 5,264,221 to Tagawa et al. discloses protein-bonded Eposomes and asserts that the contents of such liposomes may include an dsRNA RNA. U.S. Pat. No. 5,665,7 10 to Rahman et al. describes certain methods of encapsulating oligonucleotides in liposomes. WO 97/04787 to Love et al. discloses liposomes comprising dsRNA dsRNAs targeted to the raf gene.

Transfersomes are yet another type of liposomes, and are highly deformable lipid aggregates which are attractive candidates for drug delivery vehicles. Transfersomes may be described as lipid droplets which are so highly deformable that they are easily able to penetrate through pores which are smaller than the droplet. Transfersomes are adaptable to the environment in which they are used, e.g. they are self-optimizing (adaptative to the shape of pores in the skin), self-repairing, frequently reach their targets without fragmenting, and often self-loading. To make transfersomes it is possible to add surface edge-activators, usually surfactants, to a standard liposomal composition. Transfersomes have been used to deliver serum albumin to the skin. The transfersome-mediated delivery of serum albumin has been shown to be as effective as subcutaneous injection of a solution containing serum albumin.

Surfactants find wide application in formulations such as emulsions (including microemulsions) and liposomes. The most common way of classifying and ranking the properties of the many different types of surfactants, both natural and synthetic, is by the use of the hydrophile/lipophile balance (HLB). The nature or the hydrophilic group (also known as the "head") provides the most useful means for categorizing the different surfactants used in formulations (Rieger, in Pharmaceutical Dosage Forms, Marcel Dekker, Inc., New York, N.Y., 1988, p. 285).

If the surfactant molecule is not ionized, it is classified as a nonionic surfactant. Nonionic surfactants find wide application in pharmaceutical and cosmetic products and are usable over a wide range of pH values. In general their HLB values range from 2 to about 18 depending on their structure. Nonionic surfactants include nonionic esters such as ethylene glycol esters, propylene glycol esters, glyceryl esters, polyglyceryl esters, sorbitan esters, sucrose esters, and ethoxylated esters. Nonionic alkanolamides and ethers such as fatty alcohol ethoxylates, propoxylated alcohols, and ethoxylated/propoxylated block polymers are also included in this class. The polyoxyethylene surfactants are the most popular members of the nonionic surfactant class.

If the surfactant molecule carries a negative charge when it is dissolved or dispersed in water, the surfactant is classified as anionic. Anionic surfactants include carboxylates such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates. The most important members of the anionic surfactant class are the alkyl sulfates and the soaps.

If the surfactant molecule carries a positive charge when it is dissolved or dispersed in water, the surfactant is classified as cationic. Cationic surfactants include quaternary ammonium salts and ethoxylated amines. The quaternary ammonium salts are the most used members of this class.

If the surfactant molecule has the ability to carry either a positive or negative charge, the surfactant is classified as amphoteric. Amphoteric surfactants include acrylic acid derivatives, substituted alkylamides, N-alkylbetaines and phosphatides.

The used of surfactants in drug products, formulations and in emulsions has been reviewed (Rieger, in Pharmaceutical Dosage Forms, Marcel Dekker, inc., New York, N.Y., 1988, p. 285).

Agents that enhance uptake of dsRNAs at the cellular level may also be added to the pharmaceutical and other compositions of the present invention. For example, cationic lipids, such as lipofectin (Junichi et al, U.S. Pat. No. 5,705,188), cationic glycerol derivatives, and polycationic molecules, such as polylysine (Lollo et al., PCT Application WO 97/30731), are also known to enhance the cellular uptake of dsRNAs.

Other agents may be utilized to enhance the penetration of the administered nucleic acids, including glycols such as ethylene glycol and propylene glycol, pyrrols such as 2-pyrrol, azones, and terpenes such as limonene and menthone.

Carriers

Certain compositions of the present invention also incorporate carrier compounds in the formulation. As used herein, "carrier compound" or "carrier" can refer to a nucleic acid, or analog thereof, which is inert (i.e., does not possess biological activity per se) but is recognized as a nucleic acid by in vivo processes that reduce the bioavailability of a nucleic acid having biological activity by, for example, degrading the biologically active nucleic acid or promoting its removal from circulation. The coadministration of a nucleic acid and a carrier compound, typically with an excess of the latter substance, can result in a substantial reduction of the amount of nucleic acid recovered in the liver, kidney or other extracirculatory reservoirs, presumably due to competition between the carrier compound and the nucleic acid for a common receptor. For example, the recovery of a partially phosphorothioate dsRNA in hepatic tissue can be reduced when it is coadmmislered with polyinosinic acid, dextran sulfate, polycytidic acid or 4-acetamido-4'isothiocyano-stilbene-2,2'-dusulfonic acid (Miyao et al., DsRNA Res. Dev., 1995, 5, 115-121; Takakura et al., DsRNA & Nucl. Acid Drug Dev., 1996, 6, 177-183.

Excipients

In contrast to a carrier compound, a "pharmaceutical carrier" or "excipient" is a pharmaceutically acceptable solvent, suspending agent or any other pharmacologically inert vehicle for delivering one or more nucleic acids to an animal. The excipient may be liquid or solid and is selected, with the planned manner of administration in mind, so as to provide for the desired bulk, consistency, etc., when combined with a nucleic acid and the other components of a given pharmaceutical composition. Typical pharmaceutical carriers include, but are not limited to, bending agents (e.g., pregelatinized maize starch, polyvinylpyrrolidone or hydroxypropyl methylcellulose, etc.); fillers (e.g., lactose and other sugars, microcrystalline cellulose, pectin, gelatin, calcium sulfate, ethyl cellulose, polyacrylates or calcium hydrogen phosphate, etc.); lubricatants (e.g., magnesium stearate, talc, silica, colloidal silicon dioxide, stearic acid, metallic stearates, hydrogenated vegetable oils, corn starch, polyethylene glycols, sodium benzoate, sodium acetate, etc.); disintegrants (e.g., starch, sodium starch glycolate, etc.); and wetting agents (e.g., sodium lauryl sulphate, etc).

Pharmaceutically acceptable organic or inorganic excipient suitable for non-parenteral administration which do not deleteriously react with nucleic acids can also be used to formulate the compositions of the present invention. Suitable pharmaceutically acceptable carriers include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone and the like.

Suitable pharmaceutically acceptable excipients include, but are not limited to, water, salt solutions, alcohol, polyethylene glycols, gelatin, lactose, amylose, magnesium stearate, talc, silicic acid, viscous paraffin, hydroxymethylcellulose, polyvinylpyrrolidone and the like.

Other Components

The compositions of the present invention may additionally contain other adjunct components conventionally found in pharmaceutical compositions, at their art-established usage levels. Thus, for example, the compositions may contain additional, compatible, pharmaceutically-active materials such as, for example, antiprurities, astringents, local anesthetics or anti-inflammatory agents, or may contain additional materials useful in physically formulating various dosage forms of the compositions of the present invention, such as dyes, flavoring agents, preservatives, antioxidants, opacifiers, thickening agents and stabilizers However, such materials, when added, should not unduly interfere with the biological activities of the components of the composition of the present invention. The formulations can be sterilized and, if desired, mixed with auxiliary agents, e.g., lubricants, preservatives, stabilizers, wetting agents, emulsifiers, salts for influencing osmotic pressure, buffers, colorings, flavorings and/or aromatic substances and the like which do not deteteriously interact with the nucleic acid(s) of the formulation.

Aqueous suspensions may contain substances which increase the viscosity of the suspension including, for example, sodium carboxymethylcellulose, sorbitol and/or dextran. The suspension may also contain stabilizers.

Certain embodiments of the invention provide pharmaceutical compositions containing (a) one or more antisense compounds and (b) one or more other chemotherapeutic agents which function by a non-antisense mechanism. Examples of such chemotherapeutic agents include but are not limited to dannorubicin, daumomycin, dactinomycin, doxorubicin, epirubicin, idarubicin. esorubicin, bleomycin, mafosfamide, ifosfamide, cytosine arabinoside, bis-chloroethylnitrosurea, busulfan, mitomycin C, actinomycin D, mithramycin, prednisone, hydroxyprogesterone, testosterone, tamoxifen, dacarbazinz, procarbazine, hexamethylmelamine, pentamethylmelamine, mitoxantrone, amsacrine, chlorambucil, methylcyclohexylnitrosurea, nitrogen mustards, melphalan, cyclophosphamide, 6-mercaptopurine, 6-thioguanine, cytarabine, 5-azacytidine, hydroxyurea, deoxycoformycin, 4-hydroxyperoxycyclophosphor- amide, 5-fluorouracil (5-FU), 5-fluorodeoxyuridine (5-FUdR), methotrexate (MTX), colchicine, taxol, vincristine, vinblastine, etoposide (VP-16), trimetraxate, irinotecan, topotecan, gerncitabine, teniposide, cisphatin and diethylstilbestrol (DES). See, generally. The Merck Manual of Diagnosis and Therapy, 15th Ed. 1987, pp. 1206-1228, Berkow et al., eds., Rahway, N.J. When. used with the compounds of the invention. such chemotherapeutic agents may be used individually (e.g., 5-FU and oligonucleotide), sequentially (e.g., 5-FU and oligonucleotide for a period of time followed by MTX and oligonucleotide), of in combination with one or more other such chemotherapeutic agents (e.g., 5-FU, MTX and oligonucleotide, or 5-FU, radiotherapy and oligonucleotide). Anti-inflammatory drugs, including but not limited to nonsteroidal anti-inflammatory drugs and corticosteroids, and antiviral drugs, including but not limited to ribivirin, vidarabine, acyclovir and ganciclovir, may also be combined in compositions of the invention. See, generally, The Merck Manual of Diagnosis and Therapy, 15th Ed., Berkow et al., eds., 1987, Rahway, N.J., pages 2499-2506 and 46-49, respectively). Other non-antisense chemotherapeutic agents are also within the scope of this invention. Two or more combined compounds may be used together or sequentially.

Toxicity and therapeutic efficacy of such compounds can be determined by standard pharmaceutical procedures in cell cultures or experimental animal, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred.

The data obtained from cell culture assays and animal studies can be used in formulation a range of dosage for use in humans. The dosage of compositions of the invention lies generally within a range of circulating concentrations that include the ED50 with little or no toxicity. The dosages may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range of the compound or, when appropriate, of the polypeptide product of a target sequence (e.g., achieving a decreased concentration of the polypeptide) that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

In addition to their administration individually or as a plurality, as discussed above, the dsRNAs of the invention can be administered in combination with other known agents effective in treatment of pathological processes mediated by JC virus expression. In any event, the administering physician can adjust the amount and timing of dsRNA administration on the basis of results observed using standard measures of efficacy known in the art or described herein.

## Methods for treating diseases caused by expression of a gene from the JC Virus

The invention relates in particular to the use of a dsRNA as defined by the claims or a pharmaceutical composition prepared therefrom for the treatment or prevention of pathological conditions associated with JC Virus infection, e.g., PML. Owing to the inhibitory effect on JC virus expression, an dsRNA according to the invention or a pharmaceutical composition prepared therefrom can enhance the quality of life, particularly in a patient being treated with an anti-VLA4 antibody as part of treatment for MS.

The invention furthermore relates to the use of such an dsRNA or a pharmaceutical composition thereof for treating PML in combination with other pharmaceuticals and/or other therapeutic methods, e.g., with known pharmaceuticals and/or known therapeutic methods, such as, for example, those which are currently employed for treating cancer and/or for preventing tumor metastasis. Preference is given to a combination with radiation therapy and chemotherapeutic agents, such as cisplatin, cyclophosphamide, 5-fluorouracil, adriamycin, daunorubicin or tamoxifen.

The invention can also be practiced by including with a specific RNAi agent, in combination with another anticancer chemotherapeutic agent, such as any conventional chemotherapeutic agent. The combination of a specific binding agent with such other agents can potentiate the chemotherapeutic protocol. Numerous chemotherapeutic protocols will present themselves in the mind of the skilled practitioner as being capable of incorporation into the method of the invention. Any chemotherapeutic agent can be used, including alkylating agents, antimetabolites, hormones and antagonists, radioisotopes, as well as natural products. For example, the compound of the invention can be administered with antibiotics such as doxorubicin and other anthracycline analogs, nitrogen mustards such as cyclophosphamide, pyrimidine analogs such as 5-fluorouracil, cisplatin, hydroxyurea, taxol and its natural and synthetic derivatives, and the like. As another example, in the case of mixed tumors, such as adenocarcinoma of the breast, where the tumors include gonadotropin-dependent and gonadotropin-independent cells, the compound can be administered in conjunction with leuprolide or goserelin (synthetic peptide analogs of LH-RH). Other antineoplastic protocols include the use of a tetracycline compound with another treatment modality, e.g., surgery, radiation, etc., also referred to herein as "adjunct antineoplastic modalities." Thus, the method of the invention can be employed with such conventional regimens with the benefit of reducing side effects and enhancing efficacy.

## Methods for inhibiting expression of a gene from the JC Virus

Also described herein is a method for inhibiting the expression of a gene from the JC Virus in a mammal. The method comprises administering a composition of the invention to the mammal such that expression of the target JC virus genome is silenced. Because of their high specificity, the dsRNAs of the invention specifically target RNAs (primary or processed) of the target JC virus gene. Compositions and methods for inhibiting the expression of these JC virus genes using dsRNAs can be performed as described elsewhere herein.

The method may comprises administering a composition comprising a dsRNA, wherein the dsRNA comprises a nucleotide sequence which is complementary to at least a part of an RNA transcript of a gene from the JC Virus, to the mammal to be treated. When the organism to be treated is a mammal such as a human, the composition may be administered by any means known in the art including, but not limited to oral or parenteral routes, including intravenous, intramuscular, subcutaneous, transdermal, airway (aerosol), nasal, administration. The compositions may be administered by intravenous infusion or injection.

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below. In case of conflict, the present specification, including definitions, will control. In addition, the materials, methods, and examples are illustrative only and not intended to be limiting.

## EXAMPLES

### Design of JCV siRNAs

Full-length genome sequences to JC virus available on April 10, 2006, were obtained, resulting in a target pool of 388 sequences (accession numbers: AB038249.1 - AB038255.1; AB048545.1 - AB048582.1; AB074575.1 - AB074591.1; ; AB077855.1 - AB077879.1; AB081005.1 - AB081030.1; AB081600.1 - AB081618.1; AB081654.1; AB092578.1 - AB092587.1; AB103387.1; AB103402.1 - AB103423.1; AB104487.1; AB113118.1 - AB113145.1; AB118651.1 - AB118659.1; AB126981.1 - AB127027.1; AB127342.1; AB127344.1; AB127346.1 - AB127349.1; AB127352.1 - AB127353.1; AB198940.1 - AB198954.1; AB220939.1 - AB220943.1; AF004349.1 - AF004350.1; AF015526.1 - AF015537.1; AF015684.1; AF030085.1; AF281599.1 - AF281626.1; AF295731.1 - AF295739.1; AF300945.1 - AF300967.1; AF363830.1 - AF363834.1; AF396422.1 - AF396435.1; AY121907.1 - AY121915.1; NC_ 001699.1; U61771.1; U73500.1 - U73502. 1). NC_001699 was defined as reference sequence.

The siRNA selection process was run as follows: ClustalW multiple alignment was used to generate a global alignment of all sequences from the target pool. An IUPAC consensus sequence was then generated.

All conserved 19mer target sequences from the IUPAC consensus represented by stretches containing only A, T, C or G bases, which are therefore present in all sequences of the target pool were selected. In order to only select siRNAs that target transcribed sequence parts of the JC virus, candidate target sequences were selected out of the pool of conserved 19mer target sequences. For this, candidate target sequences covering regions between nucleotide 163-2594 and between 2527-5115 relative to reference sequence were extracted for late and early genes, respectively. Further, as sequences for early genes are in reverse complement orientation compared with genomic sequences, candidate target sequences of these genes were transferred to reverse complement sequences and replaced the former pool of candidate target sequences.

In order to rank candidate target sequences and their respective siRNAs and select appropriate ones, their predicted potential for interacting with irrelevant targets (off-target potential) was taken as a ranking parameter. siRNAs with low off-target potential were defined has preferable and assumed to be more specific *in vivo*.

For predicting siRNA-specific off-target potential, the following assumptions were made:

1) positions 2 to 9 (counting 5' to 3') of a strand (seed region) may contribute more off-target potential than rest of sequence (non-seed and cleavage site region)

2) positions 10 and 11 (counting 5' to 3') of a strand (cleavage site region) may contribute more to off-target potential than non-seed region

3) an off-target score can be calculated for each hit, based on identify to siRNA. sequence and position of mismatches

4) assuming potential abortion of sense strand activity by internal modifications introduced, only off-target potential of antisense strand will be relevant.

To identify potential off-target genes, 19mer input sequences were subjected to a homology search against publically available human mRNA sequences.

To this purposes, fastA (version 3.4) searches were performed with an 19mer candidate target sequences against a human RefSeq diabase (downloaded available version from ftp://ftp.ncbi.nih.gov/refseq/ on November 7, 2006). FastA searches were executed with parameters-values-pairs -f50 -g 50 in order to take into account the homology over the full length, of the 19mer without any gaps. In order to ensure the listing of all relevant off-target hits in the fastA output file the parameter -E 30000 was used in addition. A scoring matrix was applied for the run that assessed every nucleotide match with a score of 13 and every mismatch with a score of -7. The search resulted in a list of potential off-targets for each candidate siRNA.

To sort the resulting list of potential off-targets for each siRNA, fastA output files were analyzed to identify the best off-target and its off-target score. The following off-target properties for each 19mer input sequence were extracted for each off-target to the score:

- Number of mismatches in non-seed region
- Number of mismatches in non-seed region
- Number of mismatches in seed region
- Number of mismatches in cleavage site region

The off-target score was calculated for considering assomption 1 to 3 as follows:

$$\text{Off-target score} = \text{number of seed mismatches} * 10 + \text{number of cleavage site mismatches} * 1.2 + \text{number of non-seed mismatches} * 1$$

The most relevant off-target gene for input each 19mer input sequences was defined as the gene with the lowest off-target score. Accordingly, the lowest off-target score was defined as the relevant off-target score for the corresponding siRNA.

In order to generate a ranking for siRNAs, calculated relevant off-target scores were transferred into a result table. All siRNAs were sorted according to the off-target score (descending).

An off-target score of 2.2 was defined as cut-off for siRNA selection (specificity criterion). In addition, all sequences with only one mismatch in the seed region were eliminated from the set. The selection procedure resulted in a set of 93 JCV specific siRNAs (Table 1a).

An expanded screening was generated by re-calculating the predicted specifically bused on the newly available human RefSeq database (Human mRNA sequences in RefSeq release version 21 (downloaded January 12, 2007) and selecting only 208 siRNAs that did not contain more than 3 G's in a row and had an off-target score of at least 2 for the antisense strand (Table 1b).

__Synthesis of JCV siRNAs__

All siRNAs were synthesized in 0.2 μmole synthesis scale on an ABI3900 DNA synthesizer according to standard procedures.

For the initial screening set (93 different siRNA sequences), 4 different strategies of chemical modification were used:

a) exo/endo light (EEL):

- sense strand: 2'-O-methyl @ all pyrimidines, PTO between nucleotides 20 and 21 (counting from 5'-end), dTdT at 3'-end (nucleotides) 20 and 21)
- antisense strand: 2'-O-methyl at pyrimidines in 5'-UA-3' and 5'-CA-3' motives, PTO between nucleotides 20 and 21 (counting from 5'-end), dTdT at 3'-end (nucleotides 20 and 21)

b) EEL plus 2'-O-methyl in position 2 of antisense strand (only if no 5'-UA-3' and 5'-CA-3' at 5'-end, otherwise already covered by EEL)

c) EEL plus 2'-O-methyl in position 2 of sense strand (only if no pyrimidine in position 2 otherwise already covered by EEL)

d) EEL plus 2'-O-methyl in position 2 of sense and antisense strand (only if not already covered by a, b, and c) (Table 1a)

For the expanded screnning set (208 dífferent siRNA sequences), siRNAs were composed of unmodified RNA oligonucleotides with dT/dT overhangs (dTdT at 3'-end (nucleotides 20 and 21) of antisense and sense strands) (Table 1b).

**Screening of JCV siRNAs**

Construction of reporter-systems encoding JCV transcripts

. The sequence of the early JCV transcript (E) was synthesized at GENEART (Regensburg, Germany) and cloned into GENEART standard vectors. The sequence of the late JCV transcript was subdivided in a first approach into two fragments: L1, including the transcript sequence of the VP1 protein, and LA23, including the sequences of VP2, VP3 and the Agnoprotein. Due to cloning problems with fragment LA23, this sequence was subdivided in a second approach into two fragments (LA23 1-700 and LA23 701-1438). All sequences were synthesized at GENEART and cloned into GENEART standard vectors. All fragments (E, L1, LA23 1-700 and LA23 701-1438) were subcloned into psiCheck-2 (Promega, Mannheim, Germany) via XhoI and NotI (both NEB, Frankfurt, Germany), resulting in constructs with the JCV sequences between the stop-codon and the polyA-signal of Renilla luciferase.

**L1**

**CTCGAG**ACTTTTAGGGTTGTACGGGACTGTAACACCTGCTCTTGAAGCATATGAAGATGGCCCCA
ACAAAAAGAAAAGGAGAAAGGAAGGACCCCGTGCAAGTTCCAAAACTTCTTATAAGAGGAGGAGT
AGAAGTTCTAGAAGTTAAAACTGGGGTTGACTCAATTACAGAGGTAGAATGCTTTTTAACTCCAG
AAATGGGTGACCCAGATGAGCATCTTAGGGGTTTTAGTAAGTCAATTTCTATATCAGATACATTT
GAAAGTGACTCCCCAAATAAGGACATGCTTCCTTGTTACAGTGTGGCCAGAATTCCACTACCCAA
TCTAAATGAGGATCTAACCTGTGGAAATATACTAATGTGGGAGGCTGTGACCTTAAAAACTGAGG
TTCTAGGGGTGACAACTTTGATGAATGTGCACTCTAATGGTCAAGCAACTCATGACAATGGTGCA

GGAAAGCCAGTGCAGGGCACCAGCTTTCATTTTTTTTCTGTTGGCGGGGAGGCTTTAGAATTACA
GGGGGTGGTTTTTAATTACAGAACAAAGTACCCAGATGGAACAATTTTTCCAAAGAATGCAACAG
TGCAATCTCAAGTAATGAACACAGAGCACAAGGCGTACCTAGATAAGAACAAAGCATATCCTGTT
GAATGTTGGGTTCCTGATCCCACCAGAAATGAAAACACAAGATATTTTGGGACACTAACAGGAGG
AGAAAATGTTCCTCCAGTTCTTCATATAACAAACACTGCCACAACAGTGCTGCTTGATGAATTTG
GTGTTGGGCCACTTTGCAAAGGTGACAACTTGTATTTGTCAGCTGTTGATGTTTGTGGAATGTTT
ACTAACAGATCTGGTTCCCAGCAGTGGAGAGGACTGTCCAGATATTTTAAGGTTCAGCTCAGAAA
AAGGAGGGTTAAAAACCCCTACCCAATTTCTTTCCTTCTTACTGATTTGATTAACAGAAGGACCC
CTAGAGTTGATGGGCAACCTATGTATGGTATGGATGCTCAGGTAGAGGAGGTTAGAGTTTTTGAG
GGGACAGAGGAACTTCCAGGGGACCCAGACATGATGAGATATGTTGACAGATATGGACAGTTGCA
AACAAAGATGCTGTAATCAAAATCCTTTATTGTAATATGCAGTACATTTTAATAAAGTATAACCA
GCTTTACTTTACAGTTGCAGTCAT**GCGGCCGC** **(SEQ ID NO:931)**

**E**

**CTCGAG**CCGCCTCCAAGCTTACTCAGAAGTAGTAAGGGCGTGGAGGCTTTTTAGGAGGCC
AGGGAAATTCCCTTGTTTTTCCCTTTTTTGCAGTAATTTTTTGCTGCAAAAAGCTAAAATGGACA
AAGTGCTGAATAGGGAGGAATCCATGGAGCTTATGGATTTATTAGGCCTTGATAGGTCTGCATGG
GGGAACATTCCTGTCATGAGAAAAGCTTATCTGAAAAAATGCAAAGAACTCCACCCTGATAAAGG
TGGGGACGAAGACAAGATGAAGAGAATGAATTTTTTATATAAAAAAATGGAACAAGGTGTAAAAG
TTGCTCATCAGCCTGATTTTGGTACATGGAATAGTTCAGAGGTTGGTTGTGATTTTCCTCCTAAT
TCTGATACCCTTTATTGCAAGGAATGGCCTAACTGTGCCACTAATCCTTCAGTGCATTGCCCCTG
TTTAATGTGCATGCTAAAATTAAGGCATAGAAACAGAAAATTTTTAAGAAGCAGCCCACTTGTGT
GGATAGATTGCTATTGCTTTGATTGCTTCAGACAATGGTTTGGGTGTGACTTAACCCAAGAAGCT
CTTCATTGCTGGGAGAAAGTTCTTGGAGACACCCCCTACAGGGATCTAAAGCTTTAAGTGCCAAC
CTATGGAACAGATGAATGGGAATCCTGGTGGAATACATTTAATGAGAAGTGGGATGAAGACCTGT
TTTGCCATGAAGAAATGTTTGCCAGTGATGATGAAAACACAGGATCCCAACACTCTACCCCACCT
AAAAAGAAAAAAAAGGTAGAAGACCCTAAAGACTTTCCTGTAGATCTGCATGCATTCCTCAGTCA
AGCTGTGTTTAGTAATAGAACTGTTGCTTCTTTTGCTGTGTATACCACTAAAGAAAAAGCTCAAA
TTTTATATAAGAAACTTATGGAAAAATATTCTGTAACTTTTATAAGTAGACATGGTTTTGGGGGT
CATAATATTTTGTTTTTCTTAACACCACATAGACATAGAGTGTCAGCAATTAATAACTACTGTCA
AAAACTATGTACCTTTAGTTTTTTAATTTGTAAAGGTGTGAATAAGGAATACTTGTTTTATAGTG

CCCTGTGTAGACAGCCATATGCAGTAGTGGAAGAAAGTATTCAGGGGGGCCTTAAGGAGCATGAC
TTTAACCCAGAAGAACCAGAAGAAACTAAGCAGGTTTCATGGAAATTAGTTACACAGTATGCCTT
GGAAACCAAGTGTGAGGATGTTTTTTTGCTTATGGGCATGTACTTAGACTTTCAGGAAAACCCAC
AGCAATGCAAAAAATGTGAAAAAAAGGATCAGCCAAATCACTTTAACCATCATGAAAAACACTAT
TATAATGCCCAAATTTTTGCAGATAGCAAAAATCAAAAAAGCATTTGCCAGCAGGCTGTTGATAC
TGTAGCAGCCAAACAAAGGGTTGACAGCATCCACATGACCAGAGAAGAAATGTTAGTTGAAAGGT
TTAATTTCTTGCTTGATAAAATGGACTTAATTTTTGGGGCACATGGCAATGCTGTTTTAGAGCAA
TATATGGCTGGGGTGGCCTGGATTCATTGCTTGCTGCCTCAAATGGACACTGTTATTTATGACTT
TCTAAAATGCATTGTATTAAACATTCCAAAAAAAAGGTACTGGCTATTCAAGGGGCCAATAGACA
GTGGCAAAACTACTTTAGCTGCAGCTTTACTTGATCTCTGTGGGGGAAAGTCATTAAATGTTAAT
ATGCCATTAGAAAGATTAAACTTTGAATTAGGAGTGGGTATAGATCAGTTTATGGTTGTATTTGA
GGATGTAAAAGGCACTGGTGCAGAGTCAAGGGATTTACCTTCAGGGCATGGCATAAGCAACCTTG
ATTGCTTAAGAGATTACTTAGATGGAAGTGTAAAAGTTAATTTAGAGAGAAAACACCAAAACAAA
AGAACACAGGTGTTTCCACCTGGAATTGTAACCATGAATGAATATTCAGTGCCTAGAACTTTACA
GGCCAGATTTGTAAGGCAGATAGATTTTAGACCAAAGGCCTACCTGAGAAAATCACTAAGCTGCT
CTGAGTATTTGCTAGAAAAAAGGATTTTGCAAAGTGGTATGACTTTGCTTTTGCTTTTAATCTGG
TTTAGGCCAGTTGCTGACTTTGCAGCTGCCATTCATGAGAGGATTGTGCAGTGGAAAGAAAGGCT
GGATTTAGAAATAAGCATGTATACATTTTCTACTATGAAAGCTAATGTTGGTATGGGGAGACCCA
TTCTTGACTTTCCTAGAGAGGAAGATTCTGAAGCAGAAGACTCTGGACATGGATCAAGCACTGAA
TCACAATCACAATGCTTTTCCCAGGTCTCAGAAGCCTCTGGTGCAGACACACAGGAAAACTGCAC
TTTTCACATCTGTAAAGGCTTTCAATGTTTCAAAAAACCAAAGACCCCTCCCCCAAAATAACTGC
AACTGT**GCGGCCGC** **(SEQ ID NO:932)**

**LA23 1-700**

**CTCGAG**CAGCTAACAGCCAGTAAACAAAGCACAAGGGGAAGTGGAAAGCAGCCAAGGGAACATGT
TTTGCGAGCCAGAGCTGTTTTGGCTTGTCACCAGCTGGCCATGGTTCTTCGCCAGCTGTCACGTA
AGGCTTCTGTGAAAGTTAGTAAAACCTGGAGTGGAACTAAAAAAAGAGCTCAAAGGATTTTAATT
TTTTTGTTAGAATTTTTGCTGGACTTTTGCACAGGTGAAGACAGTGTAGACGGAAAAAAAGACA
GAGACACAGTGGTTTGACTGAGCAGACATACAGTGCTTTGCCTGAACCAAAGCTACATAGGTAA
GTAATGTTTTTTTTTGTGTTTTCAGGTTCATGGGTGCCGCACTTGCACTTTTGGGGGACCTAGTT
GCTACTGTTTCTGAGGCTGCTGCTGCCACAGGATTTTCAGTAGCTGAAATTGCTGCTGGAGAGGC

TGCTGCTACTATAGAAGTTGAAATTGCATCCCTTGCTACTGTAGAGGGGATTACAAGTACCTCTG
AGGCTATAGCTGCTATAGGCCTTACTCCTGAAACATATGCTGTAATAACTGGAGCTCCGGGGGCT
GTAGCTGGGTTTGCTGCATTGGTTCAAACTGTAACTGGTGGTAGTGCTATTGCTCAGTTGGGATA
TAGATTTTTTGCTGACTGGGATCATAAAGTTTCAACAGTTGGGCTTTTTC**GCGGCCGC** **(SEQ ID NO:933)**

**LA23 701-1438**

**CTCGAG**AGCAGCCAGCTATGGCTTTACAATTATTTAATCCAGAAGACTACTATGATATTTTATTT
CCTGGAGTGAATGCCTTTGTTAACAATATTCACTATTTAGATCCTAGACATTGGGGCCCGTCCTT
GTTCTCCACAATCTCCCAGGCTTTTTGGAATCTTGTTAGAGATGATTTGCCAGCCTTAACCTCTC
AGGAAATTCAGAGAAGAACCCAAAAACTATTTGTTGAAAGTTTAGCAAGGTTTTTGGAAGAAACT
ACTTGGGCAATAGTTAATTCACCAGCTAACTTATATAATTATATTTCAGACTATTATTCTAGATT
GTCTCCAGTTAGGCCCTCTATGGTAAGGCAAGTTGCCCAAAGGGAGGGAACCTATATTTCTTTTG
GCCACTCATACACCCAAAGTATAGATGATGCAGACAGCATTCAAGAAGTTACCCAAAGGCTAGAT
TTAAAAACCCCAAATGTGCAATCTGGTGAATTTATAGAAAGAAGTATTGCACCAGGAGGTGCAAA
TCAAAGATCTGCTCCTCAATGGATGTTGCCTTTACTTTTAGGGTTGTACGGGACTGTAACACCTG
CTCTTGAAGCATATGAAGATGGCCCCAACAAAAAGAAAAGGAGAAAGGAAGGACCCCGTGCAAGT
TCCAAAACTTCTTATAAGAGGAGGAGTAGAAGTTCTAGAAGTTAAAACTGGGGTTGACTCAATTA
CAGAGGTAGAATGCT**GCGGCCGC** **(SEQ ID NO:934)**

Screen of JCV siRNAs in transfected cells

Cos-7 cells (DSMZ, Braunschweig, Germany, # ACC-60) were seeded at 1.5 x $10^4$ cells /well on white 96-well plates with clear bottoms (Greiner Bio-One GmbH, Frickenhausen, Germany) in 75 μl of growth medium. Directly after seeding the cells, 50 ng of the corresponding reporter-plasmid per well was transfected with Lipofectamine™ 2000 (Invitrogen GmbH. Karlsruhe, Germany), with the plasmid diluted in Opti-MEM to a final volume of 12.5 μl per well, prepared as a mastermix for the whole plate.

4h after plasmid transfection, growth medium was removed from cells and replaced by 100 μl / well of fresh medium. siRNA transfections were performed using Lipofectamine™ 2000 (Invitrogen GmbH, Karlsruhe, Germany) as described by the manufacturer. Cells were incubated for 24 h at 37°C and 5 % $CO_2$ in a humidified incubator (Heraeus GmbH, Hanau, Germany). For the primary screen, all siRNAs were screened at a final concentration of 30nM. Selected sequences were rescreened at a siRNA concentration of 300pM. Each siRNA was tested. in quadruplicate for each concentration.

Cells were lysed by removing growth medium and application of 150 μl of a 1:1 mixture consisting of medium and substrate from the Dual-Glo Luciferase Assay System (Promega, Mannheim, Germany). The luciferase assay was performed according to the manufacturer's protocol for Dual-Glo Luciferase assay luminescence was measured in a Victor-Light 1420 Luminescence Counter (Perkin Elmer, Rodgau-Jügesheim, Germany). Values obtained with Renilla luciferase were normalized to the respective values obtained with Firefly luciferase in order to correct for transfection efficacy. Renilla/Firefly luciferase activities obtained after transfection with siRNAs directed against a JCV gene were non-normlized to Renilla/Firefly lacificrase activities obtained after transfection of an unrelated control siRNA set to 100%. Tables 1a and b provides the results where the siRNAs, the sequences of which are given in Tables 1a and b, were tested at a single dose of 30nM. The percentage inhihition ± standard deviation, compared to the unrelated control siRNA, is indicated in the column 'Remaining luciferase activity (% of control)'. A number of JCV siRNAs at 30nM were effective at reducing levels of the targeted mRNA by more than 70% in Cos-7 cells (i.e. remaining luciferase activity was less than 30%).

Selected JCV siRNAs from the single dose screen were further characterized by dose response curves. Transfections of JCV siRNAs for generation of dose response curves were performed with the following siRNA concentrations according to the above protocol:

- from 33nM in 3-fold dilutions down to 0.005nM (for fragment L1)
- from 24nM in 4-fold dilutions down to 0.001nM (for fragment B and fragments LA23 1-700 and LA23 701-1438).

IC50 values were determined by parameterized curve fitting using the program XL fit IC50 values were determined by parameterized carve fitting using the program X.L fit (IDBS, Guildford, Great Britain). Table 2 provides the results from two independent experiments for 32 selected JCV siRNAs. The mean IC50 from these two independent experiments is shown. Several JCV siRNAs (AD-12622, AD-12677, AD-12709, AD-12710, experiments is shown. Several JVC siRNAS (AD-12622, AD-12677, AD-12709, AD-12710, AD-12722, AD-12724, AD-12728, AD-12763, AD-12767, AD-12768, AD-12769, AD-12771, AD-12774, AD-12775, AD-12777, AD-12781, AD-12784, AD-12795, AD-12813, AD-12821, AD-12823, AD-12824, AD-12825, AD-12827, AD-12829, AD-12842) were particularity potent in this experimental paradigm, and exhibited $IC_{50}$ values between 70pM: and 1nM.

**Table 2: IC50s**

| Duplex name | Mean IC50 [nM] |
|---|---|
| AD-12599 | 2.37 |
| AD-12622 | 0.57 |
| AD- 12666 | 3.7 |
| AD-12677 | 0.49 |
| AD-12709 | 0.19 |
| AD-12710 | 0.47 |
| AD- 12712 | 2.33 |
| AD-12722 | 0.12 |
| AD-12724 | 0.26 |
| AD-12728 | 0.8 |
| AD-12761 | 1.2 |
| AD-12763 | 0.95 |
| AD-12767 | 0.09 |
| AD-12768 | 0.19 |
| AD-12769 | 0.35 |
| AD-12771 | 0.35 |
| AD-12774 | 0.13 |
| AD-12775 | 0.18 |
| AD-12777 | 0.17 |
| AD-12778 | 12.65 |
| AD-12781 | 0.18 |
| AD-12784 | 0.44 |
| AD-12795 | 0.65 |
| AD-12813 | 0.2 |
| AD-12818 | 1.88 |
| AD-12821 | 0.07 |
| AD-12843 | 0.46 |
| AD-12824 | 0.25 |
| AD-12825 | 0.52 |
| AD-12827 | 0.15 |
| AD-12829 | 0.14 |
| AD-12842 | 0.44 |

**Screen of JCV siRNAs against live JC Virus in SVG-A cells**

Cells and Virus

SVG-A cells (human fetal glial cells transformed by SV40 T antigen) obtained from Waller Atwood at Brown University were cultured in Eagle's Minimum Essential Media (ATCC, Manassas, Virginia) supplemented to contain 10% fetal bovine serum (FBS) (Omega Scientific, Tarzana, California), Penicillin 100U/ml, Streptomycin 100ug/ml (Invitrogen, Carisbad California) at 37°C in an atmosphere with 5% $CO_2$ in a humidified incubator (Heraeus HERAcell, Thermo Electron Corporation, Ashville, North Carolina). The Mad-1-SVEΔ strain of JCV obtained from Walter Atwood at Brown University was used in all experiments; viral stocks were prepared using SVG-A cells according to standard published methods (Liu and Atwood, Propagation and assay of the JC Virus, Methods Mol Biol. 2001: 165:9-17).

Prophylaxis assay

SVG-A cells were seeded on glass coverslips in 6-well dishes 24 hours prior to transfection in the media described above minus antibiotics. Cells were transfected with the indicated concentration of siRNA (10nM, 50nM, or 100nM) using Lipofectamine™ 2000 according to the manufacturer's instructions (Invitrogen, Carlsbad, California). Twenty-four hours post-.transfection cells were washed with media containing 2% FBS and then infected with a 1:25 dilution of JCV virus stock (Mad-1-SVEΔ strain) diluted in 2% FBS media. Cells were rocked every 15 minutes by hand several times to get equal virus binding across the entire coverslip for one hour and then additional 10% FBS media was added and the infection was allowed to proceed for 72 hours. Seventy two hours post-infection, cells were fixed in acetone and stained for late viral protein (VP1) by standard immunofluoresence methods using hybridoma supernatant PAB597 recognizing JCV VPI (obtained from Walter Atwood at Brown University) with goat anti-mouse Alexa Fluor 488 secondary antibody (Invitrogen, Carlsbad, California). Infected cells were scored by counting VP1-immunoreactive cells using a fluorescence microscope ( Zeiss, Imager.Z1, Thornwood, New Jersey) and data were expressed as the percentage of infected cells counted for the control coverslips transfected with Luciferase siRNA. Table 3 shows the results of the prophylaxis assays at different siRNA concentrations (10nM. 50nM or 100nM). The VP1 siRNAs were the most potent as a group, followed by the T antigen siRNAs, with the VP2/3 siRNAs being the least potent. The VP1 siRNAs most effective in reducing virus were consistently AD-12622, AD-12728, AD-12795, and AD-12842. The most potent T antigen siRNA was AD-12813.

**Table 3. Prophylaxis Assay**

| | | Remaining Virus (% of Luciferase Control) | | |
|---|---|---|---|---|
| **Duplex Number** | **Targeted JCV Transcript** | **50nM** | **10nM** | **100nM** |
| AD-12599 | \/P1 | 79.9 | ND | ND |
| AD-12709 | VP1 | 46.0 | ND | ND |
| AD-12710 | VP1 | 25.9 | ND | ND |
| AD-12784 | VP1 | 30.9 | ND | ND |
| AD-12712 | VP1 | 29.7 | ND | ND |
| AD-12724 | VP1 | 30.5 | 38.9 | 25.8 |
| AD-12622 | VP1 | 22.9 | 28.2 | 9.1 |
| AD-12728 | VP1 | 21.1 | 22.2 | ND |
| AD-12795 | VP1 | 13.6 | 16.9 | 8.5 |
| AD-12842 | VP1 | 16.0 | 23.4 | 12.7 |
| AD-12761 | VP1 | 26.4 | 52.3 | ND |
| AD.12818 | VP1 | 24.0 | 50.2 | 28.0 |
| AD-12666 | VP1 | 54.1 | ND | ND |
| AD-12763 | VP1 | 39.5 | ND | ND |
| AD-12722 | T Antigen | 43.6 | 82.1 | ND |

(continued)

| | | Remaining Virus (% of Luciferase Control) | | |
|---|---|---|---|---|
| Duplex Number | Targeted JCV Transcript | 50nM | 10nM | 100nM |
| AD-12813 | Antigen | 21.5 | 48.8 | 19.4 |
| AD-12767 | T Antigen | 37.6 | 52.2 | 30.9 |
| AD-12821 | T Antigen | 33.0 | 51.2 | 30.8 |
| AD-12774 | T Anitgen | 74.0 | 89.2 | ND |
| AD-12827 | T Antigen | 77.0 | 92.0 | ND |
| AD-12775 | T Antigen | 81.6 | 95.4 | ND |
| AD-12777 | T Antigen | 73.3 | 93.9 | ND |
| AD-12829 | T Antigen | 78.6 | 93.5 | ND |
| AD-12781 | T Antigen | 38.8 | 62.6 | 34.4 |
| AD-12768 | VP2/3 | 73.9 | 92.4 | ND |
| AD-12771 | VP2/3 | 51.6 | 83.6 | ND |
| AD-12824 | VP2/3 | 42.1 | 79.0 | 43.7 |
| AD-12769 | VP2/3 | 35.2 | 78.0 | 39.7 |
| AD-12823 | VP2/3 | 38.1 | 78.1 | 42.0 |
| AD-12677 | VP2/3 | 99,1 | 102.1 | ND |
| AD-12825 | VP2/3 | 100.8 | 99.1 | ND |
| ND indicates no data. | | | | |

Post-infection treatment assay

SVG-A cells were seeded on glass coverslips in (j-well dishes 24 hours prior to infection in 10% FBS media. Cells were washed with media containing 2% FBS and then infected with a 1:25 dilution of JCV virus stock diluted in 2% FBS media. Cells were rocked by hand approximately 8-10 times to get equal virus binding across the entire coverslip every 15 minutes for one hour and then additional 10% FBS media was added. Twenty-four and ferty-eight hours postinfection, cells were washed with 10% FBS media containing no antibiotics and then transfected with 50nM of the indicated siRNA using Lipofectamine™ 2000 according to the manufacturer's instructions (Invitrogen, Carlsbad, California). Seventy-two hours postinfection. cells were fixed in acetone and stained for late viral protein (VP1) by standard immunofluoresence methods using hybridoma supernatant PAB597 recognizing JCV VP1 (obtained from Walter Atwood at Brown University) with goat anti-mouse Alexa Fluor 488 secondary antibody (Molecular Probes, Eugene, Oregon). Infected cells were scored by counting VP1-immunoreactive cells using a fluorescence microscope (Zeiss, Imager.Z.I, Thomwood, New Jersey) and data were expressed as the percentage of infected cells counted for control coverslips transfected with Luciferase siRNA. Table 4 shows the results of the post-infection treatment experiments. An of the siRNAs tested in the treatment assay showed significant antiviral activity against JCV, such that the remaining virus was significantly less than that in the luciferase -siRNA control.

**Table 4. treatment Assay**

| Duplex Number | Targeted JCV Transcript | Remaining Virus (% of Luciferase Control) |
|---|---|---|
| AD-12724 | VP1 | 38.9 |
| AD-12622 | VP1 | 28.2 |
| AD-12795 | VP1 | 16.9 |
| AD-12842 | VP1 | 23.4 |
| AD-12818 | VP1 | ND |

(continued)

| Duplex Number | Targeted JCV Transcript | Remaining Virus (% of Luciferase Control) |
|---|---|---|
| AD-12813 | T Antigen | 48 |
| AD-12767 | T Antigen | 56.9 |
| AD-12821 | T Antigen | 75.8 |
| AD-12781 | T Antigen | 75.8 |
| AD-12834 | VP2/3 | 30.4 |
| AD-12769 | VP2/3 | 70.7 |
| AD-12823 | VP2/3 | 72.4 |
| ND indicates no data. | | |

**Prophylaxis administration of JCV siRNAs inhibits the production of active progeny JC virus**

SVG-A cells were seeded in 6-well dishes 24 hours prior to transaction in the media described above minus antibiotics. Cells were transfected with 10nM of line indicated siRNA using Lipofectamine™ 2000 according to the manufacturer's instructions (Invitrogen, Clarlsbad, California). Twenty-four hours post-transfection cells were washed with media containing 2% FBS and then infected with a 1:25 dilution or JCV virus stock (Mad-1-SVEΔ diluted in 2% FBS media. Cells were rocked every 1.5 minutes by hand several times to get equal virus binding across the entire coverslip for one hour and then additional 10% FBS media was added and the infection was allowed to proceed for 6 days. Six days post-infection, progeny virus was collected either by removal of overlay media from infected cells or by scraping cells and performing virus preparations. The virus preparations consisted of scraping cells into the supernatant media, vortexing, freeze-thawing the re-suspended cells 2 times with vortexing in between, then spinning down the cell debris and taking the supernatant. Fresh SVG-A cells seeded on glass coverslips were infected secondarily with virus collected by either method using the same procedure done with the initial infection to determine the amount of infections virus produced by cells transfected with the various siRNAs. At 72 hours post-infection of coverslips, cells were fixed in acetone and stained for late viral protein (VP1) by standard immunofluoresence methods using hybridoma supernatant PAB597 recognizing JCV VPI (obtained from Walter Atwood at Brown University) with goat anti-mouse Alexa Fluor 488 secondary antibody (Invitrogen. Carlsbad, California). Infected cells were scored by counting VP1-immunoreactive cells using a fluorescence microscope (Zeiss, Imager.Z.I, Thornwood, New Jersey) and data were expressed as the percentage of infected cells counted for the control coverslips transfected with Luciferase siRNA. Table 5 shows the results for selected siRNAs, demonstrating the ability of prophylaxis siRNA treatment to inhibit active progeny virus Production by either method of virus collection. Transfection with siRNAs targeting VP1 (AD-12622 and AD-12842) had the greatest effect on inhibiting the production of active progeny virus regardless of whether virus was collected from media or from infected cell preparations. The T antigen siRNA AD-12813 had the next strongest inhibitory effect, whereas the VP2/3 siRNAs AD-12824 and AD-12769 still showed some albeit a lesser ability to inhibit active progeny JCV production.

**Table 5. Prophylaxis administration of JCV siRNAs inhibits the production of active progeny JC virus capable of secondary infection**

| | | Remaining Virus (% of Luciferase Control) | |
|---|---|---|---|
| Duplex Name | Targeted Transcript | Media | Virus Preparation |
| AD-12622 | VP1 | 30.8 | 24.9 |
| AD-12842 | VP1 | 33.3 | 26.9 |
| AD-12813 | T Antigen | 57.8 | 38.7 |
| AD-12824 | VP2/3 | 83.6 | 57.6 |
| AD-12769 | VP2/3 | 79.1 | 52.2 |

<u>**Stability in cerebrospinal fluid (CSF) of selected siRNAs targeting JCV**</u>

<u>Eleven selected JCV siRNAs were tested for stability at 5 uM over 48h at 37°C in human CSF, as well as in PBS for comparison.</u> 30μl of human cerebrospinal fluid (CSF) was mixed with 3 μl of 50 μM duplex (siRNA) solution

(150pmole/well) in a 96-well plate, sealed to avoid evaporation and incubated for the indicated time at 37°C. Incubation of the siRNA in 30ul PBS for 48h served as a control for non-specific degradation. Reactions were stopped by the addition of 4ul proteinase K (20mg/ml) and 25ul of proteinase K. buffer, and an incubation for 20' at 42°C. Samples were then spin filtered through a 0.2 μm 96 well filter plate at 3000 rpm for 20'. Incubation wells were washed with 50ul Millipore water twice and the combined washing solutions were spin filtered also.

Samples were analyzed by ion exchange HPLC under denaturing conditions. Samples were transferred to single autosampler vials. IEX-HPLC analysis was performed under the following conditions: Dionex DNAPac PA200 (4x250 mm analytical column), temperature of 45°C (denaturing conditions by pH=11), flow rate of 1 ml/min, injection volume of 50 ul, and detection wavelength of 260 nm with 1 nm bandwidth (reference wavelength 600 nm). In addition, the gradient conditions were as follows with HPLC Eluent A: 20mM $Na_3PO_4$ in 10% ACN; PH=11 and HPLC Eluent B: 1 M NaBr in HPLC Eluent A:

| Time | %A | %B |
|---|---|---|
| 0.00 min | 75 | 25 |
| 1.00 min | 75 | 25 |
| 19.0 min | 38 | 62 |
| 19.5 min | 0 | 100 |
| 21.5 min | 0 | 100 |
| 22.0 min | 75 | 25 |
| 24.0 min | 75 | 25 |

Under the above denaturing IEX-HPLC conditions, the duplexes eluted as two separated single strands. All chromatograms were integrated automatically by the Dionex Chromeleon 6.60 HPLC software, and were adjusted manually as necessary. The area under the peak for each strand was calculated and the %-values for each intact full length product (FLP) for each time points were calculated by the following equation:

$$\%\text{-}FLP_{(s/as;\ t=x)} = (PeakArea_{(s/as;\ t=x)} / PeakArea_{(s/as;\ t=0min)}) * 100\%$$

All values were normalized to FLP at t=0 min. Table 6 provides the results after 48 hours of incubation in human CSF at 37°C. At least 75% of both antisense and sense strands of ten JCV siRNAs (AD-12622, AD-12724, AD-12767, AD-12769, AD-12795, AD-12813, AD-12818, AD-12823, AD-12824, AD-12842) were recovered, demonstrating that these siRNAs are highly stable in human CSF at 37°C. For AD-12821, 59% of the antisense and 97% of the sense strand was recovered after 48h of incubation in human CSF at 37°C, showing that this siRNA has a half-life of greather than 48h in human CSF at 37°C.

Table 6: Stability in human CSF

| | % full length material after 48 hours | |
|---|---|---|
| Duplex name | antisense | sense |
| AD-12622 | 93 | 105 |
| AD-12724 | 90 | 106 |
| AD-12767 | 85 | 104 |
| AD-12769 | 100 | 104 |
| AD-12795 | 86 | 109 |
| AD-12813 | 94 | 98 |
| AD-12818 | 75 | 99 |
| AD-12821 | 59 | 97 |
| AD-12823 | 98 | 98 |
| AD-12824 | 84 | 98 |
| AD-12842 | 87 | 102 |

**dsRNA expression vectors**

In another aspect of the invention, JC virus specific dsRNA molecules that modulate JC virus genome expression activity are expressed from transcription units inserted into DNA or RNA vectors (see, e.g., Couture, A, et al., TIG. (1996), 12:5-10; Skillern, A., et al., International PCT Publication No. WO 00/22113, Conrad, International PCT Publication No. WO 00/22114, and Conrad, US Pat. No. 6,054,299). These transgenes can be introduced as a linear construct, a circular plasmid, or a viral vector, which can be incorporated and inherited as a transgene integrated into the host genome. The transgene can also be constructed to permit it to be inherited as an extrachromosomal plasmid (Gassmann, et al., Proc. Natl. Acad. Sci. USA (1995) 92:1292).

The individual strands of a dsRNA can be transcribed by promoters on two separate expression vectors and co-transfected into a target cell, Alternatively each individual strand of the dsRNA can be transcribed by promoters both of which are located on the same expression plasmid. In a preferred embodiment, a dsRNA is expressed as an inverted repeat joined by a linker polynucleotide sequence such that the dsRNA has a stem and loop structure.

The recombinant dsRNA expression vectors are generally DNA plasmids or viral vectors, dsRNA expressing viral vectors can be constructed based on, but not limited to, adeno-associated virus (for a review, see Muzyczka, et al., Curr. Topics Micro. Immunol. (1992) 158:97-129)); adenovirus (see, for example, Berkner, et al., BioTechniques (1998) 6:616), Rosenfeld et al., (1991, Science 252:431-434), and Rosenfeld et al. (1992), Cell 68:143-155)); or alphavirus as well as others known in the art. Retroviruses have been used to introduce a variety of genes into many different cell types, including epithelial cells, in vitro and/or in vivo (see, e.g., Eglitis, et al., Science (1985) 230:1395-1398: Danos and Mulligan. Proc. Natl. Acad. Sci. USA (1998) al., Science (1985) 230:1395-1398: Danos and Mulligan, Proc. Natl. Acad. Sci. USA (1998) 85:6460-6464; Wilson et al., 1988, Proc. Natl. Acad. Sci. USA 85:3014-3018; Armentano et al., 1990, Proc. Natl. Acad. Sci. USA 87:61416145; Huber et al., 1991, Proc. Natl. Acad. Sci. USA 88:8039-8043; Ferry et al., 1991, Proc. Natl. Acad. Sci. USA 88:8377-8381; Chowdhury et al., 1991, Science 254:1802-1805; van Beusechem. et al., 1992, Proc. Nad. Acad. Sci. USA 89:7640-19 ; Kay et al., 1992, Human Gene Therapy 3:641-647; Dai et al., 1992, Proc. Natl.Acad. Sci. USA 89:10892-10895; Hwu et al., 1993, J. Immunol. 150:4104-4115; U.S. Patent No. 4,868,116: U.S. Patent No. 4,980,286; PCT Application WO 89/07136; PCT Application WO 89/02468; PCT Application WO 89/05345; and PCT Application WO 92/07573). Recombinant retroviral vectors capable of transducing and expressing genes inserted into the genome of a cell can be produced by transfecting the recombinant retroviral genome into suitable packaging cell lines such as PA317 and Psi-CRIP (Cornette et al., 1991., Human Gene Therapy 2:5-10; Conc ei al., 1984, Proc. Natl. Acad. Sci. USA 81:6349). Recombinant adenoviral vectors can be used to infect a wide variety of cells and tissues in susceptible hosts (e.g., rat, hamster, dog, and chimpanzee) (Hsu et al., 1992, J. Infectious Disease, 166: 769), and also have the advantage of not requiring mitotically active cells for infection.

The promoter driving dsRNA expression in either a DNA plasmid or viral vector of the invention may be a eukaryotic RNA polymerase I (e.g. ribosomal RNA promoter), RNA polymerase II (e.g. CMV early promoter or actin promoter or U1 snRNA promoter) or generally RNA polymerase III promoter (e.g. U6 snRNA or 7SK RNA promoter) or a prokaryotic promoter, for example the T7 promoter, provided the expression plasmid also encodes T7 RNA polymerase required for transcription from a T7 promoter. The promoter can also direct transgene expression to the pancreas (see, e.g. the insulin regulatory sequence for pancreas (Bucchini et al., 1986, Proc. Natl. Acad. Sci. USA 83:2511-2515)).

In addition, expression of the transgene can be precisely regulated, for example, by using an inducible regulatory sequence and expression systems such as a regulatory sequence that its sensitive to certain physiological regulators, e.g., circulating glucose levels, or hormones (Docherty et al., 1994, FASEB J. 8:20-24). Such inclucible expression systems, suitable for the control of transgene expression in cells or in mammals include regulation by ecdysone, by estrogen, progesterone, tetracycline, chemical inducers of dimerization, and isopropyl-beta-D1 - thiogalactopyranoside (EPTG). A person skilled in the art would be able to choose the appropriate regulatory/promoter sequence based on the intended use of the dsRNA transgene.

Generally, recombinant vectors capable of expressing dsRNA molecules are delivered as described below, and persist in target cells. Alternatively, viral vectors can be used that provide for transient expression of dsRNA molecules. Such vectors can be repeatedly administered as necessary. Once expressed, the dsRNAs bind to target DNA and modulate its function or expression. Delivery of dsRNA expressing vectors can be systemic, such as by intravenous or intramuscular administration, by adminstration to target cells ex-planted from the patient followed by reintroduction into the patient, or by any other means that allows for introduction into a desired target cell.

dsRNA expression DNA plasmids are typically transfected into target cells as a complex with cationic lipid carriers (e.g. Oligofectamine) or non-rationic lipid-based carriers (e.g. Transit-TKO™). Multiple lipid transfections for dsRNA-mediated knockdowns targeting different regions of a single JC virus genome or multiple JC virus genomes over a period of a week or more are also contemplated by the invention. Successful introduction of the vectors of the invention into host cells can be monitored using various known methods. For example, transient transfection can be signaled with a reporter, such as a fluorescent marker, such as Green Fluorescent Protein (GFP). Stable transfection of ex vivo cells can be ensured using markers that provide the transfected cell with resistance to specific environmental factors (e.g.,

antibiotics and drugs), such as hygromycin B resistance.

The JC virus specific dsRNA molecules can also be inserted into vectors and used as gene therapy vectors for human patients. Gene therapy vectors can be delivered to a subject by, for example, intravenous injection, local administration (see U.S. Patent 5,328,470) or by stereotactic injection (see e.g., Chen et al. (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

**Table 1a**

**JCV Gene Walk**

siRNAs targeting >95% of all strains (>=369 out of 388)

Human specific pan-JCV: 208 siRNAs

all siRNAs double overhang design, dTdT, no modifications

| | | sense strand | | antisense strand | | | | | | | |
|---|---|---|---|---|---|---|---|---|---|---|---|
| position in consensus | duplex name | SEQ ID NO: | sequence (533') | SEQ ID NO: | sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
| 1533-1551 | AD-14742 | 515 | CUUAUAAGAGGAGGAGUAGTT | 516 | CUACUCCUCCUCUUAUAAGTT | **40.85** | 4.38 | 41 ± 4% | **82.24** | 8.83 | 82 ± 9 % |
| 1703-1721 | AD-14743 | 517 | CAUGCUUCCUUGUUACAGUTT | 518 | ACUGUAACAAGGAAGCAUGTT | **20.92** | 4.10 | 21 ± 4% | **109.97** | 21.56 | 110 ± 22 % |
| 1439-1457 | AD-14744 | 519 | UACGGGACUGUAACACCUGTT | 520 | CAGGUGUUACAGUCCCGUATT | **62.20** | 4.47 | 62 ± 4 % | **52.56** | 3.78 | 53 ± 4 % |
| 1705-1723 | AD-14745 | 521 | UGCUUCCUUGUUACAGUGUTT | 522 | ACACUGUAACAAGGAAGCATT | **43.97** | 2.60 | 44 ± 3 % | **77.91** | 4.60 | 78 ± 5 % |
| 2064-2082 | AD-14746 | 523 | CCUGUUGAAUGUUGGGUUCTT | 524 | GAACCCAACAUUCAACAGGTT | **24.52** | 1.96 | 25 ± 2 % | **104.96** | 8.38 | 105 ± 8 % |
| 2067-2085 | AD-14747 | 525 | GUUGAAUGUUGGGUUCCUGTT | 526 | CAGGAACCCAACAUUCAACTT | **32.67** | 4.51 | 33 ± 5 % | **93.62** | 12.94 | 94 ± 13 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 2071-2089 | AD-14748 | 527 | AAUGUUGGGUUCCUGAUCCTT | 528 | GGAUCAGGAACCCAACAUUTT | **93.99** | 3.23 | 94 ± 3 % | **8.36** | 0.29 | 8 ± 0 % |
| 2121-2139 | AD-14749 | 529 | ACACUAACAGGAGGAGAAATT | 530 | UUUCUCCUCCUGUUAGUGUTT | **55.16** | 2.81 | 55 ± 3 % | **62.35** | 3.18 | 62 ± 3 % |
| 1535-1553 | AD-14750 | 531 | UAUAAGAGGAGGAGUAGAATT | 532 | UUCUACUCCUCCUCUUAUATT | **30.86** | 3.11 | 31 ± 3 % | **96.14** | 9.70 | 96 ± 10 % |
| 1536-1554 | AD-14751 | 533 | AUAAGAGGAGGAGUAGAAGTT | 534 | CUUCUACUCCUCCUCUUAUTT | **54.44** | 4.03 | 54 ± 4 % | **63.35** | 4.69 | 63 ± 5 % |
| 1445-1463 | AD-14752 | 535 | ACUGUAACACCUGCUCUUGTT | 536 | CAAGAGCAGGUGUUACAGUTT | **53.88** | 7.58 | 54 ± 8 % | **64.13** | 9.02 | 64 ± 9 % |
| 1700-1718 | AD-14753 | 537 | GGACAUGCUUCCUUGUUACTT | 538 | GUAACAAGGAAGCAUGUCCTT | **35.24** | 7.45 | 35 ± 7 % | **90.05** | 19.03 | 90 ± 19 % |
| 1702-1720 | AD-14754 | 539 | ACAUGCUUCCUUGUUACAGTT | 540 | CUGUAACAAGGAAGCAUGUTT | **70.39** | 2.80 | 70 ± 3 % | **41.17** | 1.64 | 41 ± 2 % |
| 1704-1722 | AD-14755 | 541 | AUGCUUCCUUGUUACAGUGTT | 542 | CACUGUAACAAGGAAGCAUTT | **41,80** | 1.60 | 42 ± 2 % | **80.93** | 3.10 | 81 ± 3 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 2065-2083 | AD-14756 | 543 | CUGUUGAAUGUUGGGUUCCTT | 544 | GGAACCCAACAUUCAACAGTT | **56.69** | 3.05 | 57 ± 3 % | **60.22** | 3.24 | 60 ± 3 % |
| 2070-2088 | AD-14757 | 545 | GAAUGUUGGGUUCCUGAUCTT | 546 | GAUCAGGAACCCAACAUUCTT | **39.16** | 2.16 | 39 ± 2 % | **84.60** | 4.67 | 85 ± 5 % |
| 1441-1459 | AD-14758 | 547 | CGGGACUGUAACACCUGCUTT | 548 | AGCAGGUGUUACAGUCCCGTT | **39.79** | 2.95 | 40 ± 3 % | **83.72** | 6.22 | 84 ± 6 % |
| 1443-1461 | AD-14759 | 549 | GGACUGUAACACCUGCUCUTT | 550 | AGAGCAGGUGUUACAGUCCTT | **30.62** | 1.01 | 31 ± 1% | **96.48** | 3.20 | 96 ± 3 % |
| 1444-1462 | AD-14760 | 551 | GACUGUAACACCUGCUCUUTT | 552 | AAGAGCAGGUGUUACAGUCTT | **28.14** | 2.74 | 28 ± 3 % | **99.93** | 9.72 | 100 ± 10 % |
| 1609-1627 | AD-14761 | 553 | CUCCAGAAAUGGGUGACCCTT | 554 | GGGUCACCCAUUUCUGGAGTT | **67.42** | 2.83 | 67 ± 3 % | **45.30** | 1.90 | 45 ± 2 % |
| 1537-1555 | AD-14762 | 555 | UAAGAGGAGGAGUAGAAGUTT | 556 | ACUUCUACUCCUCCUCUUATT | **36.10** | 1.30 | 36 ± 1% | **88.85** | 3.21 | 89 ± 3 % |
| 629-647 | AD-14763 | 557 | GAGGCUGCUGCUACUAUAGTT | 558 | CUAUAGUAGCAGCAGCCUCTT | **49.39** | 6.77 | 49 ± 7 % | **78.14** | 10.71 | 78 ± 11 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 656-674 | AD-14764 | 559 | AUUGCAUCCCUUGCUACUGTT | 560 | CAGUAGCAAGGGAUGCAAUTT | **74.04** | 5.32 | 74 ± 5 % | **40.09** | 2.88 | 40 ± 3 % |
| 658-676 | AD-14765 | 561 | UGCAUCCCUUGCUACUGUATT | 562 | UACAGUAGCAAGGGAUGCATT | **50.84** | 10.47 | 51 ± 10 % | **75.91** | 15.63 | 76 ± 16 % |
| 517-535 | AD-14766 | 563 | UUGUGUUUUCAGGUUCAUGTT | 564 | CAUGAACCUGAAAACACAATT | **72.59** | 3.55 | 73 ± 4 % | **42.32** | 2.07 | 42 ± 2 % |
| 559-577 | AD-14767 | 565 | GGACCUAGUUGCUACUGUUTT | 566 | AACAGUAGCAACUAGGUCCTT | **34.82** | 7.41 | 35 ± 7 % | **100.63** | 21.40 | 101 ± 21 % |
| 591-609 | AD-14768 | 567 | CUGCCACAGGAUUUUCAGUTT | 568 | ACUGAAAAUCCUGUGGCAGTT | **48.68** | 6.31 | 49 ± 6 % | **79.24** | 10.27 | 79 ± 10 % |
| 638-656 | AD-14769 | 569 | GCUACUAUAGAAGUUGAAATT | 570 | UUUCAACUUCUAUAGUAGCTT | **39.07** | 5.53 | 39 ± 6 % | **94.08** | 13.31 | 94 ± 13 % |
| 655-673 | AD-14770 | 571 | AAUUGCAUCCCUUGCUACUTT | 572 | AGUAGCAAGGGAUGCAAUUTT | **45.59** | 5.89 | 46 ± 6 % | **84.01** | 10.85 | 84 ± 11 % |
| 561-579 | AD-14771 | 573 | ACCUAGUUGCUACUGUUUCTT | 574 | GAAACAGUAGCAACUAGGUTT | **45.57** | 4.10 | 46 ± 4 % | **84.04** | 7.56 | 84 ± 8 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 639-657 | AD-14772 | 575 | CUACUAUAGAAGUUGAAAUTT | 576 | AUUUCAACUUCUAUAGUAGTT | **33.12** | 3.64 | 33 ± 4 % | **103.26** | 11.36 | 103 ± 11 % |
| 715-733 | AD-14773 | 577 | AGGCCUUACUCCUGAAACATT | 578 | UGUUUCAGGAGUAAGGCCUTT | **37.38** | 5.72 | 37 ± 6 % | **96.69** | 14.78 | 97 ± 15 % |
| 716-734 | AD-14774 | 579 | GGCCUUACUCCUGAAACAUTT | 580 | AUGUUUCAGGAGUAAGGCCTT | **42.38** | 4.41 | 42 ± 4 % | **88.96** | 9.26 | 89 ± 9 % |
| 326-344 | AD-14775 | 581 | GUAAAACCUGGAGUGGAACTT | 582 | GUUCCACUCCAGGUUUUACTT | **46.59** | 3.00 | 47 ± 3 % | **82.47** | 5.31 | 82 ± 5 % |
| 518-536 | AD-14776 | 583 | UGUGUUUUCAGGUUCAUGGTT | 584 | CCAUGAACCUGAAAACACATT | **71.28** | 8.67 | 71 ± 9 % | **44.35** | 5.40 | 44 ± 5 % |
| 520-538 | AD-14777 | 585 | UGUUUUCAGGUUCAUGGGUTT | 586 | ACCCAUGAACCUGAAAACATT | **84.55** | 6.21 | 65 ± 6 % | **54.74** | 5.26 | 55 ± 5 % |
| 661-679 | AD-14778 | 587 | AUCCCUUGCUACUGUAGAGTT | 588 | CUCUACAGUAGCAAGGGAUTT | **60.45** | 8.91 | 60 ± 9 % | **61.07** | 9.00 | 61 ± 9 % |
| 560-578 | AD-14779 | 589 | GACCUAGUUGCUACUGUUUTT | 590 | AAACAGUAGCAACUAGGUCTT | **32.46** | 0.82 | 32 ± 1 % | **104.27** | 2.63 | 104 ± 3 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 681-699 | AD-14780 | 591 | GGAUUACAAGUACCUCUGATT | 592 | UCAGAGGUACUUGUAAUCCTT | **22.96** | 2.86 | 23 ± 3 % | **118.94** | 14.81 | 119 ± 15 % |
| 714-732 | AD-14781 | 593 | UAGGCCUUACUCCUGAAACTT | 594 | GUUUCAGGAGUAAGGCCUATT | **56.99** | 9.43 | 57 ± 9 % | **66.41** | 10.99 | 66 ± 11 % |
| 377-395 | AD-14782 | 595 | UGUUAGAAUUUUUGCUGGATT | 596 | UCCAGCAAAAAUUCUAACATT | **29.90** | 8.74 | 30 ± 9 % | **108.24** | 31.65 | 108 ± 32 % |
| 589-607 | AD-14783 | 597 | UGCUGCCACAGGAUUUUCATT | 598 | UGAAAAUCCUGUGGCAGCATT | **42.63** | 6.57 | 43 ± 7 % | **88.58** | 13.66 | 89 ± 14 % |
| 594-612 | AD-14784 | 599 | CCACAGGAUUUUCAGUAGCTT | 600 | GCUACUGAAAAUCCUGUGGTT | **67.06** | 1.35 | 67 ± 1 % | **50.86** | 1.03 | 51 ± 1 % |
| 648-666 | AD-14785 | 601 | AAGUUGAAAUUGCAUCCCUTT | 602 | AGGGAUGCAAUUUCAACUUTT | **48.90** | 3.32 | 49 ± 3 % | **78.89** | 5.35 | 79 ± 5 % |
| 649-667 | AD-14786 | 603 | AGUUGAAAUUGCAUCCCUUTT | 604 | AAGGGAUGCAAUUUCAACUTT | **27.74** | 2.06 | 28 ± 2 % | **111.57** | 8.29 | 112 ± 8 % |
| 587-605 | AD-14787 | 605 | GCUGCUGCCACAGGAUUUUTT | 606 | AAAAUCCUGUGGCAGCAGCTT | **38.77** | 6.24 | 39 ± 6 % | **94.53** | 15.22 | 95 ± 15 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 325-343 | AD-14788 | 607 | AGUAAAACCUGGAGUGGAATT | 608 | UUCCACUCCAGGUUUUACUTT | **32.84** | 8.60 | 33 ± 9 % | **103.70** | 27.17 | 104 ± 27 % |
| 515-533 | AD-14789 | 609 | UUUUGUGUUUUCAGGUUCATT | 610 | UGAACCUGAAAACACAAAATT | **46.96** | 1.70 | 47 ± 2 % | **81.89** | 2.96 | 82 ± 3 % |
| 516-534 | AD-14790 | 611 | UUUGUGUUUUCAGGUUCAUTT | 612 | AUGAACCUGAAAACACAAATT | **43.61** | 4.90 | 44 ± 5 % | **87.06** | 9.79 | 87 ± 10 % |
| 519-537 | AD-14791 | 613 | GUGUUUUCAGGUUCAUGGGTT | 614 | CCCAUGAACCUGAAAACACTT | **35.55** | 4.34 | 36 ± 4 % | **99.51** | 12.15 | 100 ± 12 % |
| 521-539 | AD-14792 | 615 | GUUUUCAGGUUCAUGGGUGTT | 616 | CACCCAUGAACCUGAAAACTT | **38.22** | 3.51 | 38 ± 4 % | **95.38** | 8.75 | 95 ± 9 % |
| 522-540 | AD-14793 | 617 | UUUUCAGGUUCAUGGGUGCTT | 618 | GCACCCAUGAACCUGAAAATT | **90.85** | 5.92 | 91 ± 6 % | **14.13** | 0.92 | 14 ± 1 % |
| 523-541 | AD-14794 | 619 | UUUCAGGUUCAUGGGUGCCTT | 620 | GGCACCCAUGAACCUGAAATT | **83.37** | 3.27 | 83 ± 3 % | **25.68** | 1.01 | 26 ± 1 % |
| 616-634 | AD-14795 | 621 | AAUUGCUGCUGGAGAGGCUTT | 622 | AGCCUCUCCAGCAGCAAUUTT | **55.06** | 3.61 | 55 ± 4 % | **69.38** | 4.55 | 69 ± 5 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 657-675 | AD-14796 | 623 | UUGCAUCCCUUGCUACUGUTT | 624 | ACAGUAGCAAGGGAUGCAATT | **30.98** | 5.78 | 31 ± 6 % | **106.56** | 19.89 | 107 ± 20 % |
| 761-779 | AD-14797 | 625 | GCUGUAGCUGGGUUUGCUGTT | 626 | CAGCAAACCCAGCUACAGCTT | **28.95** | 3.15 | 29 ± 3 % | **109.70** | 11.95 | 110 ± 12 % |
| 645-663 | AD-14798 | 627 | UAGAAGUUGAAAUUGCAUCTT | 628 | GAUGCAAUUUCAACUUCUATT | **67.39** | 3.70 | 67 ± 4 % | **50.35** | 2.76 | 50 ± 3 % |
| 647-665 | AD-14799 | 629 | GAAGUUGAAAUUGCAUCCCTT | 630 | GGGAUGCAAUUUCAACUUCTT | **66.83** | 4.72 | 67 ± 5 % | **51.21** | 3.61 | 51 ± 4 % |
| 660-678 | AD-14800 | 631 | CAUCCCUUGCUACUGUAGATT | 632 | UCUACAGUAGCAAGGGAUGTT | **33.26** | 5.72 | 33 ± 6 % | **103.04** | 17.71 | 103 ± 18 % |
| 324-342 | AD-14801 | 633 | UAGUAAAACCUGGAGUGGATT | 634 | UCCACUCCAGGUUWACUATT | **39.15** | 4.57 | 39 ± 5 % | **93.96** | 10.97 | 94 ± 11 % |
| 372-390 | AD-14802 | 635 | UUUUUUGUUAGAAUUUUUGTT | 636 | CAAAAAUUCUAACAAAAAATT | **91.20** | 5.35 | 91 ± 5 % | **13.58** | 0.80 | 14 ± 1 % |
| 640-658 | AD-14803 | 637 | UACUAUAGAAGUUGAAAUUTT | 638 | AAUUUCAACUUCUAUAGUATT | **34.15** | 7.94 | 34 ± 8 % | **101.67** | 23.64 | 102 ± 24 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 562-580 | AD-14804 | 639 | CCUAGUUGCUACUGUUUCUTT | 640 | AGAAACAGUAGCAACUAGGTT | **30.08** | 6.54 | 30 ± 7 % | **107.96** | 23.48 | 108 ± 23 % |
| 563-581 | AD-14805 | 641 | CUAGUUGCUACUGUUUCUGTT | 642 | CAGAAACAGUAGCAACUAGTT | **32.44** | 4.27 | 32 ± 4 % | **104.31** | 13.73 | 104 ± 14 % |
| 566-584 | AD-14806 | 643 | GUUGCUACUGUUUCUGAGGTT | 644 | CCUCAGAAACAGUAGCAACTT | **35.62** | 3.11 | 36 ± 3 % | **99.41** | 8.67 | 99 ± 9 % |
| 625-643 | AD-14807 | 645 | UGGAGAGGCUGCUGCUACUTT | 646 | AGUAGCAGCAGCCUCUCCATT | **28.27** | 7.28 | 28 ± 7 % | **110.76** | 28.52 | 111 ± 29 % |
| 627-645 | AD-14808 | 647 | GAGAGGCUGCUGCUACUAUTT | 648 | AUAGUAGCAGCAGCCUCUCTT | **30.29** | 3.96 | 30 ± 4 % | **107.63** | 14.08 | 108 ± 14 % |
| 628-646 | AD-14809 | 649 | AGAGGCUGCUGCUACUAUATT | 650 | UAUAGUAGCAGCAGCCUCUTT | **31.59** | 4.46 | 32 ± 4 % | **105.63** | 14.91 | 106 ± 15 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 632-650 | AD-14810 | 651 | GCUGCUGCUACUAUAGAAGTT | 652 | CUUCUAUAGUAGCAGCAGCTT | **30.11** | 5.71 | 30 ± 6 % | **107.91** | 20.46 | 108 ± 20 % |
| 513-531 | AD-14811 | 653 | UUUUUUGUGUUUUCAGGUUTT | 654 | AACCUGAAAACACAAAAAATT | **55.27** | 6.82 | 55 ± 7 % | **69.06** | 8.52 | 69 ± 9 % |
| 641-659 | AD-14812 | 655 | ACUAUAGAAGUUGAAAUUGTT | 656 | CAAUUUCAACUUCUAUAGUTT | **45.27** | 5.99 | 45 ± 6 % | **84.51** | 11.19 | 85 ± 11 % |
| 323-341 | AD-14813 | 657 | UUAGUAAAACCUGGAGUGGTT | 658 | CCACUCCAGGUUUUACUAATT | **77.97** | 7.01 | 78 ± 7 % | **34.01** | 3.06 | 34 ± 3 % |
| 717-735 | AD-14814 | 659 | GCCUUACUCCUGAAACAUATT | 660 | UAUGUUUCAGGAGUAAGGCTT | **29.54** | 3.56 | 30 ± 4 % | **108.78** | 13.09 | 109 ± 13 % |
| 646-664 | AD-14815 | 661 | AGAAGUUGAAAUUGCAUCCTT | 662 | GGAUGCAAUUUCAACUUCUTT | **65.04** | 3.18 | 65 ± 3 % | **53.97** | 2.64 | 54 ± 3 % |
| 592-610 | AD-14816 | 663 | UGCCACAGGAUUUUCAGUATT | 664 | UACUGAAAAUCCUGUGGCATT | **64.03** | 4.63 | 64 ± 5 % | **55.53** | 4.02 | 56 ± 4 % |
| 590-608 | AD-14817 | 665 | GCUGCCACAGGAUUUUCAGTT | 666 | CUGAAAAUCCUGUGGCAGCTT | **37.83** | 2.89 | 38 ± 3 % | **95.99** | 7.33 | 96 ± 7 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 526-544 | AD-14818 | 667 | CAGGUUCAUGGGUGCCGCATT | 668 | UGCGGCACCCAUGAACCUGTT | **28.88** | 5.60 | 29 ± 6 % | **109.82** | 21.30 | 110 ± 21 % |
| 615-633 | AD-14819 | 669 | AAAUUGCUGCUGGAGAGGCTT | 670 | GCCUCUCCAGCAGCAAUUUTT | **92.90** | 4.87 | 93 ± 5 % | **10.97** | 0.58 | 11 ± 1 % |
| 617-635 | AD-14820 | 671 | AUUGCUGCUGGAGAGGCUGTT | 672 | CAGCCUCUCCAGCAGCAAUTT | **75.41** | 3.69 | 75 ± 4 % | **37.97** | 1.86 | 38 % 2 % |
| 652-670 | AD-14821 | 673 | UGAAAUUGCAUCCCUUGCUTT | 674 | AGCAAGGGAUGCAAUUUCATT | **73.08** | 6.22 | 73 ± 6 % | **41.57** | 3.54 | 42 ± 4 % |
| 374-392 | AD-14822 | 675 | UUUUGUUAGAAUUUUUGCUTT | 676 | AGCAAAAAUUCUAACAAAATT | **86.39** | 9.34 | 86 ± 9 % | **21.02** | 2.27 | 21 ± 2 % |
| 375-393 | AD-14823 | 677 | UUUGUUAGAAUUUUUGCUGTT | 678 | CAGCAAAAAUUCUAACAAATT | **96.50** | 10.46 | 97 ± 10 % | **5.40** | 0.59 | 5 ± 1 % |
| 631-649 | AD-14824 | 679 | GGCUGCUGCUACUAUAGAATT | 680 | UUCUAUAGUAGCAGCAGCCTT | **32.62** | 3.41 | 33 ± 3 % | **104.03** | 10.89 | 104 ± 11 % |
| 376-394 | AD-14825 | 681 | UUGUUAGAAUUUUUGCUGGTT | 682 | CCAGCAAAAAUUCUAACAATT | **102.71** | 7.66 | 103 ± 8 % | **-4.18** | 0.31 | -4 ± 0 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 512-530 | AD-14826 | 683 | UUUUUUUGUGUUUUCAGGUTT | 684 | ACCUGAAAACACAAAAAAATT | **92.45** | 5.66 | 92 ± 6 % | **11.66** | 0.71 | 12 ± 1 % |
| 1127-1145 | AD-14827 | 685 | GAAACUACUUGGGCAAUAGTT | 686 | CUAUUGCCCAAGUAGUUUCTT | **63.46** | 16.38 | 63 ± 16 % | **46.00** | 11.88 | 46 ± 12 % |
| 1410-1428 | AD-14828 | 687 | AAUGGAUGUUGCCUUUACUTT | 688 | AGUAAAGGCAACAUCCAUUTT | 45.99 | 15.21 | 46 ± 15 % | 67.99 | 22.49 | 68 ± 22 % |
| 1406-1424 | AD-14829 | 689 | CCUCAAUGGAUGUUGCCUUTT | 690 | AAGGCAACAUCCAUUGAGGTT | 40.54 | 16.03 | 41 ± 16 % | 74.86 | 29.60 | 75 ± 30 % |
| 1418-1436 | AD-14830 | 691 | UUGCCUUUACUUUUAGGGUTT | 692 | ACCCUAAAAGUAAAGGCAATT | 117.10 | 3.66 | 117 ± 4% | -21.52 | 0.67 | -22 ± 1 % |
| 1126-1144 | AD-14831 | 693 | AGAAACUACUUGGGCAAUATT | 694 | UAUUGCCCAAGUAGUUUCUTT | 54.78 | 21.12 | 55 ± 21 % | 56.93 | 21.95 | 57 ± 22 % |
| 1125-1143 | AD-14832 | 695 | AAGAAACUACUUGGGCAAUTT | 696 | AUUGCCCAAGUACUUUCUUTT | 67.07 | 10.81 | 67 ± 11 % | 41.46 | 6.68 | 41 ± 7 % |
| 1419-1437 | AD-14833 | 697 | UGCCUUUACUUUUAGGGUUTT | 698 | AACCCUAAAAGUAAAGGCATT | 71.52 | 11.90 | 72 ± 12 % | 35.85 | 5.97 | 36 ± 6 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 1420-1438 | AD-14834 | 699 | GCCUUUACUUUUAGGGUUGTT | 700 | CAACCCUAAAAGUAAAGGCTT | 58.05 | 16.37 | 58 ± 16 % | 52.81 | 14.89 | 53 ± 15 % |
| 1422-1440 | AD-14835 | 701 | CUUUACUUUUAGGGUUGUATT | 702 | UACAACCCUAAAAGUAAAGTT | 83.36 | 5.43 | 93 ± 5 % | 8.36 | 0.49 | 8 ± 0 % |
| 1423-1441 | AD-14836 | 703 | UUUACUUUUAGGGUUGUACTT | 704 | GUACAACCCUAAAAGUAAATT | 108.84 | 4.85 | 109 ± 5 % | -11.13 | 0.50 | -11 ± 0 % |
| 1425-1443 | AD-14837 | 705 | UACUUUUAGGGUUGUACGGTT | 706 | CCGUACAACCCUAAAAGUATT | 106.68 | 10.06 | 107 ± 10 % | -8.41 | 0.79 | -8 ± 1 % |
| 1123-1141 | AD-14838 | 707 | GGAAGAAACUACUUGGGCATT | 708 | UGCCCAAGUAGUUUCUUCCTT | 37.06 | 6.68 | 37 ± 7 % | 79.23 | 14.28 | 79 ± 14 % |
| 1409-1427 | AD-14839 | 709 | CAAUGGAUGUUGCCUUUACTT | 710 | GUAAAGGCAACAUCCAUUGTT | 36.03 | 7.54 | 36 ± 8 % | 80.53 | 16.84 | 81 ± 17 % |
| 1413-1431 | AD-14840 | 711 | GGAUGUUGCCUUUACUUUUTT | 712 | AAAAGUAAAGGCAACAUCCTT | 38.51 | 5.90 | 39 ± 6% | 77.40 | 11.86 | 77 ± 12 % |
| 1416-1434 | AD-14841 | 713 | UGUUGCCUUUACUUUUAGGTT | 714 | CCUAAAAGUAAAGGCAACATT | 110.86 | 8.91 | 111 ± 9 % | -13.67 | 1.10 | -14 ± 1 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 1414-1432 | AD-14842 | 715 | GAUGUUGCCUUUACUUUUATT | 716 | UAAAAGUAAAGGCAACAUCTT | 34.83 | 5.51 | 35 ± 6 % | 82.04 | 12.98 | 82 ± 13 % |
| 911-929 | AD-14843 | 717 | CCAGAAGACUACUAUGAUATT | 718 | UAUCAUAGUAGUCUUCUGGTT | 23.75 | 6.04 | 24 ± 6 % | 95.99 | 24.41 | 96 ± 24 % |
| 910-928 | AD-14844 | 719 | UCCAGAAGACUACUAUGAUTT | 720 | AUCAUAGUAGUCUUCUGGATT | 27.47 | 5.29 | 27 ± 5 % | 91.30 | 17.57 | 91 ± 18 % |
| 1120-1138 | AD-14845 | 721 | UUUGGAAGAAACUACUUGGTT | 722 | CCAAGUAGUUUCUUCCAAATT | 93.12 | 4.70 | 93 ± 5 % | 8.67 | 0.44 | 9 ± 0 % |
| 1404-1422 | AD-14846 | 723 | CUCCUCAAUGGAUGUUGCCTT | 724 | GGCAACAUCCAUUGAGGAGTT | 81.72 | 8.26 | 82 ± 8 % | 23.01 | 2.33 | 23 ± 2 % |
| 1337-1355 | AD-14847 | 725 | CCAAAUGUGCAAUCUGGUGTT | 726 | CACCAGAUUGCACAUUUGGTT | 77.88 | 5.29 | 78 ± 5 % | 27.83 | 1.89 | 28 ± 2 % |
| 1338-1356 | AD-14848 | 727 | CAAAUGUGCAAUCUGGUGATT | 728 | UCACCAGAUUGCACAUUUGTT | 44.40 | 4.95 | 44 ± 5 % | 69.99 | 7.81 | 70 ± 8 % |
| 1397-1415 | AD-14849 | 729 | AGAUCUGCUCCUCAAUGGATT | 730 | UCCAUUGAGGAGCAGAUCUTT | 46.41 | 5.08 | 46 ± 5 % | 67.46 | 7.38 | 67 ± 7 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 1407-1425 | AD-14850 | 731 | CUCAAUGGAUGUUGCCUUUTT | 732 | AAAGGCAACAUCCAUUGAGTT | 35.52 | 6.70 | 36 17 % | 81.17 | 15.31 | 81 ± 15 % |
| 4157-4175 | AD-14851 | 733 | GCUCAAAUUUUAUAUAAGATT | 734 | UCUUAUAUAAAAUUUGAGCTT | 36.07 | | 1.13 36 ± 1 % | 102.63 | 3.22 | 103 ± 3 % |
| 4795-4813 | AD-14852 | 735 | AGCCUGAUUUUGGUACAUGTT | 736 | CAUGUACCAAAAUCAGGCUTT | 67.98 | 6.75 | 68 ± 7 % | 51.41 | 5.11 | 51 ± 5 % |
| 4156-4174 | AD-14853 | 737 | CUCAAAUUUUAUAUAAGAATT | 738 | UUCUUAUAUAAAAUUUGAGTT | 69.44 | 3.07 | 69 ± 3 % | 49.05 | 2.17 | 49 ± 2 % |
| 5002-5020 | AD-14854 | 739 | ACAAAGUGCUGAAUAGGGATT | 740 | UCCCUAUUCAGCACUUUGUTT | 29.12 | 6.88 | 29 ± 7 % | 113.79 | 26.89 | 114 ± 27 % |
| 4792-4810 | AD-14855 | 741 | CUGAUUUUGGUACAUGGAATT | 742 | UUCCAUGUACCAAAAUCAGTT | 36.04 | 7.07 | 36 ± 7 % | 102.68 | 20.14 | 103 ± 20 % |
| 4790-4808 | AD-14856 | 743 | GAUUUUGGUACAUGGAAUATT | 744 | UAUUCCAUGUACCAAAAUCTT | 33.61 | 7.93 | 34 ± 8 % | 106.57 | 25.15 | 107 ± 25 % |
| 4801-4819 | AD-14857 | 745 | CUCAUCAGCCUGAUUUUGGTT | 746 | CCAAAAUCAGGCUGAUGAGTT | 50.76 | 8.76 | 51 ± 9 % | 79.04 | 13.64 | 79 ± 14 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 4622-4640 | AD-14858 | 747 | AGCCCACUUGUGUGGAUAGTT | 748 | CUAUCCACACAAGUGGGCUTT | 53.60 | 7.26 | 54 ± 7 % | 74.49 | 10.09 | 74 ± 10 % |
| 4997-5015 | AD-14859 | 749 | GUGCUGAAUAGGGAGGAAUTT | 750 | AUUCCUCCCUAUUCAGCACTT | 39.07 | 9.34 | 39 ± 9 % | 97.82 | 23.38 | 98 ± 23 % |
| 5094-5112 | AD-14860 | 751 | AGUAAGGGCGUGGAGGCUUTT | 752 | AAGCCUCCACGCCCUUACUTT | 62.78 | 6.85 | 63 ± 7 % | 59.75 | 6.52 | 60 ± 7 % |
| 4564-4582 | AD-14861 | 753 | GUGACUUAACCCAAGAAGCTT | 754 | GCUUCUUGGGUUAAGUCACTT | 87.47 | 1.86 | 87 ± 2 % | 20.12 | 0.43 | 20 ± 0 % |
| 5095-5113 | AD-14862 | 755 | UAGUAAGGGCGUGGAGGCUTT | 756 | AGCCUCCACGCCCUUACUATT | 79.95 | 4.02 | 80 ± 4% | 32.19 | 1.62 | 32 ± 2% |
| 4800-4818 | AD-14863 | 757 | UCAUCAGCCUGAUUUUGGUTT | 758 | ACCAAAAUCAGGCUGAUGATT | 30.46 | 4.49 | 30 ± 4% | 111.64 | 16.46 | 112 ± 16 % |
| 4265-4283 | AD-14864 | 759 | GUAGAAGACCCUAAAGACUTT | 760 | AGUCUUUAGGGUCUUCUACTT | 33.18 | 5.07 | 33 ± 5% | 107.26 | 16.38 | 107 ± 16 % |
| 4267-4285 | AD-14865 | 761 | AGGUAGAAGACCCUAAAGATT | 762 | UCUUUAGGGUCUUCUACCUTT | 26.25 | 3.98 | 26 ± 4% | 118.39 | 17.96 | 118 ± 18 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 4270-4288 | AD-14866 | 763 | AAAAGGUAGAAGACCCUAATT | 764 | UUAGGGUCUUCUACCUUUUTT | 36.73 | 1.24 | 37 ± 1% | 101.57 | 3.44 | 102 ± 3% |
| 4269-4287 | AD-14867 | 765 | AAAGGUAGAAGACCCUAAATT | 766 | UUUAGGGUCUUCUACCUUUTT | 33.16 | 3.13 | 33 ± 3% | 107.30 | 10.12 | 107±10 % |
| 2874-2892 | AD-14868 | 767 | GAUUGUGCAGUGGAAAGAATT | 768 | UUCUUUCCACUGCACAAUCTT | 29.91 | 4.56 | 30 ± 5% | 112.52 | 17.16 | 113±17 % |
| 2875-2893 | AD-14869 | 769 | GGAUUGUGCAGUGGAAAGATT | 770 | UCUUUCCACUGCACAAUCCTT | 28.24 | 3.66 | 28 ± 4% | 115.20 | 14.91 | 115 ± 15 % |
| 3950-3968 | AD-14870 | 771 | UGUAGACAGCCAUAUGCAGTT | 772 | CUGCAUAUGGCUGUCUACATT | 50.37 | 3.04 | 50 ± 3% | 79.67 | 4.81 | 80 ± 5% |
| 3896-3914 | AD-14871 | 773 | CAUGACUUUAACCCAGAAGTT | 774 | CUUCUGGGUUAAAGUCAUGTT | 39.37 | 5.11 | 39 ± 5% | 97.32 | 12.63 | 97 ± 13% |
| 4990-5008 | AD-14872 | 775 | AUAGGGAGGAAUCCAUGGATT | 776 | UCCAUGGAUUCCUCCCUAUTT | 34.71 | 4.12 | 35 ± 4% | 104.82 | 12.43 | 105 ± 12 % |
| 4994-5012 | AD-14873 | 777 | CUGAAUAGGGAGGAAUCCATT | 778 | UGGAUUCCUCCCUAUUCAGTT | 32.14 | 1.79 | 32 ± 2% | 108.93 | 6.07 | 109 ± 6% |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 5000-5018 | AD-14874 | 779 | AAAGUGCUGAAUAGGGAGGTT | 780 | CCUCCCUAUUCAGCACUUUTT | 101.77 | 4.87 | 102 ± 5% | -2.85 | 0.14 | -3 ± 0% |
| 4563-4581 | AD-14875 | 781 | UGACUUAACCCAAGAAGCUTT | 782 | AGCUUCUUGGGUUAAGUCATT | 80.81 | 4.39 | 81 ± 4% | 30.80 | 1.67 | 31 ± 2% |
| 3895-3913 | AD-14876 | 783 | AUGACUUUAACCCAGAAGATT | 784 | UCWCUGGGWAAAGUCAUTT | 30.74 | 1.88 | 31 ± 2% | 111.18 | 6.81 | 111 ± 7% |
| 4262-4280 | AD-14877 | 785 | GAAGACCCUAAAGACUUUCTT | 786 | GAAAGUCUUUAGGGUCUUCTT | 57.38 | 2.84 | 57 ± 3% | 68.42 | 3.39 | 68 ± 3% |
| 4162-4180 | AD-14878 | 787 | AAAAAGCUCAAAUUUUAUATT | 788 | UAUAAAAUUUGAGCUUUUUTT | 70.23 | 3.35 | 70 ± 3% | 47.79 | 2.28 | 48 ± 2% |
| 4798-4816 | AD-14879 | 769 | AUCAGCCUGAUUUUGGUACTT | 790 | GUACCAAAAUCAGGCUGAUTT | 79.03 | 7.72 | 79 ± 6% | 33.66 | 3.29 | 34 ± 3% |
| 4799-4817 | AD-14880 | 791 | CAUCAGCCUGAUUUUGGUATT | 792 | UACCAAAAUCAGGCUGAUGTT | 21.65 | 2.46 | 22 ± 2% | 125.78 | 14.28 | 126 ± 14 % |
| 5006-5024 | AD-14881 | 793 | AUGGACAAAGUGCUGAAUATT | 794 | UAUUCAGCACUUUGUCCAUTT | 27.66 | 1.71 | 28 ± 2% | 116.13 | 7.17 | 116 ± 7% |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 4264-4282 | AD-14882 | 795 | UAGAAGACCCUAAAGACWTT | 796 | AAGUCUUUAGGGUCUUCUATT | 34.01 | 2.94 | 34 ± 3% | 105.93 | 9.16 | 106 ± 9% |
| 4268-4286 | AD-14863 | 797 | AAGGUAGAAGACCCUAAAGTT | 798 | CUUUAGGGUCUUCUACCUUTT | 40.62 | 3.22 | 41 ± 3% | 95.33 | 7.56 | 95 ± 8% |
| 4623-4641 | AD-14884 | 799 | CAGCCCACUUGUGUGGAUATT | 800 | UAUCCACACAAGUGGGCUGTT | 35.73 | 5.94 | 36 ± 6% | 103.18 | 17.14 | 103 ± 17 % |
| 4788-4806 | AD-14885 | 801 | UUUUGGUACAUGGAAUAGUTT | 802 | ACUAUUCCAUGUACCAAAATT | 47.40 | 7.65 | 47 ± 6% | 84.45 | 13.63 | 84 ± 14% |
| 4993-5011 | AD-14886 | 803 | UGAAUAGGGAGGAAUCCAUTT | 804 | AUGGAUUCCUCCCUAUUCATT | 37.23 | 3.94 | 37 ± 4% | 100.76 | 10.67 | 101 ± 11 % |
| 4995-5013 | AD-14887 | 805 | GCUGAAUAGGGAGGAAUCCTT | 806 | GGAUUCCUCCCUAUUCAGCTT | 42.94 | 7.26 | 43 ± 7% | 91.61 | 15.50 | 92 ± 15% |
| 4996-5014 | AD-14888 | 807 | UGCUGAAUAGGGAGGAAUCTT | 808 | GAUUCCUCCCUAUUCAGCATT | 32.58 | 4.06 | 33 ± 4% | 108.24 | 13.50 | 108 ± 14 % |
| 3952-3970 | AD-14889 | 809 | UGUGUAGACAGCCAUAUGCTT | 810 | GCAUAUGGCUGUCUACACATT | 83.09 | 2.98 | 83 ± 3% | 27.15 | 0.97 | 27 ± 1% |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 4595-4613 | AD-14890 | 811 | UGCWUGAWGCWCAGACTT | 812 | GUCUGAAGCAAUCAAAGCATT | 59.49 | 2.94 | 59 ± 3% | 65.04 | 3.22 | 65 ± 3% |
| 4596-4614 | AD-14891 | 813 | UUGCUUUGAUUGCUUCAGATT | 814 | UCUGAAGCAAUCAAAGCAATT | 21.93 | 5.52 | 22 ± 6% | 125.32 | 31.52 | 125 ± 32 % |
| 4597-4615 | AD-14892 | 815 | AUUGCUUUGAUUGCUUCAGTT | 816 | CUGAAGCAAUCAAAGCAAUTT | 72.69 | 2.19 | 73 ± 2% | 43.84 | 1.32 | 44 ± 1% |
| 4599-4617 | AD-14893 | 817 | CUAUUGCUUUGAUUGCUUCTT | 818 | GAAGCAAUCAAAGCAAUAGTT | 24.43 | 7.07 | 24 ± 7% | 121.32 | 35.11 | 121 ± 35 % |
| 4726-4744 | AD-14894 | 819 | AUUGCAAGGAAUGGCCUAATT | 820 | UUAGGCCAUUCCUUGCAAUTT | 33.84 | 5.08 | 34 ± 5% | 106.20 | 15.95 | 106 ± 16 % |
| 4753-4771 | AD-14895 | 821 | AUUUUCCUCCUAAUUCUGATT | 822 | UCAGAAUUAGGAGGAAAAUTT | 21.68 | 4.46 | 22 ± 4% | 125.73 | 25.84 | 126 ± 26 % |
| 4802-4820 | AD-14896 | 823 | GCUCAUCAGCCUGAUUUUGTT | 824 | CAAAAUCAGGCUGAUGAGCTT | 26.99 | 5.01 | 27 ± 5% | 117.20 | 21.73 | 117 ± 22 % |
| 4803-4821 | AD-14897 | 825 | UGCUCAUCAGCCUGAUUUUTT | 826 | AAAAUCAGGCUGAUGAGCATT | 29.04 | 2.72 | 29 ± 3% | 113.92 | 10.67 | 114 ± 11 % |

(continued)

| | | sense strand | | antisense strand | | | | | | | |
|---|---|---|---|---|---|---|---|---|---|---|---|
| position in consensus | duplex name | SEQ ID NO: | sequence (5'-3') | SEQ ID NO: | sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
| 4806-4824 | AD-14898 | 827 | AGUUGCUCAUCAGCCUGAUTT | 828 | AUCAGGCUGAUGAGCAACUTT | 32.64 | 4.87 | 33 ± 5% | 108.14 | 16.13 | 108 ± 16 % |
| 5091-5109 | AD-14899 | 829 | AAGGGCGUGGAGGCUUUUUTT | 830 | AAAAAGCCUCCACGCCCUUTT | 61.71 | 4.59 | 62 ± 5% | 61.47 | 4.57 | 61 ± 5% |
| 5093-5111 | AD-14900 | 831 | GUAAGGGCGUGGAGGCUUUTT | 832 | AAAGCCUCCACGCCCUUACTT | 31.01 | 2.84 | 31 ± 3% | 110.75 | 10.14 | 111 ± 10 % |
| 4259-4277 | AD-14901 | 833 | GACCCUAAAGACUUUCCUGTT | 834 | CAGGAAAGUCUUUAGGGUCTT | 31.47 | 1.57 | 31 ± 2% | 110.01 | 5.49 | 110 ± 5% |
| 3901-3919 | AD-14902 | 835 | AGGAGCAUGACUUUAACCCTT | 836 | GGGUUAAAGUCAUGCUCCUTT | 76.99 | 0.55 | 77 ± 1% | 36.95 | 0.26 | 37 ± 0% |
| 4757-4775 | AD-14903 | 837 | UGUGAUUUUCCUCCUAAUUTT | 838 | AAUUAGGAGGAAAAUCACATT | 20.55 | 3.55 | 21 ± 4 % | 127.55 | 22.05 | 126 ± 22 % |
| 4758-4776 | AD-14904 | 839 | UUGUGAUUUUCCUCCUAAUTT | 840 | AUUAGGAGGAAAAUCACAATT | 22.65 | 6.87 | 23 ± 7 % | 124.18 | 37.68 | 124 ± 38 % |
| 4562-4580 | AD-14905 | 841 | GACUUAACCCAAGAAGCUCTT | 842 | GAGCUUCUUGGGUUAAGUCTT | 56.98 | 4.94 | 57 ± 5% | 69.07 | 5.99 | 69 ± 6% |

(continued)

| | | sense strand | | antisense strand | | | | | | | |
|---|---|---|---|---|---|---|---|---|---|---|---|
| position in consensus | duplex name | SEQ ID NO: | sequence (5'-3') | SEQ ID NO: | sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
| 4585-4603 | AD-14906 | 843 | GCUUCAGACAAUGGWUGGTT | 844 | CCAAACCAWGUCUGAAGCTT | 34.20 | 3.66 | 34 * 4 % | 105.63 | 11.29 | 106 ± 11 % |
| 4587-4605 | AD-14907 | 845 | UUGCUUCAGACAAUGGUUUTT | 846 | AAACCAUUGUCUGAAGCAATT | 28.59 | 8.12 | 29 ± 8% | 114.64 | 32.56 | 115 ± 33 % |
| 4588-4606 | AD-14908 | 847 | AUUGCUUCAGACAAUGGUUTT | 848 | AACCAWGUCUGAAGCAAUTT | 34.08 | 3.36 | 34 ± 3% | 105.82 | 10.44 | 106 ± 10 % |
| 4591-4609 | AD-14909 | 849 | UUGAUUGCUUCAGACAAUGTT | 850 | CAUUGUCUGAAGCAAUCAATT | 76.57 | 2.33 | 77 ± 2% | 37.61 | 1.15 | 38 ± 1% |
| 5003-5021 | AD-14910 | 851 | GACAAAGUGCUGAAUAGGGTT | 852 | CCCUAUUCAGCACUUUGUCTT | 48.50 | 4.14 | 46 ± 4% | 85.89 | 7.64 | 86 ± 8% |
| 4165-4183 | AD-14911 | 853 | AAGAAAAAGCUCAAAUUUUTT | 854 | AAAAUUUGAGCUUUUCUUTT | 29.62 | 2.02 | 30 ± 2% | 112.99 | 7.69 | 113 ± 8% |
| 4166-4184 | AD-14912 | 855 | AAAGAAAAAGCUCAAAUUUTT | 856 | AAAUUUGAGCUUUUUCUUUTT | 22.27 | 0.48 | 22 ± 0% | 124.78 | 2.69 | 125 ± 3% |
| 4263-4281 | AD-14913 | 857 | AGAAGACCCUAAAGACUUUTT | 858 | AAAGUCUUUAGGGUCUUCUTT | 59.80 | 2.85 | 60 ± 3% | 64.53 | 3.08 | 65 ± 3% |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 4274-4292 | AD-14914 | 859 | AAAAAAAAGGUAGAAGACCTT | 860 | GGUCUUCUACCUUUUUUUUTT | 93.21 | 5.10 | 93 ± 5% | 10.90 | 0.60 | 11 ± 1% |
| 4266-4284 | AD-14915 | 861 | GGUAGAAGACCCUAAAGACTT | 862 | GUCUUUAGGGUCUUCUACCTT | 25.99 | 4.45 | 26 ± 4% | 118.82 | 20.34 | 119 ± 20 % |
| 4272-4290 | AD-14916 | 863 | AAAAAAGGUAGAAGACCCUTT | 864 | AGGGUCUUCUACCUUUUUUTT | 48.20 | 1.46 | 48 ± 1% | 83.16 | 2.51 | 83 ± 3% |
| 4271-4289 | AD-14917 | 865 | AAAAAGGUAGAAGACCCUATT | 866 | UAGGGUCUUCUACCUUUUUTT | 41.03 | 3.07 | 41 ± 3% | 94.67 | 7.08 | 95 ± 7% |
| 4559-4577 | AD-14918 | 867 | UUAACCCAAGAAGCUCUUCTT | 868 | GAAGAGCUUCUUGGGUUAATT | 110.62 | 6.34 | 111 ± 6% | -17.04 | 0.98 | -17 ± 1% |
| 4789-4807 | AD-14919 | 869 | AUUUUGGUACAUGGAAUAGTT | 870 | CUAUUCCAUGUACCAAAAUTT | 73.66 | 3.68 | 74 ± 4% | 42.29 | 2.11 | 42 ± 2% |
| 4998-5016 | AD-14920 | 871 | AGUGCUGAAUAGGGAGGAATT | 872 | UUCCUCCCUAUUCAGCACUTT | 19.80 | 1.72 | 20 ± 2% | 128.75 | 11.20 | 129 ± 11 % |
| 5070-5088 | AD-14921 | 873 | GAGGCCAGGGAAAUUCCCUTT | 874 | AGGGAAUUUCCCUGGCCUCTT | 33.13 | 1.14 | 33 ± 1% | 107.34 | 3.71 | 107 ± 4% |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 4158-4176 | AD-14922 | 875 | AGCUCAAAUUUUAUAUAAGTT | 876 | CUUAUAUAAAAUUUGAGCUTT | 52.94 | 6.99 | 53 ± 7% | 63.41 | 8.37 | 63 ± 8% |
| 5065-5083 | AD-14923 | 877 | CAGGGAAAUUCCCUUGUUUTT | 878 | AAACAAGGGAAUUUCCCUGTT | 33.77 | 8.92 | 34 ± 9% | 89.23 | 23.56 | 89 ± 24% |
| 2872-2890 | AD-14924 | 879 | UUGUGCAGUGGAAAGAAAGTT | 880 | CUUUCUUUCCACUGCACAATT | 64.47 | 10.96 | 64 ± 11% | 47.86 | 8.13 | 48 ± 8% |
| 4782-4800 | AD-14925 | 881 | UACAUGGAAUAGUUCAGAGTT | 882 | CUCUGAACUAWCCAUGUATT | 97.16 | 7.57 | 97 ± 8% | 3.83 | 0.30 | 4 ± 0% |
| 4783-4801 | AD-14926 | 883 | GUACAUGGAAUAGUUCAGATT | 884 | UCUGAACUAWCCAUGUACTT | 27.29 | 8.79 | 27 ± 9% | 97.96 | 31.56 | 98 ± 32 % |
| 5064-5082 | AD-14927 | 885 | AGGGAAAUUCCCUUGUUUUTT | 886 | AAAACAAGGGAAUUUCCCUTT | 27.02 | 10.01 | 27 ± 10% | 98.33 | 36.42 | 98 ± 36% |
| 5071-5089 | AD-14928 | 887 | GGAGGCCAGGGAAAWCCCTT | 888 | GGGAAUUUCCCUGGCCUCCTT | 76.75 | 4.78 | 77 ± 5% | 31.32 | 1.95 | 31 ± 2% |
| 3951-3969 | AD-14929 | 889 | GUGUAGACAGCCAUAUGCATT | 890 | UGCAUAUGGCUGUCUACACTT | 32.92 | 9.44 | 33 ± 9% | 90.38 | 25.93 | 90 ± 26 % |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 3949-3967 | AD-14930 | 891 | GUAGACAGCCAUAUGCAGUTT | 892 | ACUGCAUAUGGCUGUCUACTT | 31.00 | 9.40 | 31 ± 9% | 92.97 | 28.21 | 93 ± 28 % |
| 4355-4373 | AD-14931 | 893 | GAAGACCUGUUUUGCCAUGTT | 894 | CAUGGCAAAACAGGUCUUCTT | 31.36 | 8.73 | 31 ± 9% | 92.48 | 25.74 | 92 ± 26% |
| 4363-4381 | AD-14932 | 895 | AGUGGGAUGAAGACCUGUUTT | 896 | AACAGGUCUUCAUCCCACUTT | 32.42 | 9.01 | 32 ± 9% | 91.05 | 25.29 | 91 ± 25% |
| 4356-4374 | AD-14933 | 897 | UGAAGACCUGUUUUGCCAUTT | 898 | AUGGCAAAACAGGUCWCATT | 39.94 | 6.96 | 40 ± 7% | 80.92 | 14.10 | 81 ± 14% |
| 4361-4379 | AD-14934 | 899 | UGGGAUGAAGACCUGUUWTT | 900 | AAAACAGGUCUUCAUCCCATT | 42.94 | 7.66 | 43 ± 8% | 76.88 | 13.71 | 77 ± 14% |
| 4560-4578 | AD-14935 | 901 | CUUAACCCAAGAAGCUCUUTT | 902 | AAGAGCUUCUUGGGUUAAGTT | 47.74 | 8.48 | 48 ± 8% | 70.41 | 12.51 | 70 ± 13% |
| 2873-2891 | AD-14936 | 903 | AUUGUGCAGUGGAAAGAAATT | 904 | UUUCUUUCCACUGCACAAUTT | 35.21 | 4.02 | 35 ± 4% | 87.29 | 9.97 | 87 ± 10% |
| 4730-4748 | AD-14937 | 905 | CUUUAUUGCAAGGAAUGGCTT | 906 | GCCAWCCWGCAAUAAAGTT | 89.25 | 3.53 | 89 ± 4% | 14.48 | 0.57 | 14 ± 1% |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 3899-3917 | AD-14938 | 907 | GAGCAUGACUUUAACCCAGTT | 908 | CUGGGWAAAGUCAUGCUCTT | 29.38 | 6.46 | 29 ± 6% | 95.15 | 20.91 | 95 ± 21% |
| 4756-4774 | AD-14939 | 909 | GUGAUUUUCCUCCUAAUUCTT | 910 | GAAUUAGGAGGAAAAUCACTT | 26.45 | 8.33 | 26 ± 8% | 99.09 | 31.21 | 99 ± 31 % |
| 4590-4608 | AD-14940 | 911 | UGAUUGCUUCAGACAAUGGTT | 912 | CCAUUGUCUGAAGCAAUCATT | 77.50 | 6.51 | 78 ± 7% | 30.31 | 2.55 | 30 ± 3% |
| 4159-4177 | AD-14941 | 913 | AAGCUCAAAUUUUAUAUAATT | 914 | UUAUAUAAAAUUUGAGCUUTT | 36.40 | 10.76 | 36 ± 11% | 85.68 | 25.32 | 86 ± 25% |
| 2743-2761 | AD-14942 | 915 | CUGGACAUGGAUCAAGCACTT | 916 | GUGCUUGAUCCAUGUCCAGTT | 65.11 | 5.84 | 65 ± 6% | 47.01 | 4.22 | 47 ± 4% |
| 4155-4173 | AD-14943 | 917 | UCAAAUUUUAUAUAAGAAATT | 918 | UUUCUUAUAUAAAAUUUGATT | 89.96 | 4.69 | 90 ± 5% | 13.52 | 0.71 | 14 ± 1% |
| 2871-2889 | AD-14944 | 919 | UGUGCAGUGGAAAGAAAGGTT | 920 | CCUUUCUUUCCACUGCACATT | 48.98 | 5.85 | 49 ± 6% | 68.74 | 8.21 | 69 ± 8% |
| 4786-4804 | AD-14945 | 921 | UUGGUACAUGGAAUAGUUCTT | 922 | GAACUAUUCCAUGUACCAATT | 43.45 | 3.29 | 43 ± 3% | 76.18 | 5.77 | 76 ± 6% |

(continued)

| position in consensus | duplex name | sense strand SEQ ID NO: | sense strand sequence (5'-3') | antisense strand SEQ ID NO: | antisense strand sequence (5'-3') | Residual luciferase activity (relative to control siRNA treated cells) | SD of residual luciferase activity | Residual luciferase activity+/- SD | Relative siRNA activity (normalized to positive control luc-siRNA) | SD of relative siRNA activity | Relative siRNA activity+/- SD |
|---|---|---|---|---|---|---|---|---|---|---|---|
| 4364-4382 | AD-14946 | 923 | AAGUGGGAUGAAGACCUGUTT | 924 | ACAGGUCUUCAUCCCACUUTT | 41.25 | 1.26 | 41 ± 1% | 79.15 | 2.43 | 79 ± 2% |
| 4359-4377 | AD-14947 | 925 | GGAUGAAGACCUGUUUUGCTT | 926 | GCAAAACAGGUCWCAUCCTT | 42.10 | 7.34 | 42 ± 7% | 78.01 | 13.60 | 78 ± 14% |
| 2744-2762 | AD-14948 | 927 | UCUGGACAUGGAUCAAGCATT | 928 | UGCUUGAUCCAUGUCCAGATT | 42.39 | 5.75 | 42 ± 6% | 77.62 | 10.54 | 78 ± 11% |
| 4787-4805 | AD-14949 | 929 | UUUGGUACAUGGAAUAGUUTT | 930 | AACUAWCCAUGUACCAAATT | 27.68 | 5.79 | 28 ± 6% | 97.44 | 20.39 | 97 ± 20% |

Table Ib

siRNAs targeting JCV transcripts for primary screen

Description of chemistries:

| | |
|---|---|
| a | exo/endo-light + 2'-O-methyl in position 2 of antisense |
| b | exo/endo-light: sense strand: dTsdT + 2'OMe@all Py; antisense strand: dTsdT + 2'OMe@ Py in uA,cA |
| c | exo/endo-light + 2'-O-methyl in position 2 of sense |
| d | exo/endo-light + 2'-O-methyl in position 2 of sense and antisense |

| | | | sense strand | | antisense strand | | | | | | | |
|---|---|---|---|---|---|---|---|---|---|---|---|---|
| duplex name | chemistry | position in consensus | SE QID No : | sequence (5'-3') | SEQ ID NO: | sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
| AD-12598 | a | 1426-1444 | 1 | AcuuuuAGGGuuGuAcGGGTsT | 2 | CcCGuAcAACCCuAAAAGUTsT | 91 | 11 | 91 ± 11% | 9 | 2 | 9 ± 2% |
| AD-12708 | b | 1426-1444 | 3 | AcuuuuAGGGuuGuAcGGGTsT | 4 | CCCGuAcAACCCuAAAAGUTsT | 32 | 5 | 32 ± 5 % | 76 | 17 | 76 ± 17% |
| AD-12599 | a | 1427-1445 | 5 | cuuuuAGGGuuGuAcGGGATsT | 6 | UcCCGuAcAACCCuAAAAGTsT | 25 | 6 | 25*6% | 79 | 13 | 79 ± 13 % |
| AD-12709 | b | 1427-1445 | 7 | cuuuuAGGGuuGuAcGGGATsT | 8 | UCCCGuAcAACCCuAAAAGTsT | 16 | 4 | 16 ± 44% | 97 | 26 | 97 ± 26 % |
| AD-12600 | a | 2026-2044 | 9 | cAGAGcAcAAGGcGuAccuTsT | 10 | AgGuACGCCUUGUGCUCUGTsT | 79 | 9 | 79 ± 9% | 21 | 3 | 21 ± 3% |

(continued)

| | | | sense strand | | antisense strand | | | | | | | |
|---|---|---|---|---|---|---|---|---|---|---|---|---|
| duplex name | chemistry | position in consensus | SE QID No : | sequence (5'-3') | SEQ ID NO: | sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
| AD-12710 | b | 2026-2044 | 11 | cAGAGcACAAGGcGuAccuTeT | 12 | AGGuACGCCUUGUGCUCUGTsT | 25 | 4 | 25 ± 4% | 85 | 24 | 85 ± 24% |
| AD-12784 | c | 2026-20d4 | 13 | caGAGcAcAAGGcGuAccuTsT | 14 | AGGuACGCCUUGUGCUCUGTsT | 23 | 2 | 23 ± 2% | 87 | 14 | 87 ± 14 % |
| AD-12832 | d | 2026-2044 | 15 | caGAGcAcAAGGcGuAccuTsT | 16 | AgGuACGCCWGUGCUCUGTST | 84 | 11 | 84 ± 11 % | 18 | 4 | 18 ± 4 % |
| AD-12601 | a | 1431-1449 | 17 | uAGGGuuGuAcGGGAcuGuTsT | 18 | AcAGUCCCGuAcAACCCuATsT | 102 | 8 | 102 ± 8 % | -6 | 1 | -6 ± 1% |
| AD-12785 | c | 1431-1449 | 19 | uaGGGuuGuAcGGGAcuGuTsT | 20 | AcAGUCCCGuAcAACCCuATsT | 95 | 10 | 95 ± 10% | 6 | 1 | 6 ± 1% |
| AD-12602 | a | 1432-1450 | 21 | AGGGuuGuAcGGGAcuGuATsT | 22 | uacAGUCCCGuAcAACCCUTsT | 107 | 9 | 107 ± 9% | -11 | 2 | -11 ± 2% |
| AD-12711 | b | 1432-1450 | 23 | AGGGuuGuAcGGGAcuGuATsT | 24 | uAcAGUCCCGuAcAACCCUTsT | 70 | 4 | 70 ± 4% | 34 | 3 | 34 ± 3% |
| AD-12786 | c | 1432-1450 | 25 | AgGGuuGuAcGGGAcuGuATsT | 26 | uAcAGUCCCGuAcAACCCUTsT | 69 | 8 | 69 ± 8 % | 35 | 7 | 35 ± 7 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12833 | d | 1432-1450 | 27 | AgGGuuGuAcGGGAcuGuATsT | 28 | uacAGUCCCGuAcAACCCUTsT | 94 | 8 | 94 ± 8% | 7 | 1 | 7 ± 1% |
| AD-12603 | a | 1436-1454 | 29 | uuGuAcGGGAcuGuAAcAcTsT | 30 | GuGUuAcAGUCCCGuAcAATsT | 100 | 9 | 100 ± 9 % | -4 | 1 | -4 ± 1% |
| AD-12712 | b | 1436-1454 | 31 | uuGuAcGGGAcuGuAAcAcTsT | 32 | GuGUuAcAGUCCCGuAcAATsT | 27 | 5 | 27 ± 5 % | 82 | 16 | 82 ± 16 % |
| AD-12604 | a | 4794-4812 | 33 | GccuGAuuuuGGuAcAuGGTsT | 34 | CcAUGuACcAAAAUcAGGCTsT | 15 | 2 | 15 ± 2% | 94 | 13 | 94 ± 13% |
| AD-12605 | a | 5099-5117 | 35 | GAAGuAGuAAGGGcGuGGATsT | 36 | UccACGCCCUuACuACUUCTsT | 94 | 5 | 94 ± 5% | 7 | 0 | 7 ± 0% |
| AD-12713 | b | 5099-5117 | 37 | GAAGuAGuAAGGGcGuGGATsT | 38 | UCcACGCCCUuACuACWCTsT | 61 | 10 | 61 ± 10% | 41 | 8 | 41 ± 8% |
| AD-12787 | c | 5099-5117 | 39 | GaAGuAGuAAGGGcGuGGATsT | 40 | UCcACGCCCUuACuACUUCTsT | 55 | 6 | 55 ± 6% | 47 | 6 | 47 ± 6% |
| AD-12834 | d | 5099-5117 | 41 | GaAGuAGuAAGGGcGuGGATsT | 42 | UccACGCCCUuACuACUUCTsT | 92 | 16 | 92 ± 16 % | 8 | 2 | 8 ± 2% |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12606 | a | 713-731 | 43 | AuAGGccuuAcuccuGAAATsT | 44 | UuUcAGGAGuAAGGCCuAUTST | 78 | 3 | 78 ± 3% | 25 | 1 | 25 ± 1% |
| AD-12714 | b | 713-731 | 45 | AuAGGccuuAcuccuGAAATsT | 46 | UUUcAGGAGuAAGGCCuAUTsT | 63 | 6 | 63 ± 6% | 42 | 5 | 42 ± 5 % |
| AD-12607 | a | 3946-3964 | 47 | GAcAGccAuAuGcAGuAGuTsT | 48 | AcuACUGcAuAUGGCUGUCTsT | 101 | 9 | 101 ± 9% | -1 | 0 | -1 ± 0% |
| AD-12715 | b | 3946-3964 | 49 | GAcAGccAuAuGcAGuAGuTsT | 50 | ACuACUGcAuAUrGCUGUCTsT | 101 | 5 | 101 ± 5% | -1 | 0 | -1 ± 0% |
| AD-12788 | c | 3946-3964 | 51 | GacAGccAuAuGcAGuAGuTsT | 52 | ACuACUGcAuAUGGCUGUCTsT | 85 | 18 | 85 ± 18 % | 15 | 4 | 15 ± 4% |
| AD-12835 | d | 3946-3964 | 53 | GacAGccAuAuGcAGuAGuTsT | 54 | AcuACUGcAuAUGGCUGUCTsT | 95 | 9 | 95 ± 9% | 6 | 1 | 6 ± 1% |
| AD-12608 | a | 1128-1146 | 55 | AAAcuAcuuGGGcAAuAGuTsT | 56 | AcuAUUGCCcAAGuAGUUUTsT | 103 | 13 | 103 ± 13 % | -3 | 0 | -3 ± 0 % |
| AD-12716 | b | 1128-1146 | 57 | AAAcuAcuuGGGcAAuAGuTsT | 58 | ACuAWGCCcAAGuAGUWTsT | 81 | 9 | 81 ± 9% | 22 | 3 | 22 ± 3 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12789 | c | 1128-1146 | 59 | AaAcuAcuuGGGcAAuAGuTsT | 60 | ACuAUUGCCcAAGuAGUUUTsT | 61 | 4 | 61 ± 4% | 44 | 4 | 44 ± 4 % |
| AD-12836 | d | 1128-1146 | 61 | AaAcuAcuuGGGcAAuAGuTsT | 62 | AcuAUUGCCcAAGuAGUUUTsT | 103 | 11 | 103 ± 11 % | -3 | 0 | -3 ± 0 % |
| AD-12609 | a | 525-543 | 63 | ucAGGuucAuGGGuGccGcTsT | 64 | GcGGcACCcAUGAACCUGATsT | 108 | 19 | 108 ± 19 % | -9 | 2 | -9 ± 2 % |
| AD-12717 | b | 525-543 | 65 | ucAGGuucAuGGGuGccGcTsT | 66 | GCGGcACCcAUGAACCUGATsT | 94 | 17 | 94 ± 17 % | 7 | 1 | 7 ± 1 % |
| AD-12610 | a | 5096-5114 | 67 | GuAGuAAGGGcGuGGAGGcTsT | 68 | GcCUCcACGCCCUuACuACTST | 88 | 9 | 88 ± 9% | 14 | 2 | 14 ± 2 % |
| AD-12718 | b | 5096-5114 | 69 | GuAGuAAGGGcGuGGAGGcTsT | 70 | GCCUCcACGCCCUuACuACTsT | 39 | 4 | 39 ± 4 % | 64 | 8 | 64 ± 8% |
| AD-12611 | a | 4727-4745 | 71 | uAuuGcAAGGAAuGGccuATsT | 72 | uaGGCcAUUCCUUGcAAuATsT | 38 | 6 | 38 ± 6 % | 69 | 12 | 69 ± 12 % |
| AD-12719 | b | 4727-4745 | 73 | uAuuGcAAGGAAuGGccuATsT | 74 | uAGGCcAUUCCUUGcAAuATsT | 26 | 4 | 25 ± 4 % | 78 | 13 | 78 ± 13 % |

(continued)

| | | | sense strand | | antisense strand | | | | | | | |
|---|---|---|---|---|---|---|---|---|---|---|---|---|
| duplex name | chemistry | position in consensus | SE QID No : | sequence (5'-3') | SEQ ID NO: | sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
| AD-12790 | c | 4727-4745 75 | | uauuGcAAGGAAuGGccuATsT | 76 | uAGGCcAUUCCUUGcAAuATsT | 17 | 3 | 17 ± 3% | 87 | 18 | 87 ± 18% |
| AD-12837 | d | 4727-4745 | 77 | uauuGcAAGGAAuGGccuATsT | 78 | uaGGCcAWCCWGcAAUATsT | 22 | 4 | 22 ± 4 % | 81 | 16 | 81 ± 16 % |
| AD-12612 | a | 5097-5115 | 79 | AGuAGuAAGGGcGuGGAGGTsT | 80 | CcUCcACGCCCUuACuACUTsT | 100 | 6 | 100 ± 6% | 0 | 0 | 0 ± 0 % |
| AD-12720 | b | 5097-5115 | 81 | AGuAGuAAGGGcGuGGAGGTsT | 82 | CCUCcACGCCCUuACuACUTsT | 73 | 6 | 73 ± 6 % | 28 | 3 | 28 ± 3 % |
| AD-12791 | c | 5097-5115 | 83 | AguAGuAAGGGcGuGGAGGTST | 84 | CCUCcACGCCCUuACuACUTsT | 46 | 9 | 46 ± 9 % | 57 | 12 | 57 ± 12 % |
| AD-12838 | d | 5097-5115 | 85 | AguAGuAAGGGcGuGGAGGTsT | 86 | CcUCcACGCCCUuACuACUTsT | 97 | 15 | 97 ± 15 % | 3 | 1 | 3 ± 1 % |
| AD-12613 | a | 4601-d619 | 87 | uGcuAuuGcuuuGAuuGcuTsT | 88 | AgcAAUcAAAGcAAuAGcATsT | 26 | 4 | 26 ± 4 % | 82 | 15 | 82 ± 15 % |
| AD-12721 | b | 4601-4619 | 89 | uGcuAuuGcuuuGAuuGcuTsT | 90 | AGcAAUcAAAGcAAuAGcATsT | 10 | 1 | 10 ± 1% | 94 | 12 | 94 ± 12 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12792 | c | 4601-4619 | 91 | ugcuAuuGcuuuGAuuGcuTsT | 92 | AGcAAUcAAAGcAAuAGcATsT | 10 | 3 | 10 * 3 °.6 | 94 | 40 | 94 ± 40 % |
| AD-12839 | d | 4601-4619 | 93 | ugcuAuuGcuuuGAuuGcuTsT | 94 | AgcAAUcAAAGcAAuAGcATsT | 22 | 3 | 22 ± 3% | 81 | 12 | 81 ± 12% |
| AD-12614 | a | 4600-4618 | 95 | GcuAuuGcuuuGAuuGcuuTsT | 96 | AaGcAAUcAAAGcAAuAGCTsT | 15 | 5 | 15 ± 5 % | 94 | 38 | 94 ± 38 % |
| AD-12722 | b | 4600-4618 | 97 | GcuAuuGcuuuGAuuGcuuTsT | 98 | AAGcAAUCAAAGCAAUAGCTsT | 6 | 1 | 6 ± 1 % | 98 | 26 | 98 ± 26 % |
| AD-12615 | a | 1421-1439 | 99 | ccuuuAcuuuuAGGGuuGuTsT | 100 | AcAACCCuAAAAGuAAAGGTsT | 93 | 4 | 93 ± 4 % | 8 | 0 | 8 ± 0 % |
| AD-12616 | a | 1424-1442 | 101 | uuAcuuuuAGGGuuGuAcGTsT | 102 | CguAcAACCCuAAAAGuAATsT | 95 | 4 | 95 ± 4 % | 5 | 0 | 5 ± 0% |
| AD-12723 | b | 1424-1442 | 103 | uuAcuuuuAGGGuuGuAcGTsT | 104 | CGuAcAACCCuAAAAGuAATsT | 73 | 7 | 73 ± 7 % | 30 | 3 | 30 ± 3% |
| AD-12617 | a | 1403-1421 | 105 | GcuccucAAuGGAuGuuGcTsT | 106 | GcAAcAUCcAUUGAGGAGCTsT | 88 | 10 | 88 ± 10% | 13 | 2 | 13 ± 2 % |

(continued)

| duplex name | chemistry | position in consensus | sense strand SE QID No : | sense strand sequence (5'-3') | antisense strand SEQ ID NO: | antisense strand sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12618 | a | 1534-1552 | 107 | uuAuAAGAGGAGGAGuAGATsT | 108 | UcuACUCCUCCUCUuAuAATsT | 42 | 7 | 42 ± 7 % | 60 | 7 | 60 ± 7 % |
| AD-12724 | b | 1534-1552 | 109 | uuAuAAGAGGAGGAGuAGATsT | 110 | UCuACUCCUCCUCUuAuAATsT | 21 | 5 | 21 ± 5 % | 89 | 32 | 89 ± 32 % |
| AD-12619 | a | 5098-5116 | 111 | AAGuAGuAAGGGcGuGGAGTsT | 112 | CuCcACGCCCUuACuACWTsT | 95 | 7 | 95 ± 7 % | 6 | 1 | 6 ± 1 % |
| AD-12725 | b | 5098-5116 | 113 | AAGuAGuAAGGGcGuGGAGTST | 114 | CUCcACGCCCUuACuACUUTST | 71 | 2 | 71 ± 2 % | 30 | 1 | 30 ± 1 % |
| AD-12793 | c | 5098-5116 | 115 | AaGuAGuAAGGGcGuGGAGTsT | 116 | CUCcACGCCCUuACuACUUTsT | 54 | | 7 54 ± 7 % | 48 | 7 | 48 ± 7 % |
| AD-12840 | d | 5098-5116 117 | | AaGuAGuAAGGGcGuGGAGTsT | 118 | CuCcACGCCCUuACuACUUTsT | 94 | | 9 94 ± 9 % | 7 | 1 | 7 ± 1 % |
| AD-12620 | a | 1430-1448 119 | | uuAGGGuuGuAcGGGAcuGTsT | 120 | caGUCCCGuAcAACCCuAATsT | 106 | | 7 106 ± 7 % | -8 | 1 | -8 ± 1 % |
| AD-12726 | b | 1430-1448 | 121 | uuAGGGuuGuAcGGGAcuGTsT | 122 | cAGUCCCGuAcAACCCuAATsT | 100 | 7 | 100 ± 7 % | 0 | 0 | 0 ± 0 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12621 | a | 1701-1719 | 123 | GAcAuGcuuccuuGuuAcATsT | 124 | UguAAcAAGGAAGcAUGUCTsT | 107 | 9 | 107 ± 9 % | -7 | 1 | -7 ± 1 % |
| AD-12727 | b | 1701-1719 | 125 | GAcAuGcuuccuuGuuAcATsT | 126 | UGuAAcAAGGAAGcAUGUCTsT | 47 | 4 | 47 ± 4 % | 60 | 8 | 60 ± 8 % |
| AD-12794 | c | 1701-1719 | 127 | GacAuGcuuccuuGuuAcATsT | 128 | UGuAAcAAGGAAGcAUGUCTsT | 40 | 8 | 40 ± 8 % | 67 | 20 | 67 ± 20 % |
| AD-12841 | d | 1701-1719 | 129 | GacAuGcuuccuuGuuAcATsT | 130 | UguAAcAAGGAAGcAUGUCTsT | 78 | 13 | 78 ± 13 % | 25 | 8 | 25 ± 8 % |
| AD-12622 | a | 2066-2084 | 131 | uGuuGAAuGuuGGGuuccuTsT | 132 | AgGAACCcAACAUCAAcATsT | 16 | 4 | 16 ± 4 % | 92 | 29 | 92 ± 29 % |
| AD-12728 | b | 2066-2084 | 133 | uGuuGAAuGuuGGGuuccuTsT | 134 | AGGAACCcAACAUUcAAcATsT | 25 | 6 | 25 ± 6 % | 84 | 29 | 84 ± 29% |
| AD-12795 | c | 2066-2084 | 135 | uguuGAAuGuuGGGuuccuTsT | 136 | AGGAACCcAAcAUUcAAcATsT | 23 | 3 | 23 ± 3 % | 86 | 20 | 86 ± 20 % |
| AD-12842 | d | 2066-2084 | 137 | uguuGAAuGuuGGGuuccuTsT | 138 | AgGAACCcAAcAUUcAAcATsT | 19 | 4 | 19 ± 4 % | 91 | 20 | 91 ± 20 % |

(continued)

| duplex name | chemistry | position in consensus | sense strand SE QID No : | sense strand sequence (5'-3') | antisense strand SEQ ID NO: | antisense strand sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12623 | a | 4561-4579 | 139 | AcuuAAcccAAGAAGcucuTsT | 140 | AgAGCWCWGGGUuAAGUTsT | 103 | 9 | 103 ± 9 % | -3 | 0 | -3 ± 0 % |
| AD-12729 | b | 4561-4579 | 141 | AcuuAAcccAAGAAGcucuTsT | 142 | AGAGCUUCUUGGGUuAAGUTsT | 84 | | 8 84 ± 8 % | 17 | 2 | 17 ± 2 % |
| AD-12624 | a | 4797-4815 | 143 | ucAGccuGAuuuuGGuAcATsT | 144 | UguACcAAAAUcAGGCUGATsT | 31 | 4 | 31 ± 4 % | 77 | 12 | 77 ± 12 % |
| AD-12730 | b | 4797-4815 | 145 | ucAGccuGAuuuuGGuAcATsT | 146 | UGuACcAAAAUcAGGCUGATsT | 18 | 1 | 18 ± 1 % | 85 | 3 | 85 ± 3 % |
| AD-12625 | a | 1428-1446 | 147 | uuuuAGGGuuGuAcGGGAcTsT | 148 | GuCCCGuAcAACCCuAAAATsT | 94 | 10 | 94 ± 10 % | 5 | 1 | 5 ± 1 % |
| AD-12731 | b | 1428-1446 | 149 | uuuuAGGGuuGuAcGGGAcTsT | 150 | GUCCCGuAcAACCCuAAAATsT | 57 | 4 | 57 ± 4 % | 48 | 4 | 48 ± 4 % |
| AD-12626 | a | 1429-1447 | 151 | uuuAGGGuuGuAcGGGAcuTsT | 152 | AgUCCCGuAcAACCCuAAATsT | 99 | | 7 99 ± 7 % | 0 | 0 | 0 ± 0 % |
| AD-12732 | b | 1429-1447 | 153 | uuuAGGGuuGuACGGGAcuTsT | 154 | AGUCCCGuAcAACCCuAAATsT | 82 | | 5 82 ± 5 % | 20 | 1 | 20 ± 1 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12627 | a | 662-680 | 155 | ucccuuGcuAcuGuAGAGGTsT | 156 | CcUCuAcAGuAGcAAGGGATsT | 80 | 6 | 80 ± 6 % | 22 | 2 | 22 ± 2 % |
| AD-12733 | b | 662-680 | 157 | ucccuuGcuAcuGuAGAGGTsT | 158 | CCUCuAcAGuAGcAAGGGATsT | 65 | 6 | 65 ± 6 % | 39 | 4 | 39 ± 4 % |
| AD-12628 | a | 663-681 | 159 | cccuuGcuAcuGuAGAGGGTsT | 160 | CcCUCuAcAGuAGcAAGGGTsT | 81 | 6 | 81 ± 6 % | 21 | 2 | 21 ± 2 % |
| AD-12734 | b | 663-681 | 161 | cccuuGcuAcuGuAGAGGGTsT | 162 | CCCUCuAcAGuAGcAAGGGTsT | 82 | 7 | 82 ± 7 % | 21 | 2 | 21 ± 2 % |
| AD-12629 | a | 1402-1420 | 163 | uGcuccucAAuGGAuGuuGTsT | 164 | caAcAUCcAUUGAGGAGcATsT | 113 | 11 | 113 ± 11 % | -14 | 2 | -14 ± 2 % |
| AD-12735 | b | 1402-1420 | 165 | uGcuccucAAuGGAuGuuGTsT | 166 | cAACAUCcAUUGAGGAGcATsT | 90 | 9 | 90 ± 9 % | 11 | 1 | 11 ± 1 % |
| AD-12796 | c | 1402-1420 | 167 | ugcuccucAAuGGAuGuuGTsT | 168 | cAACAUCcAUUGAGGAGcATsT | 92 | 8 | 92 ± 8 % | 9 | 1 | 9 ± 1 % |
| AD-12843 | d | 1402-1420 | 169 | ugcuccucAAuGGAuGuuGTsT | 170 | caAcAUCcAUUGAGGAGcATsT | 117 | 7 | 117 ± 77 % | -19 | 1 | -19 ± 1 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12630 | a | 1398-1416 | 171 | GAucuGcuccucAAuGGAuTsT | 172 | AuCcAUUGAGGAGcAGAUCTsT | 124 | 3 | 124 ± 3 % | -27 | 1 | -27 ± 1 % |
| AD-12736 | b | 1398-1416 | 173 | GAucuGcuccucAAuGGAuTsT | 174 | AUCcAUUGAGGAGcAGAUCTsT | 85 | 4 | 85 ± 4 % | 16 | 1 | 16 ± 1 % |
| AD-12797 | c | 1398-1416 | 175 | GaucuGcuccucAAuGGAuTsT | 176 | AUCcAUUGAGGAGcAGAUCTsT | 52 | 1 | 52 ± 1 % | 53 | 1 | 53 ± 1 % |
| AD-12844 | d | 1398-1416 | 177 | GaucuGcuccucAAuGGAuTsT | 178 | AuCcAUUGAGGAGcAGAUCTsT | 96 | 4 | 96 ± 4 % | 5 | 0 | 5 ± 0 % |
| AD-12631 | a | 1399-1417 | 179 | AucuGcuccucAAuGGAuGTsT | 180 | caUCcAUUGAGGAGcAGAUTsT | 110 | 11 | 110 ± 11 % | -12 | 1 | -12 ± 1 % |
| AD-12737 | b | 1399-1d17 | 181 | AucuGcuccucAAuGGAuGTsT | 182 | cAUCcAUUGAGGAGcAGAUTsT | 115 | 13 | 115 ± 13 % | -17 | 2 | -17 ± 2 % |
| AD-12632 | a | 1400-1418 | 183 | ucuGcuccucAAuGGAuGuTsT | 184 | AcAUCcAUUGAGGAGcAGATsT | 106 | 2 | 106 ± 2 % | -7 | 0 | ·7 ± 0 % |
| AD-12633 | a | 1401-1419 | 185 | cuGcuccucAAuGGAuGuuTsT | 186 | AacAUCcAUGAGGAGcAGTsT | 107 | 12 | 107 ± 12 % | -8 | 1 | -8 ± 1 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12738 | b | 1401-1419 | 187 | cuGcuccucAAuGGAuGuuTsT | 188 | AAcAUCcAUUGAGGAGcAGTsT | 88 | 5 | 88 ± 5 % | 14 | 1 | 14 ± 1 % |
| AD-12634 | a | 1435-1953 | 189 | GuuGuAcGGGAcuGuAAcATsT | 190 | UgUuAcAGUCCCGuAcAACTsT | 79 | 5 | 79 ± 5 % | 24 | 1 | 24 ± 1 % |
| AD-12739 | b | 1435-1453 | 191 | GuuGuAcGGGAcuGuAAcATsT | 192 | UGUuAcAGUCCCGuAcAACTsT | 69 | 8 | 69 ± 8 % | 35 | 6 | 35 ± 6 % |
| AD-12635 | a | 1437-1455 | 193 | uGuAcGGGAcuGuAAcAccTsT | 194 | GgUGUuAcAGUCCCGuAcATsT | 75 | 8 | 75 ± 8 % | 25 | 6 | 25 ± 6 % |
| AD-12740 | b | 1437-1455 | 195 | uGuAcGGGAcuGuAAcAccTsT | 196 | GGUGUuAcAGUCCCGuAcATsT | 65 | 8 | 65 ± 8 % | 40 | 8 | 40 ± 8 % |
| AD-12798 | c | 1437-1455 | 197 | uguAcGGGAcuGuAAcAccTsT | 198 | GGUGUuAcAGUCCCGuAcATsT | 56 | 4 | 56 ± 4 % | 50 | 6 | 50 ± 6 % |
| AD-12845 | d | 1437-1455 | 199 | uguAcGGGAcuGuAAcAccTsT | 200 | GgUGUuAcAGUCCCGuAcATsT | 74 | 6 | 74 ± 6 % | 30 | 3 | 30 ± 3 % |
| AD-12636 | a | 1438-1456 | 201 | GuAcGGGAcuGuAAcAccuTsT | 202 | AgGUGUuAcAGUCCCGuACTsT | 89 | 8 | 89 ± 8 % | 9 | 1 | 9 ± 1 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12741 | b | 1438-1456 | 203 | GuAcGGGAcuGuAAcAccuTsT | 204 | AGGUGUuAcAGUCCCGuACTsT | 31 | 4 | 31 ± 4% | 78 | 14 | 78 ± 14 % |
| AD-12637 | a | 4796-4814 | 205 | cAGccuGAuuuuGGuAcAuTsT | 206 | AuGuACcAAAAUcAGGCUGTsT | 16 | 2 | 16 ± 2 % | 93 | 14 | 93 ± 14 % |
| AD-12742 | b | 4796-4814 | 207 | cAGccuGAuuuuGGuAcAuTsT | 208 | AUGuACcAAAAUcAGGCUGTsT | 18 | 3 | 18 ± 3 % | 85 | 14 | 85 ± 14 % |
| AD-12799 | c | 4796-4814 | 209 | caGccuGAuuuuGGuAcAuTsT | 210 | AUCuACcAAAAUcAGGCUGTsT | 18 | 4 | 18 ± 4 % | 86 | 22 | 86 ± 22 % |
| AD-12846 | d | 4796-4814 | 211 | caGccuGAuuuuGGuAcAuTsT | 212 | AuGuACcAAAAUcAGGCUCTsT | 15 | 2 | 15 ± 2 % | 89 | 14 | 89 ± 14 % |
| AD-12638 | a | 4992-5010 | 213 | GAAuAGGGAGGAAuccAuGTsT | 214 | caUGGAUUCCUCCCuAUUCTsT | 95 | 6 | 95 ± 6 % | 5 | 0 | 5 ± 0 % |
| AD-12743 | b | 4992-5010 | 215 | GAAuAGGGAGGAAuccAuGTsT | 216 | cAUGGAUUCCUCCCuAUUCTsT | 23 | 4 | 23 ± 4 % | 81 | 15 | 81 ± 15 % |
| AD-12800 | c | 4992-5010 | 217 | GaAuAGGGAGGAAuccAuGTsT | 218 | cAUGGAUUCCUCCCuAUUCTsT | 14 | 1 | 14 ± 1 % | 90 | 10 | 90 ± 10 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12847 | d | 4992-5010 | 219 | GaAuAGGGAGGAAuccAuGTsT | 220 | caUGGAUUCCUCCCuAUUCTsT | 90 | 12 | 90 ± 12 % | 10 | 2 | 10 ± 2 % |
| AD-12639 | a | 4999-5017 | 221 | AAGuGcuGAAuAGGGAGGATsT | 222 | UcCUCCCuAUUcAGcACUUTsT | 113 | 11 | 113 ± 11 % | -15 | 2 | -15 ± 2 % |
| AD-12744 | b | 4999-5017 | 223 | AAGuGcuGAAuAGGGAGGATsT | 224 | UCCUCCCuAUUcAGcACUUTsT | 42 | 4 | 42 ± 4 % | 60 | 7 | 60 ± 7 % |
| AD-12801 | c | 4999-5017 | 225 | AaGuGcuGAAuAGGGAGGATsT | 226 | UCCUCCCuAUUcAGcACUUTsT | 34 | 3 | 34 ± 3 % | 68 | 8 | 68 ± 8 % |
| AD-12848 | d | 4999-5017 | 227 | AaGuGcuGAAuAGGGAGGATsT | 228 | UcCUCCCuAUUcAGcACUUTsT | 114 | 3 | 114 ± 3 % | -14 | 0 | -14 ± 0 % |
| AD-12640 | a | 630-648 | 229 | AGGcuGcuGcuAcuAuAGATsT | 230 | UcuAuAGuAGcAGcAGCCUTsT | 96 | 11 | 96 ± 11 % | 4 | 1 | 4 ± 1 % |
| AD-12745 | b | 630-648 | 231 | AGGcuGcuGcuAcuAuAGATsT | 232 | UCuAuAGuAGcAGcAGCCUTsT | 52 | 7 | 52 ± 7 % | 53 | 8 | 53 ± 8 % |
| AD-12802 | c | 630-648 | 233 | AgGcuGcuGcuAcuAuAGATsT | 234 | UCuAuAGuAGcAGcAGCCUTsT | 74 | 9 | 74 ± 9 % | 29 | 4 | 29 ± 4 % |

(continued)

| | | | sense strand | | antisense strand | | | | | | | |
|---|---|---|---|---|---|---|---|---|---|---|---|---|
| duplex name | chemistry | position in consensus | SEQ ID No : | sequence (5'-3') | SEQ ID NO: | sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
| AD-12849 | d | 630-648 | 235 | AgGcuGcuGcuAcuAuAGATsT | 236 | UcuAuAGuAGcAGcAGCCUTsT | 111 | 5 | 111 ± 5 % | -12 | 1 | -12 ± 1 % |
| AD-12641 | a | 3947-3965 | 237 | AGACAGccAuAuGcAGuAGTsT | 238 | CuACUGcAuAUGGCUGUCUTsT | 103 | 8 | 103 ± 8 % | -3 | 0 | -3 ± 0 % |
| AD-12803 | c | 3947-3965 | 239 | AgAcAGccAuAuGcAGuAGTsT | 240 | CuACUGcAuAUGGCUGUCUTsT | 94 | 13 | 94 ± 13 % | 6 | 1 | 6 ± 1 % |
| AD-12642 | a | 524-542 | 241 | uucAGGuucAuGGGuGccGTsT | 242 | CgGcACCcAUGAACCUGAATsT | 105 | 3 | 105 ± 3 % | -6 | 0 | -6 ± 0 % |
| AD-12746 | b | 524-542 | 243 | uucAGGuucAuGGGuGccGTsT | 244 | CGGcACCcAUGAACCUGAATsT | 100 | 9 | 100 ± 9 % | 0 | 0 | 0 ± 0 % |
| AD-12643 | a | 3948-3966 | 245 | uAGAcAGccAuAuGcAGuATsT | 246 | uaCUGcAuAUGGCUGUCuATsT | 33 | 4 | 33 ± 4 % | 74 | 10 | 74 ± 10 % |
| AD-12747 | b | 3948-3966 | 247 | uAGAcAGccAuAuGcAGuATsT | 248 | uACUGcAuAUGGCUGUCuATsT | 21 | 3 | 21 ± 3 % | 83 | 13 | 83 ± 13 % |
| AD-12804 | c | 3948-3966 | 249 | uaGAcAGccAuAuGcAGuATsT | 250 | uACUGcAuAUGGCUGUCuATsT | 25 | 4 | 25 ± 4 % | 78 | 14 | 78 ± 14 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12850 | d | 3948-3966 | 251 | uaGAcAGccAuAuGcAGuATsT | 252 | uaCUGcAuAUGGCUGUCuATsT | 28 | 4 | 28 ± 4 % | 75 | 11 | 75 ± 11 % |
| AD-12644 | a | 3900-3918 | 253 | GGAGcAuGAcuuuAAcccATsT | 254 | UgGGUuAAAGUcAUGCUCCTsT | 82 | 7 | 82 ± 7 % | 20 | 2 | 20 ± 2 % |
| AD-12748 | b | 3900-3918 | 255 | GGAGcAuGAcuuuAAcccATST | 256 | UGGGUuAAAGUcAUGCUCCTsT | 25 | 4 | 25 ± 4 % | 78 | 14 | 78 ± 14 % |
| AD-12805 | c | 3900-3918 | 257 | GgAGcAuGAcuuuAAcccATsT | 258 | UGGGUuAAAGUcAUGCUCCTsT | 23 | 7 | 23 ± 7 % | 80 | 30 | 80 ± 30 % |
| AD-12851 | d | 3900-3918 | 259 | GgAGcAuGAcuuuAAcccATsT | 260 | UgGGUuAAAGUcAUGCUCCTsT | 61 | 7 | 61 ± 7 % | 41 | 5 | 41 ± 5 % |
| AD-12645 | a | 1417-1435 | 261 | GuuGccuuuAcuuuuAGGGTsT | 262 | CcCuAAAAGuAAAGGcAACTsT | 112 | 6 | 112 ± 6 % | -14 | 1 | -14 ± 1 % |
| AD-12749 | b | 1417-1435 | 263 | GuuGccuuuAcuuuuAGGGTsT | 264 | CCCuAAAAGuAAAGGcAACTsT | 86 | 10 | 86 ± 10 % | 16 | 2 | 16 ± 2 % |
| AD-12646 | a | 4565-4583 | 265 | UGUGAcuuAAccCAAGAAGTsT | 266 | CuUCUUGGGUuAAGUCACATsT | 94 | 10 | 94 ± 10 % | 6 | 1 | 6 ± 1 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12750 | b | 4565-4583 | 267 | uGuGAcuuAACCCAAGAAGTsT | 268 | CUUCUUGGGUuAAGUcAcATsT | 93 | 11 | 93 ± 11 % | 7 | 1 | 7 ± 1 % |
| AD-12806 | c | 4565-4583 | 269 | uguGAcuuAACCCAAGAAGTsT | 270 | CUUCUUGGGUuAAGUcAcATsT | 77 | 8 | 77 ± 8 % | 24 | 3 | 24 ± 3 % |
| AD-12852 | d | 4565-4583 | 271 | uguGAcuuAAcccAAGAAGTsT | 272 | CuUCUUGGGUuAAGUcAcATsT | 96 | 4 | 96 ± 4 % | 5 | 0 | 5 ± 0 % |
| AD-12647 | a | 4598-4616 | 273 | uAuuGcuuuGAuuGcuucATsT | 274 | UgAAGcAAUcAAAGcAAuATsT | 27 | 3 | 27 ± 3 % | 81 | 11 | 81 ± 1 % |
| AD-12751 | b | 4598-4616 | 275 | uAuuGcuuuGAuuGcuucATsT | 276 | UGAAGcAAUcAAAGcAAuATsT | 29 | 6 | 29 ± 6 % | 74 | 19 | 74 ± 19 % |
| AD-12807 | c | 4598-4616 | 277 | uauuGcuuuGAuuGcuucATsT | 278 | UGAAGcAAUcAAAGcAAuATsT | 31 | 2 | 31 ± 2 % | 72 | 6 | 72 ± 6 % |
| AD-12853 | d | 4598-4616 | 279 | uauuGcuuuGAuuGcuucATsT | 280 | UgAAGcAAUcAAAGcAAuATsT | 26 | 3 | 26 ± 3 % | 78 | 11 | 76 ± 11 % |
| AD-12648 | a | 2060-2078 | 281 | AuAuccuGuuGAAuGuuGGTsT | 282 | CcAAcAUUcAAcAGGAuAUTsT | 81 | 9 | 81 ± 9 % | 17 | 3 | 17 ± 3 % |

(continued)

| duplex name | chemistry | position in consensus | sense strand SE QID No : | sense strand sequence (5'-3') | antisense strand SEQ ID NO: | antisense strand sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12649 | a | 4729-4797 | 283 | uuuAuuGcAAGGAAuGGccTsT | 284 | GgCcAUUCCUUGcAAuAAATsT | 92 | 9 | 92 ± 9 % | 8 | 1 | 8 ± 1 % |
| AD-12752 | b | 4729-4747 | 285 | uuuAuuGcAAGGAAuGGccTsT | 286 | GGCcAUUCCUUGcAAuAAATsT | 71 | 9 | 71 ± 9 % | 30 | 5 | 30 ± 5 % |
| AD-12650 | a | 1122-1140 | 287 | uGGAAGAAAcuAcuuGGGcTsT | 288 | GcCcAAGuAGUUUCUUCcATsT | 81 | 2 | 81 ± 2 % | 21 | 1 | 21 ± 1 % |
| AD-12753 | b | 1122-1140 | 289 | uGGAAGAAAcuAcuuGGGcTsT | 290 | GCCcAAGuAGUUUCUUCcATsT | 57 | 1 | 57 ± 1 % | 48 | 1 | 48 ± 1 % |
| AD-12808 | c | 1122-1140 | 291 | ugGAAGAAAcuAcuuGGGcTsT | 292 | GCCcAAGuAGUUUCUUCcATsT | 52 | 4 | 52 ± 4 % | 54 | 5 | 54 ± 5 % |
| AD-12854 | d | 1122-1140 | 293 | ugGAAGAAAcuAcuuGGGcTsT | 294 | GcCcA-AGuAGUUUCUUCcATsT | 77 | 5 | 77 ± 5 % | 26 | 2 | 26 ± 2 % |
| AD-12651 | a | 4261-4279 | 295 | AAGAcccuAAAGAcuuuccTsT | 296 | GgAAAGUCUUuAGGGUCUUTsT | 89 | 6 | 89 ± 6 % | 13 | 1 | 13 ± 1 % |
| AD-12754 | b | 4261-4279 | 297 | AAGAcccuAAAGAcuuuccTsT | 298 | GGAAAGUCUUuAGGGUCUUTsT | 88 | 7 | 88 ± 7 % | 12 | 1 | 12 ± 1 % |

(continued)

| duplex name | chemistry | position in consensus | sense strand SE QID No : | sense strand sequence (5'-3') | antisense strand SEQ ID NO: | antisense strand sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12809 | c | 4261-4279 | 299 | AaGAcccuAAAGAcuuuccTsT | 300 | GGAAAGUCUUuAGGGUCUUTsT | 67 | 6 | 67 ± 6 % | 35 | 4 | 35 ± 4 % |
| AD-12855 | d | 4261-4279 | 301 | AaGAcccuAAAGAcuuuccTsT | 302 | GgAAAGUCUUuAGGCUCUUTsT | 88 | 10 | 88 ± 10 % | 12 | 2 | 12 ± 2 % |
| AD-12652 | a | 1412-1430 | 303 | uGGAuGuuGccuuuAcuuuTsT | 304 | AaAGuAAAGGcAACAUCcATsT | 91 | 2 | 91 ± 2 % | 10 | 0 | 10 ± 0 % |
| AD-12755 | b | 1412-1430 | 305 | uGGAuGuuGccuuuAcuuuTsT | 306 | AAAGuAAAGGcAAcAUCcATsT | 40 | 3 | 40 ± 3 % | 67 | 6 | 87 ± 6 % |
| AD-12810 | c | 1412-1d30 | 307 | ugGAuGuuGccuuuAcuuuTsT | 308 | AAAGuAAAGGcAAcAUCcATsT | 35 | 1 | 35 ± 1 % | 72 | 3 | 72 ± 3 % |
| AD-12856 | d | 1412-1430 | 309 | ugGAuGuuGccuuuAcuuuTsT | 310 | AaAGuAAAGGcAACAUCcATsT | 75 | 8 | 75 ± 8 % | 28 | 4 | 28 ± 4 % |
| AD-12653 | a | 4592-4610 | 311 | uuuGAuuGcuucAGAcAAuTsT | 312 | AuUGUCUGAAGcAAUcAAATsT | 79 | 8 | 79 ± 8 % | 23 | 3 | 23 ± 3 % |
| AD-12756 | b | 4592-4610 | 313 | uuuGAuuGcuucAGAcAAuTsT | 314 | AUUGUCUGAAGcAAUcAAATsT | 17 | 5 | 17 ± 5 % | 86 | 27 | 86 ± 27 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12654 | a | 4991-5009 | 315 | AAuAGGGAGGAAUccAuGGTsT | 316 | CcAUGGAUUCCUCCCuAUUTsT | 97 | 6 | 97 ± 6 % | 3 | 0 | 3 ± 0 % |
| AD-12811 | c | 4991-5009 | 317 | AauAGGGAGGAAuccAuGGTsT | 318 | CcAUGGAUUCCUCCCuAUUTsT | 74 | 5 | 74 ± 5 % | 27 | 2 | 27 ± 2 % |
| AD-12655 | a | 5004-5022 | 319 | GGAcAAAGuGcuGAAuAGGTsT | 320 | CcuAUUcAGcACUUUGUCCTsT | 46 | 6 | 46 ± 6 % | 59 | 9 | 59 ± 9 % |
| AD-12757 | b | 5004-5022 | 321 | GGAcAAAGuGcuGAAUAGGTsT | 322 | CCuAUUcAGcACUUUGUCCTsT | 14 | 0 | 14 ± 0 % | 89 | 2 | 89 ± 2 % |
| AD-12812 | c | 5004-5022 | 323 | GgAcAAAGuGcuGAAuAGGTsT | 324 | CCuAUUcAGcACUUUGUCCTsT | 12 | 3 | 12 ± 3 % | 92 | 28 | 92 ± 28 % |
| AD-12857 | d | 5004-5022 | 325 | GgAcAAAGuGcuGAAuAGGTsT | 326 | CcuAUUcAGcACUUUGUCCTsT | 35 | 7 | 35 ± 7 % | 70 | 17 | 70 ± 17 % |
| AD-12656 | a | 5005-5023 | 327 | uGGAcAAAGuGcuGAAuAGTsT | 328 | CuAUUcAGcACUUUGUCcATsT | 10 | 3 | 10 ± 3 % | 99 | 33 | 99 ± 33 % |
| AD-12813 | c | 5005-5023 | 329 | ugGAcAAAGuGcuGAAuAGTsT | 330 | CuAUUcAGcACUUUGUCcATsT | 9 | 1 | 9 ± 1 % | 95 | 18 | 95 ± 18 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12657 | a | 654-672 | 331 | AAAuuGcAucccuuGcuAcTsT | 332 | GuAGcAAGGGAUGcAAUUUTsT | 108 | 1 | 108 ± 1 % | -9 | 0 | -9 ± 0 % |
| AD-12814 | c | 654-672 | 333 | AaAuuGcAucccuuGcuAcTsT | 334 | GuAGcAAGGGAUGcAAUUUTsT | 101 | 4 | 101 ± 4 % | -1 | 0 | -1 ± 0 % |
| AD-12658 | a | 659-677 | 335 | GcAucccuuGcuAcuGuAGTsT | 336 | CuAcAGuAGcAAGGGAUGCTsT | 98 | 9 | 98 ± 9 % | 2 | 0 | 2 ± 0 % |
| AD-12659 | a | 4273-4291 | 337 | AAAAAAAGGuAGAAGAcccTsT | 338 | GgGUCUUCuACCUUUUUUUTsT | 83 | 4 | 83 ± 4 % | 18 | 1 | 18 ± 1 % |
| AD-12758 | b | 4273-4291 | 339 | AAAAAAAGGuAGAAGAcccTsT | 340 | GGGUCUUCuACCUUUUUUUTsT | 80 | 14 | 80 ± 14 % | 21 | 4 | 21 ± 4 % |
| AD-12815 | c | 4273-4291 | 341 | AaAAAAAGGuAGAAGACCCTsT | 342 | GGGUCUUCuACCUUUUUUUTsT | 25 | 3 | 25 ± 3 % | 79 | 11 | 79 ± 11 % |
| AD-12858 | d | 4273-4291 | 343 | AaAAAAAGGuAGAAGAcccTsT | 344 | GgGUCUUCuACCUUUUUUUTsT | 67 | 4 | 67 ± 4 % | 35 | 2 | 35 ± 2 % |
| AD-12660 | a | 2025-2043 | 345 | AcAGAGcAcAAGGcGuAccTsT | 346 | GguACGCCUUGUGCUCUGUTsT | 95 | 11 | 95 ± 11 % | 8 | 3 | 8 ± 3 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12759 | b | 2025-2043 | 347 | AcAGAGcAcAAGGcGuAccTsT | 348 | GGuACGCCUUGUGCUCUGUTsT | 66 | 7 | 66 ± 7 % | 39 | 6 | 39 ± 6 % |
| AD-12661 | a | 4791-4809 | 349 | uGAuuuuGGuAcAuGGAAuTsT | 350 | AuUCcAUGuACcAAAAUcATsT | 34 | 2 | 34 ± 2 % | 73 | 5 | 73 ± 5 % |
| AD-12760 | b | 4791-4809 | 351 | uGAuuuuGGuAcAuGGAAuTsT | 352 | AUUCcAUGuACcAAAAUcATsT | 10 | 3 | 10 ± 3 % | 94 | 30 | 94 ± 30 % |
| AD-12816 | c | 4791-4809 | 353 | ugAuuuuGGuAcAuGGAAuTsT | 354 | AUUCcAUGuACcAAAAUcATsT | 12 | 4 | 12 14 % | 92 | 37 | 92 ± 37 % |
| AD-12859 | d | 4791-4809 | 355 | ugAuuuuGGuAcAuGGAAuTsT | 356 | AuUCcAUGuACcAAAAUcATsT | 33 | 1 | 33 ± 1 % | 72 | 2 | 72 ± 2 % |
| AD-12662 | a | 1433-1451 | 357 | GGGuuGuAcGGGAcuGuAATsT | 358 | UuAcAGUCCCGuAcAACCCTsT | 92 | 7 | 92 ± 7 % | 7 | 1 | 7 ± 1 % |
| AD-12817 | c | 1433-1451 | 359 | GgGuuGuAcGGGAcuGuAATsT | 360 | UuAcAGUCCCGuAcAACCCTsT | 91 | 11 | 91 ± 11 % | 10 | 2 | 10 ± 2 % |
| AD-12663 | a | 1434-1452 | 361 | GGuuGuAcGGGAcuGuAAcTsT | 362 | GuuAcAGUCCCGuAcAACCTsT | 99 | 10 | 99 ± 10 % | 3 | 1 | 3 ± 1 % |

(continued)

| duplex name | chemistry | position in consensus | sense strand SE QID No : | sense strand sequence (5'-3') | antisense strand SEQ ID NO: | antisense strand sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12761 | b | 1434-1452 | 363 | GGuuGuAcGGGAcuGuAAcTsT | 364 | GUuAcAGUCCCGuAcAACCTsT | 20 | 5 | 20 ± 5 % | 89 | 22 | 89 ± 22 % |
| AD-12818 | c | 1434-1452 | 365 | GguuGuAcGGGAcuGuAAcTsT | 366 | GUuAcAGUCCCGuAcAACCTsT | 20 | 4 | 20 ± 4 % | 90 | 20 | 90 ± 20 % |
| AD-12860 | d | 1434-1452 | 367 | GguuGuAcGGGAcuGuAAcTsT | 368 | GuuAcAGUCCCGuAcAACCTsT | 93 | 11 | 93 ± 11 % | 8 | 1 | 8 ± 1 % |
| AD-12664 | a | 1440-1458 | 369 | AcGGGAcuGuAAcAccuGcTsT | 370 | GcAGGUGUuAcAGUCCCGUTsT | 93 | 9 | 93 ± 9 % | 6 | 2 | 6 ± 2 % |
| AD-12665 | a | 1442-1460 | 371 | GGGAcuGuAAcAccuGcucTsT | 372 | GaGcAGGUGUuAcAGUCCCTsT | 94 | 8 | 94 ± 8 % | 10 | 1 | 10 ± 1 % |
| AD-12762 | b | 1442-1460 | 373 | GGGAcuGuAAcAccuGcucTsT | 374 | GAGcAGGUGUuAcAGUCCCTsT | 58 | 8 | 58 ± 8 % | 47 | 10 | 47 ± 10 % |
| AD-12819 | c | 1442-1460 | 375 | GgGAcuGuAAcAccuGcucTsT | 376 | GAGcAGGUGUuAcAGUCCCTsT | 49 | 6 | 49 ± 6 % | 58 | 9 | 58 ± 9 % |
| AD-12861 | d | 1442-1460 | 377 | GgGAcuGuAAcAccuGcucTsT | 378 | GaGcAGGUGUuACAGUCCCTsT | 93 | 8 | 93 ± 8 % | 8 | 1 | 8 ± 1 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12666 | a | 1608-1626 | 379 | AcuccAGAAAuGGGuGAccTsT | 380 | GgUcACCcAUUUCUGGAGUTsT | 30 | 5 | 30 ± 5 % | 76 | 18 | 76 ± 18 % |
| AD-12763 | b | 1608-1626 | 381 | AcuccAGAAAuGGGuGAccTsT | 382 | GGUcACCcAUUUCUGGAGuTsT | 25 | 2 | 25 ± 2 % | 84 | 9 | 84 ± 9 % |
| AD 12667 | a | 4793-4811 | 383 | ccuGAuuuuGGuAcAuGGATsT | 384 | UccAUGuACcAAAAUcAGGTsT | 65 | 10 | 65 ± 10 % | 38 | 7 | 38 ± 7 % |
| AD-12764 | b | 4793-4811 | 385 | ccuGAuuuuGGuAcAuGGATsT | 386 | UCcAUGuACcAAAAUcAGGTsT | 34 | 7 | 34 ± 7 % | 69 | 17 | 69 ± 17 % |
| AD-12668 | a | 5001-5019 | 387 | cAAAGuGcuGAAuAGGGAGTsT | 388 | CuCCCuAUUcAGcACUUUGTsT | 34 | 4 | 34 ± 4 % | 73 | 10 | 73 ± 10 % |
| AD-12765 | b | 5001-5019 | 389 | cAAAGuGcuGAAuAGGGAGTsT | 390 | CUCCCuAUUcAGcACUUUGTsT | 13 | 3 | 13 ± 3 % | 91 | 22 | 91 ± 22 % |
| AD-12820 | c | 5001-5019 391 | | caAAGuGcuGAAuAGGGAGTsT | 392 | CUCCCuAUUcAGcACUUUGTsT | 11 | 2 | 11 ± 2 % | 93 | 17 | 93 ± 17 % |
| AD-12862 | d | 5001-5019 | 393 | caAAGuGcuGAAuAGGGAGTsT | 394 | CuCCCuAUUcAGcACUUUGTsT | 19 | 4 | 19 ± 4 % | 87 | 22 | 87 ± 22 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12669 | a | 5066-5084 | 395 | ccAGGGAAAuucccuuGuuTsT | 396 | AacAAGGGAAUUUCCCUGGTsT | 22 | 3 | 22 ± 3 % | 87 | 12 | 87 ± 12 % |
| AD-12766 | b | 5066-5084 | 397 | ccAGGGAAAuucccuuGuuTsT | 398 | AAcAAGGGAAUUUCCCUGGTsT | 11 | 4 | 11 ± 4 % | 93 | 39 | 93 ± 39 % |
| AD-12670 | a | 5069-5087 | 399 | AGGccAGGGAAAuucccuuTsT | 400 | AaGGGAAUUUCCCUGGCCUTsT | 45 | 3 | 45 ± 3 % | 61 | 5 | 61 ± 5 % |
| AD-12767 | b | 5069-5087 | 401 | AGGccAGGGAAAuucccuuTsT | 402 | AAGGGAAUUUCCCUGGCCUTsT | 10 | 3 | 10 ± 3 % | 94 | 31 | 94 ± 31 % |
| AD-12821 | c | 5069-5087 | 403 | AgGccAGGGAAAuucccuuTsT | 404 | AAGGGAAUUUCCCUGGCCUTsT | 12 | 1 | 12 ± 1 % | 92 | 13 | 92 ± 13 % |
| AD-12863 | d | 5069-5087 | 405 | AgGccAGGGAAAuucccuuTsT | 406 | AaGGGAAUUUCCCUGGCCUTsT | 41 | 4 | 41 ± 4 % | 64 | 8 | 64 ± 8 % |
| AD-12671 | a | 564-582 | 407 | uAGuuGcuAcuGuuucuGATsT | 408 | UcAGAAAcAGuAGcAACuATsT | 83 | 9 | 83 ± 9 % | 19 | 2 | 19 ± 2 % |
| AD-12822 | c | 564-582 | 409 | uaGuuGcuAcuGuuucuGATsT | 410 | UcAGAAAcAGuAGcAACuATsT | 74 | 7 | 74 ± 7 % | 29 | 3 | 29 ± 3 % |

(continued)

| duplex name | chemistry | position in consensus | sense strand SE QID No : | sequence (5'-3') | antisense strand SEQ ID NO: | sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12672 | a | 633-651 | 411 | cuGcuGcuAcuAuAGAAGuTsT | 412 | AcUUCuAuAGuAGcAGcAGTsT | 52 | 7 | 52 ± 7 % | 54 | 9 | 54 ± 9 % |
| AD-12768 | b | 633-651 | 413 | cuGcuGcuAcuAuAGAAGuTsT | 414 | ACUUCuAuAGuAGcAGcAGTsT | 28 | 3 | 28 ± 3 % | 81 | 12 | 81 ± 12 % |
| AD-12673 | a | 634-652 | 415 | uGcuGcuAcuAuAGAAGuuTsT | 416 | AaCUUCuAuAGuAGcAGcATsT | 56 | 5 | 56 ± 5 % | 49 | 5 | 49 ± 5 % |
| AD-12769 | b | 634-652 | 417 | uGcuGcuAcuAuAGAAGuuTsT | 418 | AACUUCuAuAGuAGcAGcATsT | 36 | 2 | 36 ± 2 % | 72 | 5 | 72 ± 5 % |
| AD-12823 | c | 634-652 | 419 | ugcuGcuAcuAuAGAAGuuTsT | 420 | AACUUCuAuAGuAGcAGcATsT | 33 | 2 | 33 ± 2 % | 75 | 5 | 75 ± 5 % |
| AD-12864 | d | 634-652 | 421 | ugcuGcuAcuAuAGAAGuuTsT | 422 | AaCUUCuAuAGuAGcAGcATsT | 49 | 7 | 49 ± 7 % | 57 | 10 | 57 ± 10 % |
| AD-12674 | a | 635-653 | 423 | GcuGcuAcuAuAGAAGuuGTsT | 424 | caACUUCuAuAGuAGcAGCTsT | 90 | 9 | 90 ± 9 % | 11 | 1 | 11 ± 1 % |
| AD-12770 | b | 635-653 | 425 | GcuGcuAcuAuAGAAGuuGTsT | 426 | cAACUUCuAuAGuAGcAGCTsT | 45 | 6 | 45 ± 6% | 61 | 9 | 61 ± 9% |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12675 | a | 636-654 | 427 | cuGcuAcuAuAGAAGuuGATsT | 428 | UcAACUUCuAuAGuAGcAGTsT | 45 | 5 | 45 ± 5% | 62 | 8 | 62 ± 8% |
| AD-12676 | a | 637-655 | 429 | uGcuAcuAuAGAAGuuGAATsT | 430 | UucAACWCuAuAGuAGcATsT | 47 | 6 | 47 ± 6% | 59 | 9 | 59 ± 9 % |
| AD-12771 | b | 637-655 | 431 | uGcuAcuAuAGAAGuuGAATsT | 432 | UUcAACUUCuAuAGuAGcATsT | 31 | 4 | 31 ± 4% | 77 | 11 | 77 ±11 % |
| AD-12824 | c | 637-655 | 433 | ugcuAcuAuAGAAGuuGAATsT | 434 | UUcAACUUCuAuAGuAGcATsT | 31 | 3 | 31 ± 3 % | 77 | 10 | 77 ± 10 % |
| AD-12865 | d | 637-655 | 435 | ugcuAcuAuAGAAGuuGAATsT | 436 | UucAACWCuAuAGuAGcATsT | 43 | 7 | 43 ± 7 % | 64 | 12 | 64 ± 12 % |
| AD-12677 | a | 912-930 | 437 | cAGAAGAcuAcuAuGAuAuTsT | 438 | AuAUcAuAGuAGUCUUCUGTsT | 23 | 4 | 23 ± 4 % | 86 | 16 | 86 ± 16 % |
| AD-12825 | c | 912-930 | 439 | caGAAGAcuAcuAuGAuAuTsT | 440 | AuAUcAUAGuAGUCUUCUGTsT | 22 | 4 | 22 ± 4 % | 87 | 16 | 87 ± 16 % |
| AD-12678 | a | 4153-4171 | 441 | AAAuuuuAuAuAAGAAACuTsT | 442 | AgUUUCUuAuAuAAAAUUUTsT | 102 | 8 | 102 ± 8 % | -2 | 0 | -2 ± 0 % |

(continued)

| duplex name | chemistry | position in consensus | sense strand SE QID No : | sequence (5'-3') | antisense strand SEQ ID NO: | sequence (5'-3-) | Residual luciferase activity (relative siRNA treated cells) | SD of residu al luciferase activit y | Residual luciferase activity +/- SD | Relative siRNA activity (normali zed to control luc- siRNA) | SD of relati ve siRN A activi ty | Relative siRNA activity +/- SD |
|---|---|---|---|---|---|---|---|---|---|---|---|---|
| AD-12772 | b | 4153-4171 | 443 | AAAuuuuAuAuAAGAAAcuTsT | 444 | AGUUUCUuAuAuAAAAUUUTsT | 101 | 13 | 101 ± 13 % | -1 | 0 | -1 ± 0 % |
| AD-12826 | c | 4153-4171 | 445 | AaAuuuuAuAuAAGAAAcuTsT | 446 | AGUUUCUuAuAuAAAAUUUTsT | 99 | 1 | 99 ± 1 % | 1 | 0 | 1 ± 0 % |
| AD-12866 | d | 4153-4171 | 447 | AaAuuuuAuAuAAGAAAcuTsT | 448 | AgUUUCUuAuAuAAAAUUUTsT | 91 | 7 | 91 ± 7 % | 10 | 1 | 10 ± 1 % |
| AD-12679 | a | 4779-4797 449 | | AuGGAAuAGuucAGAGGuuTsT | 450 | AaCCUCUGAACuAUUCcAUTsT | 81 | 8 | 81 ± 8 % | 21 | 2 | 21 ± 2 % |
| AD-12773 | b | 4779-4797 | 451 | AuGGAAuAGuucAGAGGuuTsT | 452 | AACCUCUGAACuAUUCcAUTsT | 11 | 2 | 11 ± 2 % | 93 | 19 | 93 ± 19 % |
| AD-12680 | a | 4780-4798 | 453 | cAuGGAAuAGuucAGAGGuTsT | 454 | AcCUCUGAACuAUUCcAUGTsT | 17 | 3 | 17 ± 3 % | 92 | 17 | 92 ± 17 % |
| AD-12774 | b | 4780-4798 | 455 | cAuGGAAuAGuucAGAGGuTsT | 456 | ACCUCUGAACuAUUCcAUGTsT | 15 | 2 | 15 ± 2 % | 89 | 17 | 89 ± 17 % |
| AD-12827 | c | 4780-4798 | 457 | cauGGAAuAGuucAGAGGuTsT | 458 | ACCUCUGAACuAUUCcAUGTsT | 11 | 2 | 11 ± 2 % | 93 | 18 | 93 ± 18 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12867 | d | 4780-4798 | 459 | cauGGAAuAGuucAGAGGuTsT | 460 | AcCUCUGAACuAUUCcAUGTsT | 15 | 3 | 15 ± 3 % | 91 | 22 | 91 ± 22 % |
| AD-12681 | a | 4781-4799 | 461 | AcAuGGAAuAGuucAGAGGTsT | 462 | CcUCUGAACuAUUCcAUGUTsT | 28 | 3 | 28 ± 3 % | 79 | 10 | 79 ± 10 % |
| AD-12775 | b | 4781-9799 | 463 | AcAuGGAAuAGuucAGAGGTsT | 464 | CCUCUGAACuAUUCcAUGUTsT | 8 | 1 | 8 ± 1 % | 95 | 19 | 95 ± 19 % |
| AD-12682 | a | 4784-4802 | 465 | GGuACAuGGAAuAGuucAGTsT | 466 | CuGAACuAUUCcAUGuACCTsT | 43 | 6 | 43 ± 6 % | 63 | 9 | 63 ± 9 % |
| AD-12776 | b | 4784-4802 | 467 | GGuAcAuGGAAuAGuucAGTsT | 468 | CuGAACuAUUCcAUGuACCTsT | 23 | 5 | 23 ± 5 % | 80 | 19 | 80 ± 19 % |
| AD-12828 | c | 4784-4802 | 469 | GguAcAuGGAAuAGuucAGTsT | 470 | CuGAACuAUUCcAUGuACCTsT | 23 | 5 | 23 ± 5 % | 80 | 20 | 80 ± 20 % |
| AD-12868 | d | 4784-4802 | 471 | GguAcAuGGAAuAGuucAGTsT | 472 | CuGAACuAUUCcAUGuACCTsT | 25 | 4 | 25 ± 4 % | 81 | 16 | 81 ± 16 % |
| AD-12683 | a | 4785-4803 473 | | uGGuAcAuGGAAuAGuucATsT | 474 | UgAACuAUUCcAUGuACcATsT | 17 | 2 | 17 ± 2 % | 91 | 15 | 91 ± 15% |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12777 | b | 4785-4803 | 475 | uGGuAcAuGGAAuAGuucATsT | 476 | UGAACuAUUCcAUGuACcATsT | 11 | 2 | 11 ± 2 % | 92 | 22 | 92 ± 22 % |
| AD-12829 | c | 4785-4803 | 477 | ugGuAcAuGGAAuAGuucATsT | 478 | UGAACuAUUCcAUGuACcATsT | 12 | 1 | 12 ± 1 % | 92 | 11 | 92 ± 11 % |
| AD-12869 | d | 4785-4803 479 | | ugGuAcAuGGAAuAGuuCATST | 480 | UgAACuAUUCcAUGuACcATsT | 19 | 3 | 19 ± 3 % | 87 | 16 | 87 ± 16 % |
| AD-12684 | a | 719-737 | 481 | cuuAcuccuGAAAcAuAuGTsT | 482 | cauAUGUUUcAGGAGuAAGTsT | 87 | 12 | 87 ± 12 % | 14 | 2 | 14 ± 2 % |
| AD-12778 | b | 719-737 | 483 | cuuAcuccuGAAAcAuAuGTsT | 484 | cAuAUGUUUcAGGAGuAAGTsT | 41 | 4 | 41 ± 4 % | 66 | 8 | 66 ± 8 % |
| AD-12685 | a | 909-927 | 485 | AuccAGAAGAcuAcuAuGATsT | 486 | UcAuAGuAGUCUUCUGGAUTsT | 35 | 1 | 35 ± 1 % | 72 | 1 | 72 ± 1 % |
| AD-12686 | a | 1119-1137 | 487 | uuuuGGAAGAAAcuAcuuGTsT | 488 | caAGuAGUUUCUUCcAAAATsT | 68 | 5 | 68 × 5 % | 36 | 3 | 36 ± 3 % |
| AD-12779 | b | 1119-1137 | 489 | uuuuGGAAGAAAcuAcuuGTsT | 490 | cAAGuAGUUUCUUCcAAAATsT | 58 | 5 | 58 ± 5 % | 47 | 5 | 47 ± 5 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12687 | a | 1121-1139 | 491 | uuGGAAGAAAcuAcuuGGGTsT | 492 | CccAAGuAGUUUCUUCcAATsT | 73 | 8 | 73 ± 8 % | 30 | 4 | 30 ± 4 % |
| AD-12780 | b | 1121-1139 | 493 | uuGGAAGAAAcuAcuuGGGTsT | 494 | CCcAAGuAGUUUCUUCcAATsT | 62 | 8 | 62 ± 6 % | 42 | 7 | 42 ± 7 % |
| AD-12688 | a | 4357-4375 | 495 | AuGAAGAccuGuuuuGccATsT | 496 | UgGcAAAAcAGGUCUUcAUTsT | 18 | 1 | 18 ± 1 % | 91 | 4 | 91 ± 4 % |
| AD-12781 | b | 4357-4375 | 497 | AuGAAGAccuGuuuuGccATsT | 498 | UGGcAAAAcAGGUCUUcAUTsT | 11 | 3 | 11 ± 3 % | 93 | 33 | 93 ± 33 % |
| AD-12689 | a | 4358-4376 | 499 | GAuGAAGAccuGuuuuGccTsT | 500 | GgcAAAAcAGGUCUUcAUCTsT | 96 | 4 | 96 ± 4 % | 4 | 0 | 4 ± 0 % |
| AD-12782 | b | 4358-4376 | 501 | GAuGAAGAccuGuuuuGccTsT | 502 | GGcAAAAcAGGUCUUcAUCTsT | 45 | 7 | 45 ± 7 % | 58 | 10 | 58 ± 10 % |
| AD-12830 | c | 4358-4376 | 503 | GauGAAGACCUGuuuuGccTsT | 504 | GGcAAAAcAGGUCUUcAUCTsT | 15 | 3 | 15 ± 3 % | 89 | 19 | 89 ± 19 % |
| AD-12870 | d | 4358-4376 | 505 | GauGAAGAccuGuuuuGccTsT | 506 | GgcAAAAcAGGUCUUcAUCTsT | 51 | 3 | 51 ± 3 % | 52 | 4 | 52 ± 4 % |

(continued)

| duplex name | chemistry | position in consensus | sense strand SEQ ID No : | sense strand sequence (5'-3') | antisense strand SEQ ID NO: | antisense strand sequence (5'-3-) | Residual luciferase activity (relative siRNA treated cells) | SD of residual luciferase activity | Residual luciferase activity +/- SD | Relative siRNA activity (normalized to control luc- siRNA) | SD of relative siRNA activity | Relative siRNA activity +/- SD |
|---|---|---|---|---|---|---|---|---|---|---|---|---|
| AD-12690 | a | 4360-4378 | 507 | GGGAuGAAGAccuGuuuuGTsT | 508 | caAAAcAGGUCUUcAUCCCTsT | 93 | 6 | 93 ± 6 % | 8 | 1 | 8 ± 1 % |
| AD-12783 | b | 4360-4378 | 509 | GGGAuGAAGAccuGuuuuGTsT | 510 | cAAAAcAGGUCUUcAUCCCTsT | 36 | 3 | 36 ± 3 % | 66 | 7 | 66 ± 7 % |
| AD-12831 | c | 4360-4378 | 511 | GgGAuGAAGAccuGuuuuGTsT | 512 | cAAAAcAGGUCUUcAUCCCTsT | 27 | 2 | 27 ± 2 % | 76 | 7 | 76 ± 7 % |
| AD-12871 | d | 4360-4378 | 513 | GgGAuGAAGAccuGuuuuGTsT | 514 | caAAAcAGGUCUUcAUCCCTsT | 81 | 18 | 81 ± 18 % | 21 | 5 | 21 ± 5 % |

## Claims

1. A double-stranded ribonucleic acid (dsRNA) for inhibiting the expression of a human JC virus genome in a cell, wherein said dsRNA comprises at least two sequences that are complementary to each other and wherein a sense strand comprises a first sequence and an antisense strand comprises a second sequence comprising a region of complementarity which is substantially complementary to at least a part of a mRNA encoding JC virus, and wherein said region of complementarity is less than 30 nucleotides in length and wherein said dsRNA, upon contact with a cell expressing said JC virus, inhibits expression of said JC virus genome, wherein said first sequence is a partial sequence of at least 15 contiguous nucleotides of the sequence of SEQ ID NO: 131 and said second sequence is a partial sequence of at least 15 contiguous nucleotides of the sequence of SEQ ID NO: 132.

2. The dsRNA of claim 1, wherein said first sequence is the sequence of SEQ ID NO: 131 and said second sequence is the sequence of SEQ ID NO: 132.

3. The dsRNA of claim 1 or 2, wherein said dsRNA comprises at least one modified nucleotide.

4. The dsRNA of claim 3, wherein said modified nucleotide is chosen from the group of: a 2'-O-methyl modified nucleotide, a nucleotide comprising a 5'-phosphorothioate group, a terminal nucleotide linked to a cholesteryl derivative or dodecanoic acid bisdecylamide group, a 2'-deoxy-2'-fluoro modified nucleotide, a 2'-deoxy-modified nucleotide, a locked nucleotide, an abasic nucleotide, 2'-amino-modified nucleotide, 2'-alkyl-modified nucleotide, morpholino nucleotide, a phosphoramidate, and a non-natural base comprising nucleotide.

5. A cell comprising the dsRNA of claim 1.

6. A pharmaceutical composition for inhibiting the expression of a gene from the JC virus in an organism, comprising a dsRNA and a pharmaceutically acceptable carrier, wherein said dsRNA is as defined in claim 1.

7. A method for inhibiting the expression of a gene from the JC virus in a cell, the method comprising:

   (a) introducing into the cell a double-stranded ribonucleic acid (dsRNA), wherein said dsRNA is as defined in claim 1; and
   (b) maintaining the cell produced in step (a) for a time sufficient to obtain degradation of the mRNA transcript of a gene from the JC virus, thereby inhibiting expression of a gene from the JC virus in the cell,

   wherein any such method practiced as a method for treatment of the human or animal body by therapy or as a diagnostic method practiced on the human or animal body is excluded.

8. A dsRNA for use in treating, preventing or managing pathological processes mediated by JC virus expression, wherein said dsRNA is as defined in claim 1.

9. The pharmaceutical composition of claim 6, the method of claim 7, or the dsRNA for use in treating, preventing or managing pathological processes mediated by JC virus expression according to claim 8, wherein said first sequence is the sequence of SEQ ID NO: 131 and said second sequence is the sequence of SEQ ID NO: 132.

10. A vector for inhibiting the expression of a gene from the JC virus in a cell, said vector comprising a regulatory sequence operably linked to a nucleotide sequence that encodes at least one strand of a dsRNA, wherein said dsRNA is as defined in claim 1.

11. A cell comprising the vector of claim 10.

## Patentansprüche

1. Doppelsträngige Ribonukleinsäure (dsRNA) zur Inhibierung der Expression eines menschlichen JC-Virusgenoms in einer Zelle, wobei die dsRNA mindestens zwei Sequenzen umfasst, die komplementär zueinander sind, und wobei ein Sinnstrang eine erste Sequenz umfasst und ein Antisinnstrang eine zweite Sequenz umfasst, umfassend eine Region der Komplementarität, die im Wesentlichen komplementär zumindest zu einem Teil einer mRNA ist, die das JC-Virusgenom kodiert, und wobei die Region der Komplementarität weniger als 30 Nukleotide lang ist und wobei die dsRNA auf den Kontakt mit einer Zelle hin, die das JC-Virus exprimiert, die Expression des JC-Virusgenoms inhibiert, wobei die erste Sequenz eine Teilsequenz von mindestes 15 zusammenhängenden Nukleotiden der Sequenz der SEQ ID NO: 131 ist und die zweite Sequenz eine Teilsequenz von mindestens 15 zusammenhängenden Nukleotiden der Sequenz der SEQ ID NO: 132 ist.

**2.** dsRNA nach Anspruch 1, wobei die erste Sequenz die Sequenz der SEQ ID NO: 131 ist und die zweite Sequenz die Sequenz der SEQ ID NO: 132 ist.

**3.** dsRNA nach Anspruch 1 oder 2, wobei die dsRNA mindestens ein modifiziertes Nukleotid umfasst.

**4.** dsRNA nach Anspruch 3, wobei das modifizierte Nukleotid aus der Gruppe ausgewählt ist: 2'-O-Methyl-modifiziertes Nukleotid, ein Nukleotid umfassend eine 5'-Phosphorothioatgruppe, ein terminales Nukleotid verknüpft mit einem Cholesterylderivat oder einer Dodecansäurebisdecylamidgruppe, ein 2'-Desoxy-2'-Fluor-modifiziertes Nukleotid, 2'-Desoxy-modifiziertes Nukleotid, ein verbrücktes Nukleotid, ein nicht-basisches Nukleotid, 2'-Amino-modifiziertes Nukleotid, 2'-Alkyl-modifiziertes Nukleotid, ein Morpholinonukleotid, ein Phosphoramidat und ein eine nicht-natürliche Base umfassendes Nukleotid.

**5.** Zelle umfassend die dsRNA nach Anspruch 1.

**6.** Pharmazeutische Zusammensetzung zur Inhibierung der Expression eines Gens des JC-Virus in einem Organismus, umfassend eine dsRNA und einen pharmazeutisch verträglichen Träger, wobei die dsRNA wie im Anspruch 1 definiert ist.

**7.** Verfahren zur Inhibierung der Expression eines Gens des JC-Virus in einer Zelle, wobei das Verfahren umfasst:

(a) Einbringen einer doppelsträngigen Ribonukleinsäure (dsRNA) in die Zelle, wobei die dsRNA wie im Anspruch 1 definiert ist, und
(b) Erhalten der im Schritt (a) produzierten Zelle für eine Zeit, die ausreicht, den Abbau des mRNA-Transkripts eines Gens des JC-Virus zu bewirken, wodurch die Expression eines Gens des JC-Virus in der Zelle inhibiert wird,

wobei ein jegliches solches Verfahren, vorgenommen als Verfahren zur therapeutischen Behandlung des menschlichen oder tierischen Körpers oder als ein Diagnostizierverfahren, vorgenommen am menschlichen oder tierischen Körper, ausgenommen ist.

**8.** dsRNA zur Verwendung in der Behandlung, Prävention oder Bewältigung eines pathologischen Prozesses, der durch JC-Virus-Expression herbeigeführt wird, wobei die dsRNA wie im Anspruch 1 definiert ist.

**9.** Pharmazeutische Zusammensetzung nach Anspruch 6, Verfahren nach Anspruch 7, oder dsRNA zur Verwendung in der Behandlung, Prävention oder Bewältigung eines pathologischen Prozesses, der durch JC-Virus-Expression herbeigeführt wird, nach Anspruch 8, wobei die erste Sequenz die Sequenz der SEQ ID NO: 131 ist und die zweite Sequenz die Sequenz der SEQ ID NO: 132 ist.

**10.** Vektor zur Inhibierung der Expression eines Gens des JC-Virus in einer Zelle, wobei der Vektor eine regulatorische Sequenz umfasst, die funktionell mit einer Nukleotidsequenz verknüpft ist, die mindestens einen Strang einer dsRNA kodiert, wobei die dsRNA wie im Anspruch 1 definiert ist.

**11.** Zelle umfassend den Vektor nach Anspruch 10.

## Revendications

**1.** Acide ribonucléique double brin (ARNdb) pour inhiber l'expression d'un génome du virus JC humain dans une cellule, ledit ARNdb comprenant au moins deux séquences qui sont complémentaires l'une de l'autre et dans lequel un brin sens comprend une première séquence et un brin anti-sens comprend une seconde séquence comprenant une région de complémentarité laquelle est substantiellement complémentaire d'au moins une partie d'un ARNm codant le virus JC, et dans lequel ladite région de complémentarité est moins de 30 nucléotides en longueur et ledit ARNdb, par contact avec une cellule exprimant ledit virus JC, inhibe l'expression du génome dudit virus JC, dans lequel ladite première séquence est une séquence partielle d'au moins 15 nucléotides contigus de la séquence représentée par SEQ ID NO: 131 et ladite seconde séquence est une séquence partielle d'au moins 15 nucléotides contigus de la séquence représentée par SEQ ID NO : 132.

**2.** ARNdb selon la revendication 1, dans lequel ladite première séquence est la séquence représentée par SEQ ID NO: 131 et ladite seconde séquence est la séquence représentée par SEQ ID NO : 132.

**3.** ARNdb selon la revendication 1 ou 2, dans lequel ledit ARNdb comprend au moins un nucléotide modifié.

**4.** ARNdb selon la revendication 3, dans lequel ledit nucléotide modifié est choisi dans le groupe de : un nucléotide modifié par un 2'-O-méthyl, un nucléotide comprenant un groupement 5'-phosphorothioate, un nucléotide terminal lié à un dérivé du cholestéryle ou un groupement bisdecylamide d'acide dodécanoique, un nucléotide modifié par un 2'-desoxy-2'-fluoro, un nucléotide modifié par 2'-desoxy, un nucléotide verrouillé, un nucléotide abasique, un nucléotide modifié par un 2'-amino, un nucléotide modifié par un 2'-alkyl, un nucléotide morpholino, un amidate de phosphore, et une base non naturelle comprenant nucléotide.

**5.** Cellule comprenant l'ARNdb selon la revendication 1.

**6.** Composition pharmaceutique pour inhiber l'expression du gène du virus JC dans un organisme, comprenant un ARNdb et un support pharmaceutiquement acceptable, dans laquelle ledit ARNdb est tel que défini dans la revendication 1.

**7.** Procédé pour inhiber l'expression d'un gène du virus JC dans une cellule, le procédé comprenant :

(a) l'introduction dans une cellule d'un acide ribonucléique double-brin (ARNdb), dans lequel ledit ARNdb est tel que défini dans la revendication 1 ; et
(b) le maintien de la cellule produite à l'étape (a) pendant un temps suffisant pour obtenir la dégradation du transcrit ARNm d'un gène du virus JC, inhibant ainsi l'expression d'un gène du virus JC dans la cellule,

dans lequel tout telle méthode mise en oeuvre en tant que méthode de traitement du corps humain ou animal par thérapie ou en tant que méthode de diagnostic exercée sur le corps humain ou animal est exclue.

**8.** ARNdb pour son utilisation dans le traitement, la prévention ou la gestion des processus pathologiques médiés par l'expression du virus JC, dans lequel ledit ARNdb est tel que défini dans la revendication 1.

**9.** Composition pharmaceutique selon la revendication 6, le procédé selon la revendication 7, ou l'ARNdb pour son utilisation dans le traitement, la prévention ou la gestion des processus pathologiques médiés par l'expression du virus JC selon la revendication 8, dans lesquels ladite première séquence est la séquence représenté par SEQ ID NO : 131 et ladite seconde séquence est la séquence représentée par SEQ ID NO : 132.

**10.** Vecteur pour inhiber l'expression d'un gène du virus JC dans une cellule, ledit vecteur comprenant une séquence régulatrice liée de manière opérante à une séquence nucléotidique qui code pour au moins un brin de l'ARNdb, dans lequel ledit ARNdb est tel que défini dans la revendication 1.

**11.** Cellule comprenant le vecteur selon la revendication 10.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9932619 A, Fire
- WO 9953050 A
- WO 9961631 A
- WO 0044895 A, Limmer
- DE 10100586, Kreutzer
- WO 2005112636 A
- WO 2005113014 A
- US 3687808 A
- US 4469863 A
- US 4476301 A
- US 5023243 A
- US 5177195 A
- US 5188897 A
- US 5264423 A
- US 5276019 A
- US 5278302 A
- US 5286717 A
- US 5321131 A
- US 5399676 A
- US 5405939 A
- US 5453496 A
- US 5455233 A
- US 5466677 A
- US 5476925 A
- US 5519126 A
- US 5536821 A
- US 5541316 A
- US 5550111 A
- US 5563253 A
- US 5571799 A
- US 5587361 A
- US 5625050 A
- US 5034506 A
- US 5166315 A
- US 5185444 A
- US 5214134 A
- US 5216141 A
- US 5235033 A
- US 564562 A
- US 5264564 A
- US 5405938 A
- US 5434257 A
- US 5470967 A
- US 5489677 A
- US 5541307 A
- US 5561225 A
- US 5596086 A
- US 5602240 A
- US 5608046 A
- US 5610289 A
- US 5618704 A
- US 5623070 A
- US 5663312 A
- US 5633360 A
- US 5677437 A
- US 5677439 A
- US 5539082 A
- US 5714331 A
- US 5719262 A
- US 4981957 A
- US 5118800 A
- US 5319080 A
- US 5359044 A
- US 5393878 A
- US 5446137 A
- US 3466786 A
- US 5514785 A
- US 5519134 A
- US 5567811 A
- US 5576412 A
- US 5591722 A
- US 5597909 A
- US 5610300 A
- US 5627053 A
- US 5639873 A
- US 5646265 A
- US 5658873 A
- US 5670633 A
- US 5700920 A
- US 4845205 A
- US 513030 A
- US 5134066 A
- US 5175273 A
- US 5367066 A
- US 5432272 A
- US 5457187 A
- US 5459255 A
- US 5484908 A
- US 5502177 A
- US 5525711 A
- US 5552540 A
- US 5587469 A
- US 5594121 A
- US 5596091 A
- US 5614617 A
- US 5681941 A
- US 5750692 A
- US 4828979 A

- US 4948882 A
- US 5218105 A
- US 5525465 A
- US 5541313 A
- US 5545730 A
- US 5552538 A
- US 5578717 A
- US 5580731 A
- US 5591584 A
- US 5109124 A
- US 5118802 A
- US 5138045 A
- US 5414077 A
- US 5486603 A
- US 5512439 A
- US 5578718 A
- US 4587044 A
- US 4605735 A
- US 4667025 A
- US 4762779 A
- US 4789737 A
- US 4824941 A
- US 4835263 A
- US 4876335 A
- US 4904582 A
- US 4958013 A
- US 5082830 A
- US 5112963 A
- US 5214136 A
- US 5245022 A
- US 5254469 A
- US 5258506 A
- US 5262536 A
- US 5272250 A
- US 5292873 A
- US 5317098 A
- US 5371241 A
- US 5391723 A
- US 5416203 A
- US 5451463 A
- US 5510475 A
- US 5512667 A
- US 5565552 A
- US 5567810 A
- US 5574142 A
- US 5585481 A
- US 5587371 A
- US 5595726 A
- US 5597696 A
- US 5599923 A
- US 5599928 A
- US 5688941 A
- US 5252479 A
- US 5139941 A
- WO 9413788 A
- WO 9324641 A
- US 4837028 A
- WO 8804924 A
- US 5543152 A, Webb
- WO 9713499 A, Lim
- US 4426330 A, Sears
- US 4534899 A
- EP 0445131 B1
- WO 9004384 A
- US 5013556 A
- US 5356633 A
- US 3213804 A
- EP 0496813 B1
- WO 9105545 A
- US 5225212 A, Martin
- WO 9420073 A, Zalipsky
- WO 9610391 A, Choi
- US 5540935 A, Miyazaki
- US 5556948 A, Tagawa
- WO 9640062 A, Thierry
- US 5264221 A, Tagawa
- US 5665710 A, Rahman
- WO 9704787 A, Love
- US 5705188 A, Junichi
- WO 9730731 A, Lollo
- WO 0022113 A, Skillern, A.
- WO 0022114 A, Conrad
- US 6054299 A, Conrad
- US 4868116 A
- US 4980286 A
- WO 8907136 A
- WO 8902468 A
- WO 8905345 A
- WO 9207573 A
- US 5328470 A
- EP 07761452 A
- EP 60795765 A

**Non-patent literature cited in the description**

- **BERGER, J. R.** *J. Neurovirol.,* 1995, vol. 1, 5-18
- **CINQUE, P.** *J. Neurovirol.,* 2003, vol. 9 (1), 88-92
- **CLIFFORD, D. B.** *J. Neurovirol.,* 2001, vol. 4, 279
- **SWEENEY, B. J.** *J. Neurol. Neurosurg. Psychiatry,* 1994, vol. 57, 994-997
- **GORDON, J.** *Int. J. Mol. Med.,* 1998, vol. 1, 647-655
- **RAJ, G. V.** *Virology,* 1995, vol. 10, 283-291
- **GORDON, J.** *Int. J. Mol. Med.,* 1998, vol. 1, 647-653
- **KHALIL, K.** *Oncogene,* 2003, vol. 22, 5181-5191

- **DARBINYAN, A.** *Oncogene,* 2002, vol. 21, 5574-5581
- **AKSAMIT, A.** *J. Neurovirol.,* 2001, vol. 7, 386-390
- **LEVY, R. M.** *J. Neurovirol.,* 2001, vol. 7, 382-385
- **ROYAL, W., III.** *J. Neurovirol.,* 2003, vol. 9, 411-419
- **YANG, D. et al.** *Curr. Biol.,* 2000, vol. 10, 1191-1200
- **RADHAKRISHNAN, S.** *J. Vir.,* vol. 78, 7264-7269
- **ORBA, Y.** *J. Vir.,* 2004, vol. 78, 7270-7273
- **ORBA et al.** *Journal of NeuroVirology,* 2003, vol. 9 (3), 128
- **RADHAKRISHNAN et al.** *Journal of Virology,* 2004, vol. 78 (13), 7264-7269
- **ELBASHIR et al.** *EMBO,* 2001, vol. 20, 6877-6888
- Current protocols in nucleic acid chemistry. John Wiley & Sons. Inc,
- **NIELSEN et al.** *Science,* 1991, vol. 254, 1497-1500
- **MARTIN et al.** *Helv. Chim. Acta,* 1995, vol. 78, 486-504
- The Concise Encyclopedia Of Polymer Science And Engineering. John Wiley & Sons, 1990, 858-859
- **ENGLISCH et al.** *Angewandte Chemie,* 1991, vol. 30, 613
- **SANGHVI, Y S.** DsRNA Research and Applications. CRC Press, 1993, 289-302
- DsRNA Research and Applications. CRC Press, 1993, 276-278
- **LETSINGER et al.** *Proc. Natl. Acid. Sci. USA,* vol. 86, 6553-6556
- **MANOHARAN et al.** *Biorg. Med. Chem. Let.,* 1994, 4 1053-1060
- **MANOHARAN et al.** *Ann. N.Y. Acad. Sci.,* 1992, vol. 660, 306-309
- **MANOHARAN et al.** *Biorg. Med. Chem. Let.,* 1993, vol. 3, 2765-2770
- **OBERHAUSER et al.** *Nucl. Acids Res.,* 1992, vol. 20, 533-538
- **SAISON-BEHMOARAS et al.** *EMBO J,* 1991, vol. 10, 1111-1118
- **KABANOV et al.** *FEBS Lett.,* 1990, vol. 259, 327-330
- **SVINARCHUK et al.** *Biochimie,* 1993, vol. 75, 49-54
- **MANOHARAN et al.** *Tetrahedron Lett.,* 1995, vol. 36, 3651-3654
- **SHEA et al.** *Nucl. Acids Res.,* 1990, vol. 18, 3777-3783
- **MANOHARAN et al.** *Nucleosides & Nucleotides,* 1995, vol. 14, 969-973
- **MISHRA et al.** *Biochim. Biophys. Acta,* 1995, vol. 1264, 229-237
- **CROOKE et al.** *J. Pharmacol. Exp. Ther.,* 1996, vol. 277, 923-937
- **LETSINGER et al.** *Proc. Natl. Acad. Sci. USA,* 1989, vol. 86, 6553
- **MANOHARAN et al.** *Bioorg. Med. Chem. Lett.,* 1994, vol. 4, 1053
- **MANOHARAN et al.** *Ann. N.Y. Acad. Sci.,* 1992, vol. 660, 306
- **MANOHARAN et al.** *Bioorg. Med. Chem. Let.,* 1993, vol. 3, 2765
- **OBERHAUSER et al.** *Nucl. Acids Res.,* 1992, vol. 20, 533
- **SAISON-BEHMOARAS et al.** *EMBO J.,* 1991, vol. 10, 111
- **KABANOV et al.** *FEBS Lett.,* 1990, vol. 259, 327
- **SVINARCHUK et al.** *Biochimie,* vol. 75, 49
- **MANOHARAN et al.** *Tetrahedron Lett.,* 1995, vol. 36, 3651
- **SHEA et al.** *Nucl. Acids Res.,* 1990, vol. 18, 3777
- **MANOHARAN et al.** *Nucleosides & Nucleotides,* 1995, vol. 14, 969
- **MANOHARAM et al.** *Tetrahedron Lett.,* 1995, vol. 36, 3651
- **MISHRA et al.** *Biochim. Biophys. Acta,* 1995, vol. 1264, 229
- **CROOKE et al.** *J. Pharmacol. Exp. Ther.,* 1996, vol. 277, 923
- **RABINOWITZ J E et al.** *J Virol,* 2002, vol. 76, 791-801
- **DORNBURG R.** *Gene Therap.,* 1995, vol. 2, 301-310
- **EGLITIS M A.** *Biotechniques,* 1988, vol. 6, 608-614
- **MILLER A D.** *Hum Gene Therap.,* vol. 1, 5-14
- **ANDERSON W F.** *Nature,* vol. 392, 25-30
- **RUBINSON D A et al.** *Nat. Genet.,* vol. 33, 401-406
- **XIA H et al.** *Nat. Biotech.,* 2002, vol. 20, 1006-1010
- **SAMULSKI R et al.** *J. Virol.,* 1987, vol. 61, 3096-3101
- **FISHER K J et al.** *J. Virol,* 1996, vol. 70, 520-532
- **SAMULSKI R et al.** *J. Virol.,* 1989, vol. 63, 3822-3826
- **ROSOFF.** Pharmaceutical Dosage Forms. Marcel Dekker, Inc, 1988, vol. 1, 245
- **WANG et al.** *Biochem. Biophys. Res. Comman.,* 1987, vol. 147, 980-985
- **ZHOU et al.** *Journal of Controlled Release,* 1992, vol. 19, 269-274
- **WEINER et al.** *Journal of Drug Targeting,* 1992, vol. 2, 405-410
- **PLESSIS et al.** *Antiviral Research,* vol. 18, 259-265

- **HU et al.** *S.T.P.Pharma. Sci.,* 1994, vol. 4 (6), 466
- **ALLEN et al.** *FEBS Letters,* 1987, vol. 223, 42
- **WU et al.** *Cancer Research,* 1993, vol. 53, 3765
- **PAPAHADJOPOULOS et al.** *Ann. N.Y. Acad. Sci.,* 1987, vol. 507, 64
- **GABIZON.** *Proc. Nail . Acad. Sci. U.S.A.,* 1988, vol. 85, 6949
- **SUNAMOTO et al.** *Bull. Chem. Soc. Jpn.,* 1980, vol. 511, 2778
- **ILLUM et al.** *FEBS Lett.,* 1984, vol. 167, 79
- **KLIBANOV et al.** *FEBS Lett.,* 1990, vol. 268, 235
- **BLUME et al.** *Biochimica et Biophysica Acta,* 1990, vol. 1029, 91
- **RIEGER.** Pharmaceutical Dosage Forms. Marcel Dekker, Inc, 1988, 285
- **RIEGER.** Pharmaceutical Dosage Forms. Marcel Dekker, inc, 1988, 285
- **MIYAO et al.** *DsRNA Res. Dev.,* 1995, vol. 5, 115-121
- **TAKAKURA et al.** *DsRNA & Nucl. Acid Drug Dev.,* 1996, vol. 6, 177-183
- The Merck Manual of Diagnosis and Therapy. 1987, 1206-1228
- The Merck Manual of Diagnosis and Therapy. 1987, 2499-250646-49
- **LIU ; ATWOOD.** Propagation and assay of the JC Virus. *Methods Mol Biol.,* 2001, vol. 165, 9-17
- **COUTURE, A et al.** *TIG.,* 1996, vol. 12, 5-10
- **GASSMANN et al.** *Proc. Natl. Acad. Sci. USA,* 1995, vol. 92, 1292
- **MUZYCZKA et al.** *Curr. Topics Micro. Immunol.,* 1992, vol. 158, 97-129
- **BERKNER et al.** *BioTechniques,* 1998, vol. 6, 616
- **ROSENFELD et al.** *Science,* 1991, vol. 252, 431-434
- **ROSENFELD et al.** *Cell,* 1992, vol. 68, 143-155
- **EGLITIS et al.** *Science,* 1985, vol. 230, 1395-1398
- **DANOS ; MULLIGAN.** *Proc. Natl. Acad. Sci. USA,* 1998
- *Science,* 1985, vol. 230, 1395-1398
- **DANOS ; MULLIGAN.** *Proc. Natl. Acad. Sci. USA,* 1998, vol. 85, 6460-6464
- **WILSON et al.** *Proc. Natl. Acad. Sci. USA,* 1988, vol. 85, 3014-3018
- **ARMENTANO et al.** *Proc. Natl. Acad. Sci. USA,* 1990, vol. 87, 61416145
- **HUBER et al.** *Proc. Natl. Acad. Sci. USA,* 1991, vol. 88, 8039-8043
- **FERRY et al.** *Proc. Natl. Acad. Sci. USA,* 1991, vol. 88, 8377-8381
- **CHOWDHURY et al.** *Science,* 1991, vol. 254, 1802-1805
- **BEUSECHEM. et al.** *Proc. Nad. Acad. Sci. USA,* 1992, vol. 89, 7640-19
- **KAY et al.** *Human Gene Therapy,* 1992, vol. 3, 641-647
- **DAI et al.** *Proc. Natl.Acad. Sci. USA,* 1992, vol. 89, 10892-10895
- **HWU et al.** *J. Immunol.,* 1993, vol. 150, 4104-4115
- **CORNETTE et al.** *Human Gene Therapy,* 1991, vol. 2, 5-10
- **CONC.** *Proc. Natl. Acad. Sci. USA,* 1984, vol. 81, 6349
- **HSU et al.** *J. Infectious Disease,* 1992, vol. 166, 769
- **BUCCHINI et al.** *Proc. Natl. Acad. Sci. USA,* 1986, vol. 83, 2511-2515
- **DOCHERTY et al.** *FASEB J.,* 1994, vol. 8, 20-24
- **CHEN et al.** *Proc. Natl. Acad. Sci. USA,* 1994, vol. 91, 3054-3057

SEQUENCE LISTING

<110> ALNYLAM PHARMACEUTICALS INC.

<120> COMPOSITIONS AND METHODS FOR INHIBITING EXPRESSION OF A GENE FROM THE JC VIRUS

<130> P3525 EP

<140> EP 07 76 1452.7
<141> 2007-04-27

<150> 60/795,765
<151> 2006-04-28

<160> 934

<170> PatentIn version 3.5

<210> 1
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 1
acuuuuaggg uuguacgggt t 21

<210> 2
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 2
cccguacaac ccuaaaagut t 21

<210> 3
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 3
acuuuuaggg uuguacgggt t 21

<210> 4
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 4
cccguacaac ccuaaaagut t 21

<210> 5
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 5
cuuuuagggu uguacgggat t 21

<210> 6
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 6
ucccguacaa cccuaaaagt t 21

<210> 7
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 7
cuuuuagggu uguacgggat t 21

<210> 8
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 8
ucccguacaa cccuaaaagt t 21

<210> 9
<211> 21
<212> DNA
<213> Artificial sequence

<220>
<223> Description of combined DNA/RNA Molecule: Synthetic oligonucleotide

<220>
<223> Description of Artificial Sequence: Synthetic oligonucleotide

<400> 9
cagagcacaa ggcguaccut t 21

<210> 10
<211> 21
<212> DNA
<213> Artificial Sequence

<220>
<223> Description of combined DNA/RNA Molecule: Synthetic oligonucleotide

<220>
<223> Description of Artificial sequence: synthetic oligonucleotide

<400> 10
agguacgccu ugugcucugt t 21

<210> 11
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 11
cagagcacaa ggcguaccut t 21

<210> 12
<211> 21

<212> DNA
<213> Artificial Sequence

<220>
<223> Description of combined DNA/RNA Molecule: Synthetic oligonucleotide

<220>
<223> Description of Artificial sequence: Synthetic oligonucleotide

<400> 12
agguacgccu ugugcucugt t 21

<210> 13
<211> 21
<212> DNA
<213> Artificial sequence

<220>
<223> Description of combined DNA/RNA Molecule: Synthetic oligonucleotide

<220>
<223> Description of Artificial Sequence: Synthetic oligonucleotide

<400> 13
cagagcacaa ggcguaccut t 21

<210> 14
<211> 21
<212> DNA
<213> Artificial sequence

<220>
<223> Description of combined DNA/RNA Molecule: Synthetic oligonucleotide

<220>
<223> Description of Artificial Sequence: Synthetic oligonucleotide

<400> 14
agguacgccu ugugcucugt t 21

<210> 15
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 15
cagagcacaa ggcguaccut t 21

<210> 16
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 16
agguacgccu ugugcucugt t 21

<210> 17
<211> 21
<212> DNA
<213> Artificial Sequence

<220>
<223> Description of combined DNA/RNA Molecule: Synthetic oligonucleotide

<220>
<223> Description of Artificial Sequence: synthetic oligonucleotide

<400> 17
uaggguugua cgggacugut t 21

<210> 18
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 18
acagucccgu acaacccuat t 21

<210> 19
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 19
uaggguugua cgggacugut t 21

<210> 20
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 20
acagucccgu acaacccuat t 21

<210> 21
<211> 21
<212> DNA
<213> Artificial sequence

<220>
<223> Description of combined DNA/RNA Molecule: Synthetic oligonucleotide

<220>
<223> Description of Artificial Sequence: Synthetic oligonucleotide

<400> 21
aggguuguac gggacuguat t 21

<210> 22
<211> 21
<212> DNA
<213> Artificial sequence

<220>
<223> Description of combined DNA/RNA Molecule: Synthetic oligonucleotide

<220>
<223> Description of Artificial sequence: Synthetic oligonucleotide

<400> 22
uacagucccg uacaacccut t 21

<210> 23
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 23
aggguuguac gggacuguat t 21

<210> 24
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 24
uacagucccg uacaacccut t 21

<210> 25
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 25
aggguuguac gggacuguat t 21

<210> 26
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 26
uacagucccg uacaacccut t 21

<210> 27
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 27
aggguuguac gggacuguat t 21

<210> 28
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 28
uacagucccg uacaacccut t 21

<210> 29
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 29
uuguacggga cuguaacact t 21

<210> 30
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 30
guguuacagu cccguacaat t 21

<210> 31
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 31
uuguacggga cuguaacact t 21

<210> 32
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 32
guguuacagu cccguacaat t 21

<210> 33

<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 33
gccugauuuu gguacauggt t 21

<210> 34
<211> 21
<212> DNA
<213> Artificial Sequence

<220>
<223> Description of Combined DNA/RNA Molecule: Synthetic oligonucleotide

<220>
<223> Description of Artificial Sequence: synthetic oligonucleotide

<400> 34
ccauguacca aaaucaggct t 21

<210> 35
<211> 21
<212> DNA
<213> Artificial Sequence

<220>
<223> Description of Combined DNA/RNA Molecule: Synthetic oligonucleotide

<220>
<223> Description of Artificial Sequence: synthetic oligonucleotide

<400> 35
gaaguaguaa gggcguggat t 21

<210> 36
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 36
uccacgcccu uacuacuuct t 21

<210> 37
<211> 21
<212> DNA

<213> Artificial Sequence

<220>
<223> Description of combined DNA/RNA Molecule: Synthetic oligonucleotide

<220>
<223> Description of Artificial Sequence: Synthetic oligonucleotide

<400> 37
gaaguaguaa gggcguggat t 21

<210> 38
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 38
uccacgcccu uacuacuuct t 21

<210> 39
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 39
gaaguaguaa gggcguggat t 21

<210> 40
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 40
uccacgcccu uacuacuuct t 21

<210> 41
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 41
gaaguaguaa gggcguggat t 21

<210> 42
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 42
uccacgcccu uacuacuuct t 21

<210> 43
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 43
auaggccuua cuccugaaat t 21

<210> 44
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 44
uuucaggagu aaggccuaut t 21

<210> 45
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 45
auaggccuua cuccugaaat t 21

<210> 46
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 46
uuucaggagu aaggccuaut t 21

<210> 47
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 47
gacagccaua ugcaguagut t 21

<210> 48 ...
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 48
acuacugcau auggcuguct t 21

<210> 49
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 49
gacagccaua ugcaguagut t 21

<210> 50
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 50
acuacugcau auggcuguct t 21

<210> 51
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 51
gacagccaua ugcaguagut t 21

<210> 52
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 52
acuacugcau auggcuguct t 21

<210> 53
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 53
gacagccaua ugcaguagut t 21

<210> 54
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 54
acuacugcau auggcuguct t 21

<210> 55
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 55
aaacuacuug ggcaauagut t 21

<210> 56
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 56
acuauugccc aaguaguuut t 21

<210> 57
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 57
aaacuacuug ggcaauagut t 21

<210> 58
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 58
acuauugccc aaguaguuut t 21

<210> 59
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 59
aaacuacuug ggcaauagut t 21

<210> 60
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 60
acuauugccc aaguaguuut t 21

<210> 61
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 61
aaacuacuug ggcaauagut t 21

<210> 62
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 62
acuauugccc aaguaguuut t 21

<210> 63
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 63
ucagguucau gggugccgct t 21

<210> 64
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 64
gcggcaccca ugaaccugat t 21

<210> 65
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 65
ucagguucau gggugccgct t 21

<210> 66
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 66
gcggcaccca ugaaccugat t 21

<210> 67
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 67
guaguaaggg cguggaggct t 21

<210> 68
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 68
gccuccacgc ccuuacuact t 21

<210> 69
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 69
guaguaaggg cguggaggct t 21

<210> 70
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 70
gccuccacgc ccuuacuact t 21

<210> 71
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 71
uauugcaagg aauggccuat t 21

<210> 72
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 72
uaggccauuc cuugcaauat t 21

<210> 73
<211> 21
<212> DNA
<213> Artificial Sequence

<220>
<223> Description of combined DNA/RNA Molecule: Synthetic oligonucleotide

<220>
<223> Description of Artificial sequence: Synthetic oligonucleotide

<400> 73
uauugcaagg aauggccuat t 21

<210> 74
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 74
uaggccauuc cuugcaauat t 21

<210> 75
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 75
uauugcaagg aauggccuat t 21

<210> 76
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 76
uaggccauuc cuugcaauat t 21

<210> 77
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 77
uauugcaagg aauggccuat t 21

<210> 78
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 78
uaggccauuc cuugcaauat t 21

<210> 79
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 79
aguaguaagg gcguggaggt t 21

<210> 80
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 80
ccuccacgcc cuuacuacut t 21

<210> 81
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 81
aguaguaagg gcguggaggt t 21

<210> 82
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 82
ccuccacgcc cuuacuacut t 21

<210> 83
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 83
aguaguaagg gcguggaggt t 21

<210> 84
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 84
ccuccacgcc cuuacuacut t 21

<210> 85
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 85
aguaguaagg gcguggaggt t 21

<210> 86
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 86
ccuccacgcc cuuacuacut t 21

<210> 87
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 87
ugcuauugcu uugauugcut t 21

<210> 88
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 88
agcaaucaaa gcaauagcat t 21

<210> 89
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 89
ugcuauugcu uugauugcut t 21

<210> 90
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 90
agcaaucaaa gcaauagcat t 21

<210> 91
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 91
ugcuauugcu uugauugcut t 21

<210> 92
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 92
agcaaucaaa gcaauagcat t 21

<210> 93
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 93
ugcuauugcu uugauugcut t 21

<210> 94
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 94
agcaaucaaa gcaauagcat t 21

<210> 95
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 95
gcuauugcuu ugauugcuut t 21

<210> 96
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 96
aagcaaucaa agcaauagct t 21

<210> 97
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 97
gcuauugcuu ugauugcuut t 21

<210> 98
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 98
aagcaaucaa agcaauagct t 21

<210> 99
<211> 21
<212> μDNA
<213> Artificial Sequence

<220>
<223> Description of Combined DNA/RNA Molecule: Synthetic oligonucleotide

<220>
<223> Description of Artificial Sequence: Synthetic oligonucleotide

<400> 99
ccuuuacuuu uaggguugut t 21

<210> 100
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 100
acaacccuaa aaguaaaggt t 21

<210> 101
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 101
uuacuuuuag gguuguacgt t 21

<210> 102
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 102
cguacaaccc uaaaaguaat t 21

<210> 103
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 103
uuacuuuuag gguuguacgt t 21

<210> 104
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 104
cguacaaccc uaaaaguaat t 21

<210> 105
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 105
gcuccucaau ggauguugct t 21

<210> 106
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 106
gcaacaucca uugaggagct t 21

<210> 107
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 107
uuauaagagg aggaguagat t 21

<210> 108
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 108
ucuacuccuc cucuuauaat t 21

<210> 109
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 109
uuauaagagg aggaguagat t 21

<210> 110
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 110
ucuacuccuc cucuuauaat t 21

<210> 111
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 111
aaguaguaag ggcguggagt t 21

<210> 112
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 112
cuccacgccc uuacuacuut t 21

<210> 113
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 113
aaguaguaag ggcguggagt t 21

<210> 114
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 114
cuccacgccc uuacuacuut t 21

<210> 115
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 115
aaguaguaag ggcguggagt t 21

<210> 116
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 116
cuccacgccc uuacuacuut t 21

<210> 117
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 117
aaguaguaag ggcguggagt t 21

<210> 118
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 118
cuccacgccc uuacuacuut t 21

<210> 119
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 119
uuaggguugu acgggacugt t 21

<210> 120
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 120
cagucccgua caacccuaat t 21

<210> 121
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 121
uuaggguugu acgggacugt t 21

<210> 122
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 122
cagucccgua caacccuaat t 21

<210> 123
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 123
gacaugcuuc cuuguuacat t 21

<210> 124
<211> 21
<212> DNA
<213> Artificial Sequence

<220>
<223> Description of combined DNA/RNA Molecule: Synthetic oligonucleotide

<220>
<223> Description of Artificial Sequence: synthetic oligonucleotide

<400> 124
uguaacaagg aagcauguct t 21

<210> 125
<211> 21
<212> DNA
<213> Artificial Sequence

<220>
<223> Description of combined DNA/RNA Molecule: Synthetic oligonucleotide

<220>
<223> Description of Artificial Sequence: synthetic oligonucleotide

<400> 125
gacaugcuuc cuuguuacat t 21

<210> 126
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 126
uguaacaagg aagcauguct t 21

<210> 127
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 127
gacaugcuuc cuuguuacat t 21

<210> 128
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 128
uguaacaagg aagcauguct t 21

<210> 129
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 129
gacaugcuuc cuuguuacat t 21

<210> 130
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 130
uguaacaagg aagcauguct t 21

<210> 131
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 131
uguugaaugu uggguuccut t 21

<210> 132
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 132
aggaacccaa cauucaacat t 21

<210> 133
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 133
uguugaaugu uggguuccut t 21

<210> 134
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 134
aggaacccaa cauucaacat t 21

<210> 135
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 135
uguugaaugu uggguuccut t 21

<210> 136
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 136
aggaacccaa cauucaacat t 21

<210> 137
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 137
uguugaaugu uggguuccut t 21

<210> 138
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 138
aggaacccaa cauucaacat t 21

<210> 139
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 139
acuuaaccca agaagcucut t 21

<210> 140
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 140
agagcuucuu ggguuaagut t 21

<210> 141
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 141
acuuaaccca agaagcucut t 21

<210> 142
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 142
agagcuucuu ggguuaagut t 21

<210> 143
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 143
ucagccugau uuugguacat t 21

<210> 144
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 144
uguaccaaaa ucaggcugat t 21

<210> 145
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 145
ucagccugau uuugguacat t 21

<210> 146
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 146
uguaccaaaa ucaggcugat t 21

<210> 147
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 147
uuuuaggguu guacgggact t 21

<210> 148
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 148
gucccguaca acccuaaaat t 21

<210> 149
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 149
uuuuaggguu guacgggact t 21

<210> 150
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 150
gucccguaca acccuaaaat t 21

<210> 151
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 151
uuuaggguug uacgggacut t 21

<210> 152
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 152
agucccguac aacccuaaat t 21

<210> 153
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 153
uuuaggguug uacgggacut t 21

<210> 154
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 154
agucccguac aacccuaaat t 21

<210> 155
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 155
ucccuugcua cuguagaggt t 21

<210> 156
<211> 21
<212> DNA
<213> Artificial sequence

<220>
<223> Description of Combined DNA/RNA Molecule: Synthetic oligonucleotide

<220>
<223> Description of Artificial Sequence: Synthetic oligonucleotide

<400> 156
ccucuacagu agcaagggat t 21

<210> 157
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 157
ucccuugcua cuguagaggt t 21

<210> 158
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 158
ccucuacagu agcaagggat t 21

<210> 159
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 159
cccuugcuac uguagagggt t 21

<210> 160
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 160
cccucuacag uagcaagggt t 21

<210> 161
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 161
cccuugcuac uguagagggt t 21

<210> 162
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 162
cccucuacag uagcaagggt t 21

<210> 163
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 163
ugcuccucaa uggauguugt t 21

<210> 164
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 164
caacauccau ugaggagcat t 21

<210> 165
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 165
ugcuccucaa uggauguugt t 21

<210> 166
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 166
caacauccau ugaggagcat t 21

<210> 167
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 167
ugcuccucaa uggauguugt t 21

<210> 168
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 168
caacauccau ugaggagcat t 21

<210> 169
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 169
ugcuccucaa uggauguugt t 21

<210> 170
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 170
caacauccau ugaggagcat t 21

<210> 171
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 171
gaucugcucc ucaauggaut t 21

<210> 172
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 172
auccauugag gagcagauct t 21

<210> 173
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 173
gaucugcucc ucaauggaut t 21

<210> 174
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 174
auccauugag gagcagauct t 21

<210> 175
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 175
gaucugcucc ucaauggaut t 21

<210> 176
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 176
auccauugag gagcagauct t 21

<210> 177
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 177
gaucugcucc ucaauggaut t 21

<210> 178
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 178
auccauugag gagcagauct t 21

<210> 179
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 179
aucugcuccu caauggaugt t 21

<210> 180
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 180
cauccauuga ggagcagaut t 21

<210> 181
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 181
aucugcuccu caauggaugt t 21

<210> 182
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 182
cauccauuga ggagcagaut t 21

<210> 183
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 183
ucugcuccuc aauggaugut t 21

<210> 184
<211> 21

<212> ’DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 184
acauccauug aggagcagat t 21

<210> 185
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 185
cugcuccuca auggauguut t 21

<210> 186
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 186
aacauccauu gaggagcagt t 21

<210> 187
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 187
cugcuccuca auggauguut t 21

<210> 188
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 188
aacauccauu gaggagcagt t 21

<210> 189
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 189
guuguacggg acuguaacat t 21

<210> 190
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 190
uguuacaguc ccguacaact t 21

<210> 191
<211> 21
<212> DNA
<213> Artificial Sequence

<220>
<223> Description of Combined DNA/RNA Molecule: Synthetic oligonucleotide

<220>
<223> Description of Artificial Sequence: Synthetic oilgonucleotide

<400> 191
guuguacggg acuguaacat t 21

<210> 192
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 192
uguuacaguc ccguacaact t 21

<210> 193
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 193
uguacgggac uguaacacct t 21

<210> 194
<211> 21
<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 194
gguguuacag ucccguacat t 21

<210> 195
<211> 21
<212> DNA
<213> Artificial sequence

<220>
<223> Description of combined DNA/RNA Molecule: Synthetic oligonucleotide

<220>
<223> Description of Artificial Sequence: Synthetic oligonucleotide

<400> 195
uguacgggac uguaacacct t 21

<210> 196
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 196
gguguuacag ucccguacat t 21

<210> 197
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 197
uguacgggac uguaacacct t 21

<210> 198
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 198
gguguuacag ucccguacat t 21

<210> 199
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 199
uguacgggac uguaacacct t 21

<210> 200
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 200
gguguuacag ucccguacat t 21

<210> 201
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 201
guacgggacu guaacaccut t 21

<210> 202
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 202
agguguuaca gucccguact t 21

<210> 203
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 203
guacgggacu guaacaccut t 21

<210> 204
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 204
agguguuaca gucccguact t 21

<210> 205
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 205
cagccugauu uugguacaut t 21

<210> 206
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 206
auguaccaaa aucaggcugt t 21

<210> 207
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 207
cagccugauu uugguacaut t 21

<210> 208
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 208
auguaccaaa aucaggcugt t 21

<210> 209
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 209
cagccugauu uugguacaut t 21

<210> 210
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 210
auguaccaaa aucaggcugt t 21

<210> 211
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 211
cagccugauu uugguacaut t 21

<210> 212
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 212
auguaccaaa aucaggcugt t 21

<210> 213
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 213
gaauagggag gaauccaugt t 21

<210> 214
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 214
cauggauucc ucccuauuct t 21

<210> 215
<211> 21
<212> DNA
<213> Artificial sequence

<220>
<223> Description of Combined DNA/RNA Molecule: Synthetic oligonucleotide

<220>
<223> Description of Artificial Sequence: Synthetic oligonucleotide

<400> 215
gaauagggag gaauccaugt t 21

<210> 216
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 216
cauggauucc ucccuauuct t 21

<210> 217
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 217
gaauagggag gaauccaugt t 21

<210> 218
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 218
cauggauucc ucccuauuct t 21

<210> 219
<211> 21
<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 219
gaauagggag gaauccaugt t 21

<210> 220
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 220
cauggauucc ucccuauuct t 21

<210> 221
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 221
aagugcugaa uagggaggat t 21

<210> 222
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 222
uccucccuau ucagcacuut t 21

<210> 223
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 223
aagugcugaa uagggaggat t 21

<210> 224
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 224
uccucccuau ucagcacuut t 21

<210> 225
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 225
aagugcugaa uagggaggat t 21

<210> 226
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 226
uccucccuau ucagcacuut t 21

<210> 227
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 227
aagugcugaa uagggaggat t 21

<210> 228
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 228
uccucccuau ucagcacuut t 21

<210> 229
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 229
aggcugcugc uacuauagat t 21

<210> 230
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 230
ucuauaguag cagcagccut t 21

<210> 231
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 231
aggcugcugc uacuauagat t 21

<210> 232
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 232
ucuauaguag cagcagccut t 21

<210> 233
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 233
aggcugcugc uacuauagat t 21

<210> 234
<211> 21
<212> DNA
<213> Artificial Sequence

<220> .
<223> Description of Combined DNA/RNA Molecule: Synthetic oligonucleotide

<220>
<223> Description of Artificial Sequence: Synthetic oligonucleotide

<400> 234
ucuauaguag cagcagccut t 21

<210> 235
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 235
aggcugcugc uacuauagat t 21

<210> 236
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 236
ucuauaguag cagcagccut t 21

<210> 237
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 237
agacagccau augcaguagt t 21

<210> 238
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 238
cuacugcaua uggcugucut t 21

<210> 239
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 239
agacagccau augcaguagt t 21

<210> 240
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 240
cuacugcaua uggcugucut t 21

<210> 241
<211> 21
<212> DNA
<213> Artificial Sequence

<220>
<223> Description of Combined DNA/RNA Molecule: Synthetic oligonucleotide

<220>
<223> Description of Artificial Sequence: synthetic oligonucleotide

<400> 241
uucagguuca ugggugccgt t 21

<210> 242
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 242
cggcacccau gaaccugaat t 21

<210> 243
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 243
uucagguuca ugggugccgt t 21

<210> 244
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 244
cggcacccau gaaccugaat t 21

<210> 245
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 245
uagacagcca uaugcaguat t 21

<210> 246
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 246
uacugcauau ggcugucuat t 21

<210> 247
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 247
uagacagcca uaugcaguat t 21

<210> 248
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 248
uacugcauau ggcugucuat t 21

<210> 249
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 249
uagacagcca uaugcaguat t 21

<210> 250
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 250
uacugcauau ggcugucuat t 21

<210> 251
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 251
uagacagcca uaugcaguat t 21

<210> 252
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 252
uacugcauau ggcugucuat t 21

<210> 253
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 253
ggagcaugac uuuaacccat t 21

<210> 254
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 254
uggguuaaag ucaugcucct t 21

<210> 255
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 255
ggagcaugac uuuaacccat t 21

<210> 256
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 256
uggguuaaag ucaugcucct t 21

<210> 257
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 257
ggagcaugac uuuaacccat t 21

<210> 258
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 258
uggguuaaag ucaugcucct t 21

<210> 259
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 259
ggagcaugac uuuaacccat t 21

<210> 260
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 260
uggguuaaag ucaugcucct t 21

<210> 261
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 261
guugccuuua cuuuuagggt t 21

<210> 262
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 262
cccuaaaagu aaaggcaact t 21

<210> 263
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 263
guugccuuua cuuuuagggt t 21

<210> 264
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 264
cccuaaaagu aaaggcaact t 21

<210> 265
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 265
ugugacuuaa cccaagaagt t 21

<210> 266
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 266
cuucuugggu uaagucacat t 21

<210> 267
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 267
ugugacuuaa cccaagaagt t 21

<210> 268
<211> 21

<212> DNA
<213> Artificial Sequence

<220>
<223> Description of combined DNA/RNA Molecule: Synthetic oligonucleotide

<220>
<223> Description of Artificial sequence: Synthetic oligonucleotide

<400> 268
cuucuugggu uaagucacat t 21

<210> 269
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 269
ugugacuuaa cccaagaagt t 21

<210> 270
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 270
cuucuugggu uaagucacat t 21

<210> 271
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 271
ugugacuuaa cccaagaagt t 21

<210> 272
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 272
cuucuugggu uaagucacat t 21

<210> 273
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 273
uauugcuuug auugcuucat t 21

<210> 274
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 274
ugaagcaauc aaagcaauat t 21

<210> 275
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 275
uauugcuuug auugcuucat t 21

<210> 276
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 276
ugaagcaauc aaagcaauat t 21

<210> 277
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 277
uauugcuuug auugcuucat t 21

<210> 278
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 278
ugaagcaauc aaagcaauat t 21

<210> 279
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 279
uauugcuuug auugcuucat t 21

<210> 280
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 280
ugaagcaauc aaagcaauat t 21

<210> 281
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 281
auauccuguu gaauguuggt t 21

<210> 282
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 282
ccaacauuca acaggauaut t 21

<210> 283
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 283
uuuauugcaa ggaauggcct t 21

<210> 284
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 284
ggccauuccu ugcaauaaat t 21

<210> 285
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 285
uuuauugcaa ggaauggcct t 21

<210> 286
<211> 21
<212> DNA
<213> Artificial sequence

<220>
<223> Description of Combined DNA/RNA Molecule: Synthetic oligonucleotide

<220>
<223> Description of Artificial Sequence: Synthetic oligonucleotide

<400> 286
ggccauuccu ugcaauaaat t 21

<210> 287
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 287
uggaagaaac uacuugggct t 21

<210> 288
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 288
gcccaaguag uuucuuccat t 21

<210> 289
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 289
uggaagaaac uacuugggct t 21

<210> 290
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 290
gcccaaguag uuucuuccat t 21

<210> 291
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 291
uggaagaaac uacuugggct t 21

<210> 292
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 292
gcccaaguag uuucuuccat t 21

<210> 293
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 293
uggaagaaac uacuugggct t 21

<210> 294
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 294
gcccaaguag uuucuuccat t 21

<210> 295
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 295
aagacccuaa agacuuucct t 21

<210> 296
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 296
ggaaagucuu uagggucuut t 21

<210> 297
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 297
aagacccuaa agacuuucct t 21

<210> 298
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 298
ggaaagucuu uagggucuut t 21

<210> 299
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 299
aagacccuaa agacuuucct t 21

<210> 300
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 300
ggaaagucuu uagggucuut t 21

<210> 301
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 301
aagacccuaa agacuuucct t 21

<210> 302
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 302
ggaaagucuu uagggucuut t 21

<210> 303
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 303
uggauguugc cuuuacuuut t 21

<210> 304
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 304
aaaguaaagg caacauccat t 21

<210> 305
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 305
uggauguugc cuuuacuuut t 21

<210> 306
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 306
aaaguaaagg caacauccat t 21

<210> 307
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 307
uggauguugc cuuuacuuut t 21

<210> 308
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 308
aaaguaaagg caacauccat t 21

<210> 309
<211> 21
<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 309
uggauguugc cuuuacuuut t 21

<210> 310
<211> 21

<212> DNA
<213> Artificial Sequence

<220>
<223> Description of Combined DNA/RNA Molecule: Synthetic oligonucleotide

<220>
<223> Description of Artificial Sequence: synthetic oligonucleotide

<400> 310
aaaguaaagg caacauccat t 21

<210> 311
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 311
uuugauugcu ucagacaaut t 21

<210> 312
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 312
auugucugaa gcaaucaaat t 21

<210> 313
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 313
uuugauugcu ucagacaaut t 21

<210> 314
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 314
auugucugaa gcaaucaaat t 21

<210> 315
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 315
aauagggagg aauccauggt t 21

<210> 316
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 316
ccauggauuc cucccuauut t 21

<210> 317
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 317
aauagggagg aauccauggt t 21

<210> 318
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 318
ccauggauuc cucccuauut t 21

<210> 319
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 319
ggacaaagug cugaauaggt t 21

<210> 320
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 320
ccuauucagc acuuugucct t 21

<210> 321
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 321
ggacaaagug cugaauaggt t 21

<210> 322
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 322
ccuauucagc acuuugucct t 21

<210> 323
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 323
ggacaaagug cugaauaggt t 21

<210> 324
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 324
ccuauucagc acuuugucct t 21

<210> 325
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 325
ggacaaagug cugaauaggt t 21

<210> 326
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 326
ccuauucagc acuuugucct t 21

<210> 327
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 327
uggacaaagu gcugaauagt t 21

<210> 328
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 328
cuauucagca cuuuguccat t 21

<210> 329
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 329
uggacaaagu gcugaauagt t 21

<210> 330
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 330
cuauucagca cuuuguccat t 21

<210> 331
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 331
aaauugcauc ccuugcuact t 21

<210> 332
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 332
guagcaaggg augcaauuut t 21

<210> 333
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 333
aaauugcauc ccuugcuact t 21

<210> 334
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 334
guagcaaggg augcaauuut t 21

<210> 335
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 335
gcaucccuug cuacuguagt t 21

<210> 336
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 336
cuacaguagc aagggaugct t 21

<210> 337
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 337
aaaaaaaggu agaagaccct t 21

<210> 338
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 338
gggucuucua ccuuuuuuut t 21

<210> 339
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 339
aaaaaaaggu agaagaccct t 21

<210> 340
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 340
gggucuucua ccuuuuuuut t 21

<210> 341
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 341
aaaaaaaggu agaagaccct t 21

<210> 342
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 342
gggucuucua ccuuuuuuut t 21

<210> 343
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 343
aaaaaaaggu agaagaccct t 21

<210> 344
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 344
gggucuucua ccuuuuuuut t 21

<210> 345
<211> 21
<212> DNA
<213> Artificial Sequence

<220> .
<223> Description of combined DNA/RNA Molecule: Synthetic oligonucleotide

<220>
<223> Description of Artificial Sequence: Synthetic oligonucleotide

<400> 345
acagagcaca aggcguacct t 21

<210> 346
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 346
gguacgccuu gugcucugut t 21

<210> 347
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 347
acagagcaca aggcguacct t 21

<210> 348
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 348
gguacgccuu gugcucugut t 21

<210> 349
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 349.
ugauuuuggu acauggaaut t 21

<210> 350
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 350
auuccaugua ccaaaaucat t 21

<210> 351
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 351
ugauuuuggu acauggaaut t 21

<210> 352
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 352
auuccaugua ccaaaaucat t 21

<210> 353
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 353
ugauuuuggu acauggaaut t 21

<210> 354
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 354
auuccaugua ccaaaaucat t 21

<210> 355
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 355
ugauuuuggu acauggaaut t 21

<210> 356
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 356
auuccaugua ccaaaaucat t 21

<210> 357
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 357
ggguuguacg ggacuguaat t 21

<210> 358
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 358
uuacaguccc guacaaccct t 21

<210> 359
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 359
ggguuguacg ggacuguaat t 21

<210> 360
<211> 21
<212> DNA
<213> Artificial sequence

<220>
<223> Description of Combined DNA/RNA Molecule: Synthetic oligonucleotide

<220>
<223> Description of Artificial Sequence: Synthetic oligonucleotide

<400> 360
uuacaguccc guacaaccct t 21

<210> 361
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 361
gguuguacgg gacuguaact t 21

<210> 362
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 362
guuacagucc cguacaacct t 21

<210> 363
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 363
gguuguacgg gacuguaact t 21

<210> 364
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 364
guuacagucc cguacaacct t 21

<210> 365
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 365
gguuguacgg gacuguaact t 21

<210> 366
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 366
guuacagucc cguacaacct t 21

<210> 367
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 367
gguuguacgg gacuguaact t 21

<210> 368
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 368
guuacagucc cguacaacct t 21

<210> 369
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 369
acgggacugu aacaccugct t 21

<210> 370
<211> 21
<212> DNA
<213> Artificial Sequence

<220>
<223> Description of combined DNA/RNA Molecule: Synthetic oligonucleotide

<220>
<223> Description of Artificial sequence: synthetic oligonucleotide

<400> 370
gcagguguua cagucccgut t 21

<210> 371
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 371
gggacuguaa caccugcuct t 21

<210> 372
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 372
gagcaggugu uacaguccct t 21

<210> 373
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 373
gggacuguaa caccugcuct t 21

<210> 374
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 374
gagcaggugu uacaguccct t 21

<210> 375
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 375
gggacuguaa caccugcuct t 21

<210> 376
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 376
gagcaggugu uacaguccct t 21

<210> 377
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 377
gggacuguaa caccugcuct t 21

<210> 378
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 378
gagcaggugu uacaguccct t 21

<210> 379
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 379
acuccagaaa ugggugacct t 21

<210> 380
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 380
ggucacccau uucuggagut t 21

<210> 381
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 381
acuccagaaa ugggugacct t 21

<210> 382
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 382
ggucacccau uucuggagut t 21

<210> 383
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 383
ccugauuuug guacauggat t 21

<210> 384
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 384
uccauguacc aaaaucaggt t 21

<210> 385
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 385
ccugauuuug guacauggat t 21

<210> 386
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 386
uccauguacc aaaaucaggt t 21

<210> 387
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 387
caaagugcug aauagggagt t 21

<210> 388
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 388
cucccuauuc agcacuuugt t 21

<210> 389
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 389
caaagugcug aauagggagt t 21

<210> 390
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 390
cucccuauuc agcacuuugt t 21

<210> 391
<211> 21
<212> DNA
<213> Artificial sequence

<220>
<223> Description of Combined DNA/RNA Molecule: synthetic oligonucleotide

<220>
<223> Description of Artificial Sequence: Synthetic oligonucleotide

<400> 391
caaagugcug aauagggagt t 21

<210> 392
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 392
cucccuauuc agcacuuugt t 21

<210> 393
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 393
caaagugcug aauagggagt t 21

<210> 394
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 394
cucccuauuc agcacuuugt t 21

<210> 395
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 395
ccagggaaau ucccuuguut t 21

<210> 396
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 396
aacaagggaa uuucccuggt t 21

<210> 397
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 397
ccagggaaau ucccuuguut t 21

<210> 398
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 398
aacaagggaa uuucccuggt t 21

<210> 399
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 399
aggccaggga aauucccuut t 21

<210> 400
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 400
aagggaauuu cccuggccut t 21

<210> 401
<211> 21
<212> DNA
<213> Artificial Sequence

<220>
<223> Description of Combined DNA/RNA molecule: Synthetic oligonucleotide

<220>
<223> Description of Artificial Sequence: Synthetic oligonucleotide

<400> 401
aggccaggga aauucccuut t 21

<210> 402
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 402
aagggaauuu cccuggccut t 21

<210> 403
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 403
aggccaggga aauucccuut t 21

<210> 404
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 404
aagggaauuu cccuggccut t 21

<210> 405
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 405
aggccaggga aauucccuut t 21

<210> 406
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 406
aagggaauuu cccuggccut t 21

<210> 407
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 407
uaguugcuac uguuucugat t 21

<210> 408
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 408
ucagaaacag uagcaacuat t 21

<210> 409
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 409
uaguugcuac uguuucugat t 21

<210> 410
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 410
ucagaaacag uagcaacuat t 21

<210> 411
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 411
cugcugcuac uauagaagut t 21

<210> 412
<211> 21
<212> DNA
<213> Artificial sequence

<220>
<223> Description of Combined DNA/RNA Molecule: Synthetic oligonucleotide

<220>
<223> Description of Artificial Sequence: Synthetic oligonucleotide

<400> 412
acuucuauag uagcagcagt t 21

<210> 413
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 413
cugcugcuac uauagaagut t 21

<210> 414
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 414
acuucuauag uagcagcagt t 21

<210> 415
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 415
ugcugcuacu auagaaguut t 21

<210> 416
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 416
aacuucuaua guagcagcat t 21

<210> 417
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 417
ugcugcuacu auagaaguut t 21

<210> 418
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 418
aacuucuaua guagcagcat t 21

<210> 419
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 419
ugcugcuacu auagaaguut t 21

<210> 420
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 420
aacuucuaua guagcagcat t 21

<210> 421
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 421
ugcugcuacu auagaaguut t 21

<210> 422
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 422
aacuucuaua guagcagcat t 21

<210> 423
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 423
gcugcuacua uagaaguugt t 21

<210> 424
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 424
caacuucuau aguagcagct t 21

<210> 425
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 425
gcugcuacua uagaaguugt t 21

<210> 426
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 426
caacuucuau aguagcagct t 21

<210> 427
<211> 21
<212> DNA
<213> Artificial Sequence

<220>
<223> Description of Combined DNA/RNA Molecule: Synthetic oligonucleotide

<220>

<223> Description of Artificial Sequence: Synthetic oligonucleotide

<400> 427
cugcuacuau agaaguugat t 21

<210> 428
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 428
ucaacuucua uaguagcagt t 21

<210> 429
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 429
ugcuacuaua gaaguugaat t 21

<210> 430
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 430
uucaacuucu auaguagcat t 21

<210> 431
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 431
ugcuacuaua gaaguugaat t 21

<210> 432
<211> 21
<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 432
uucaacuucu auaguagcat t 21

<210> 433
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 433
ugcuacuaua gaaguugaat t 21

<210> 434
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 434
uucaacuucu auaguagcat t 21

<210> 435
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 435
ugcuacuaua gaaguugaat t 21

<210> 436
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide
<400> 436

uucaacuucu auaguagcat t 21

<210> 437
<211> 21
<212> DNA
<213> Artificial Sequence

<220> .
<223> Description of Combined DNA/RNA Molecule: Synthetic oligonucleotide

<220>
<223> Description of Artificial Sequence: Synthetic oligonucleotide

<400> 437
cagaagacua cuaugauaut t 21

<210> 438
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 438
auaucauagu agucuucugt t 21

<210> 439
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 439
cagaagacua cuaugauaut t 21

<210> 440
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 440
auaucauagu agucuucugt t 21

<210> 441
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 441
aaauuuuaua uaagaaacut t 21

<210> 442
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 442
aguuucuuau auaaaauuut t 21

<210> 443
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 443
aaauuuuaua uaagaaacut t 21

<210> 444
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 444
aguuucuuau auaaaauuut t 21

<210> 445
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 445
aaauuuuaua uaagaaacut t 21

<210> 446
<211> 21
<212> DNA
<213> Artificial sequence

<220>
<223> Description of Combined DNA/RNA Molecule: Synthetic oligonucleotide

<220>
<223> Description of Artificial Sequence: Synthetic oligonucleotide

<400> 446
aguuucuuau auaaaauuut t 21

<210> 447
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 447
aaauuuuaua uaagaaacut t 21

<210> 448
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 448
aguuucuuau auaaaauuut t 21

<210> 449
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 449
auggaauagu ucagagguut t 21

<210> 450
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 450
aaccucugaa cuauuccaut t 21

<210> 451
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 451
auggaauagu ucagagguut t 21

<210> 452
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 452
aaccucugaa cuauuccaut t 21

<210> 453
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 453
cauggaauag uucagaggut t 21

<210> 454
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 454
accucugaac uauuccaugt t 21

<210> 455
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 455
cauggaauag uucagaggut t 21

<210> 456
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 456
accucugaac uauuccaugt t 21

<210> 457
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 457
cauggaauag uucagaggut t 21

<210> 458
<211> 21
<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 458
accucugaac uauuccaugt t 21

<210> 459
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 459
cauggaauag uucagaggut t 21

<210> 460
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 460
accucugaac uauuccaugt t 21

<210> 461
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 461
acauggaaua guucagaggt t 21

<210> 462
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 462
ccucugaacu auuccaugut t 21

<210> 463
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 463
acauggaaua guucagaggt t 21

<210> 464
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 464
ccucugaacu auuccaugut t 21

<210> 465
<211> 21
<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 465
gguacaugga auaguucagt t 21

<210> 466
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 466
cugaacuauu ccauguacct t 21

<210> 467
<211> 21
<212> DNA
<213> Artificial sequence

<220>
<223> Description of Combined DNA/RNA Molecule: Synthetic oligonucleotide

<220>
<223> Description of Artificial Sequence: Synthetic oligonucleotide

<400> 467
gguacaugga auaguucagt t 21

<210> 468
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 468
cugaacuauu ccauguacct t 21

<210> 469
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 469
gguacaugga auaguucagt t 21

<210> 470
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 470
cugaacuauu ccauguacct t 21

<210> 471
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 471
gguacaugga auaguucagt t 21

<210> 472
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 472
cugaacuauu ccauguacct t 21

<210> 473
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 473
ugguacaugg aauaguucat t 21

<210> 474
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 474
ugaacuauuc cauguaccat t 21

<210> 475
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 475
ugguacaugg aauaguucat t 21

<210> 476
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 476
ugaacuauuc cauguaccat t 21

<210> 477
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 477
ugguacaugg aauaguucat t 21

<210> 478
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 478
ugaacuauuc cauguaccat t 21

<210> 479
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 479
ugguacaugg aauaguucat t 21

<210> 480
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 480
ugaacuauuc cauguaccat t 21

<210> 481
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 481
cuuacuccug aaacauaugt t 21

<210> 482
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 482
cauauguuuc aggaguaagt t 21

<210> 483
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 483
cuuacuccug aaacauaugt t 21

<210> 484
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 484
cauauguuuc aggaguaagt t 21

<210> 485
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 485
auccagaaga cuacuaugat t 21

<210> 486
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 486
ucauaguagu cuucuggaut t 21

<210> 487
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 487
uuuuggaaga aacuacuugt t 21

<210> 488
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 488
caaguaguuu cuuccaaaat t 21

<210> 489
<211> 21
<212> DNA
<213> Artificial Sequence

<220>
<223> Description of combined DNA/RNA Molecule: Synthetic oligonucleotide

<220>
<223> Description of Artificial Sequence: synthetic oligonucleotide

<400> 489
uuuuggaaga aacuacuugt t 21

<210> 490
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 490
caaguaguuu cuuccaaaat t 21

<210> 491
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 491
uuggaagaaa cuacuugggt t 21

<210> 492
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 492
cccaaguagu uucuuccaat t 21

<210> 493
<211> 21
<212> DNA
<213> Artificial Sequence

<220>
<223> Description of combined DNA/RNA Molecule: Synthetic oligonudeotide

<220>
<223> Description of Artificial Sequence: Synthetic oligonucleotide

<400> 493
uuggaagaaa cuacuugggt t 21

<210> 494
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 494
cccaaguagu uucuuccaat t 21

<210> 495
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 495
augaagaccu guuuugccat t 21

<210> 496
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 496
uggcaaaaca ggucuucaut t 21

<210> 497
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 497
augaagaccu guuuugccat t 21

<210> 498
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 498
uggcaaaaca ggucuucaut t 21

<210> 499
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 499
gaugaagacc uguuuugcct t 21

<210> 500
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 500
ggcaaaacag gucuucauct t 21

<210> 501
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 501
gaugaagacc uguuuugcct t 21

<210> 502
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 502
ggcaaaacag gucuucauct t 21

<210> 503
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 503
gaugaagacc uguuuugcct t 21

<210> 504
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 504
ggcaaaacag gucuucauct t 21

<210> 505
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 505
gaugaagacc uguuuugcct t 21

<210> 506
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 506
ggcaaaacag gucuucauct t 21

<210> 507
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 507
gggaugaaga ccuguuuugt t 21

<210> 508
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 508
caaaacaggu cuucauccct t 21

<210> 509
<211> 21
<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 509
gggaugaaga ccuguuuugt t 21

<210> 510
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 510
caaaacaggu cuucauccct t 21

<210> 511
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 511
gggaugaaga ccuguuuugt t 21

<210> 512
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 512
caaaacaggu cuucauccct t 21

<210> 513
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 513
gggaugaaga ccuguuuugt t 21

<210> 514
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 514
caaaacaggu cuucauccct t 21

<210> 515
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 515
cuuauaagag gaggaguagt t 21

<210> 516
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 516
cuacuccucc ucuuauaagt t 21

<210> 517
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 517
caugcuuccu uguuacagut t 21

<210> 518
<211> 21
<212> DNA
<213> Artificial sequence

<220>
<223> Description of combined DNA/RNA Molecule: Synthetic oligonucleotide

<220>
<223> Description of Artificial Sequence: Synthetic oligonucleotide

<400> 518
acuguaacaa ggaagcaugt t 21

<210> 519
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 519
uacgggacug uaacaccugt t 21

<210> 520
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 520
cagguguuac agucccguat t 21

<210> 521
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 521
ugcuuccuug uuacagugut t 21

<210> 522
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 522
acacuguaac aaggaagcat t 21

<210> 523
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 523
ccuguugaau guuggguuct t 21

<210> 524
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 524
gaacccaaca uucaacaggt t 21

<210> 525
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 525
guugaauguu ggguuccugt t 21

<210> 526
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 526
caggaaccca acauucaact t 21

<210> 527
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 527
aauguugggu uccugaucct t 21

<210> 528
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 528
ggaucaggaa cccaacauut t 21

<210> 529
<211> 21
<212> DNA
<213> Artificial Sequence

<220>
<223> Description of combined DNA/RNA Molecule: Synthetic oligonucleotide

<220>
<223> Description of Artificial sequence: Synthetic oligonucleotide

<400> 529
acacuaacag gaggagaaat t 21

<210> 530
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 530
uuucuccucc uguuagugut t 21

<210> 531
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 531
uauaagagga ggaguagaat t 21

<210> 532
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 532
uucuacuccu ccucuuauat t 21

<210> 533
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 533
auaagaggag gaguagaagt t 21

<210> 534
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 534
cuucuacucc uccucuuaut t 21

<210> 535
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 535
acuguaacac cugcucuugt t 21

<210> 536
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 536
caagagcagg uguuacagut t 21

<210> 537
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 537
ggacaugcuu ccuuguuact t 21

<210> 538
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 538
guaacaagga agcaugucct t 21

<210> 539
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 539
acaugcuucc uuguuacagt t 21

<210> 540
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 540
cuguaacaag gaagcaugut t 21

<210> 541
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 541
augcuuccuu guuacagugt t 21

<210> 542
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 542
cacuguaaca aggaagcaut t 21

<210> 543
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 543
cuguugaaug uuggguucct t 21

<210> 544
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 544
ggaacccaac auucaacagt t 21

<210> 545
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 545
gaauguuggg uuccugauct t 21

<210> 546
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 546
gaucaggaac ccaacauuct t 21

<210> 547
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 547
cgggacugua acaccugcut t 21

<210> 548
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 548
agcagguguu acagucccgt t 21

<210> 549
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 549
ggacuguaac accugcucut t 21

<210> 550
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 550
agagcaggug uuacagucct t 21

<210> 551
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 551
gacuguaaca ccugcucuut t 21

<210> 552
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 552
aagagcaggu guuacaguct t 21

<210> 553
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 553
cuccagaaau gggugaccct t 21

<210> 554
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 554
gggucaccca uuucuggagt t 21

<210> 555
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 555
uaagaggagg aguagaagut t 21

<210> 556
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 556
acuucuacuc cuccucuuat t 21

<210> 557
<211> 21
<212> DNA
<213> Artificial Sequence

<220>
<223> Description of Combined DNA/RNA Molecule: Synthetic oligonucleotide

<220>
<223> Description of Artificial Sequence: synthetic oligonucleotide

<400> 557
gaggcugcug cuacuauagt t 21

<210> 558
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 558
cuauaguagc agcagccuct t 21

<210> 559
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 559
auugcauccc uugcuacugt t 21

<210> 560
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 560
caguagcaag ggaugcaaut t 21

<210> 561
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 561
ugcaucccuu gcuacuguat t 21

<210> 562
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 562
uacaguagca agggaugcat t 21

<210> 563
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 563
uuguguuuuc agguucaugt t 21

<210> 564
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 564
caugaaccug aaaacacaat t 21

<210> 565
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 565
ggaccuaguu gcuacuguut t 21

<210> 566
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 566
aacaguagca acuaggucct t 21

<210> 567
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 567
cugccacagg auuuucagut t 21

<210> 568
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 568
acugaaaauc cuguggcagt t 21

<210> 569
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 569
gcuacuauag aaguugaaat t 21

<210> 570
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 570
uuucaacuuc uauaguagct t 21

<210> 571
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 571
aauugcaucc cuugcuacut t 21

<210> 572
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 572
aguagcaagg gaugcaauut t 21

<210> 573
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 573
accuaguugc uacuguuuct t 21

<210> 574
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 574
gaaacaguag caacuaggut t 21

<210> 575
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 575
cuacuauaga aguugaaaut t 21

<210> 576
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 576
auuucaacuu cuauaguagt t 21

<210> 577
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 577
aggccuuacu ccugaaacat t 21

<210> 578
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 578
uguuucagga guaaggccut t 21

<210> 579
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 579
ggccuuacuc cugaaacaut t 21

<210> 580
<211> 21
<212> DNA
<213> Artificial Sequence

<220>
<223> Description of Combined DNA/RNA Molecule: Synthetic oligonucleotide

<220>
<223> Description of Artificial Sequence: synthetic oligonucleotide

<400> 580
auguuucagg aguaaggcct t 21

<210> 581
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 581
guaaaaccug gaguggaact t 21

<210> 582
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 582
guuccacucc agguuuuact t 21

<210> 583
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 583
uguguuuuca gguucauggt t 21

<210> 584
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 584
ccaugaaccu gaaaacacat t 21

<210> 585
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 585
uguuuucagg uucaugggut t 21

<210> 586
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 586
acccaugaac cugaaaacat t 21

<210> 587
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 587
aucccuugcu acuguagagt t 21

<210> 588
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 588
cucuacagua gcaagggaut t 21

<210> 589
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 589
gaccuaguug cuacuguuut t 21

<210> 590
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 590
aaacaguagc aacuagguct t 21

<210> 591
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 591
ggauuacaag uaccucugat t 21

<210> 592
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 592
ucagagguac uuguaaucct t 21

<210> 593
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 593
uaggccuuac uccugaaact t 21

<210> 594
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 594
guuucaggag uaaggccuat t 21

<210> 595
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 595
uguuagaauu uuugcuggat t 21

<210> 596
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 596
uccagcaaaa auucuaacat t 21

<210> 597
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 597
ugcugccaca ggauuuucat t 21

<210> 598
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 598
ugaaaauccu guggcagcat t 21

<210> 599
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 599
ccacaggauu uucaguagct t 21

<210> 600
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 600
gcuacugaaa auccuguggt t 21

<210> 601
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 601
aaguugaaau ugcaucccut t 21

<210> 602
<211> 21
<212> DNA
<213> Artificial sequence

<220>
<223> Description of Combined DNA/RNA Molecule: Synthetic oligonucleotide

<220>
<223> Description of Artificial Sequence: Synthetic oligonucleotide

<400> 602
agggaugcaa uuucaacuut t 21

<210> 603
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 603
aguugaaauu gcaucccuut t 21

<210> 604
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 604
aagggaugca auuucaacut t 21

<210> 605
<211> 21
<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 605
gcugcugcca caggauuuut t 21

<210> 606
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 606
aaaauccugu ggcagcagct t 21

<210> 607
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 607
aguaaaaccu ggaguggaat t 21

<210> 608
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 608
uuccacucca gguuuuacut t 21

<210> 609
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 609
uuuuguguuu ucagguucat t 21

<210> 610
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 610
ugaaccugaa aacacaaaat t 21

<210> 611
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 611
uuuguguuuu cagguucaut t 21

<210> 612
<211> 21
<212> DNA

<213> Artificial Sequence

<220>
<223> Description of Combined DNA/RNA Molecule: Synthetic oligonucleotide

<220>
<223> Description of Artificial Sequence: Synthetic oligonucleotide

<400> 612
augaaccuga aaacacaaat t 21

<210> 613
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 613
guguuuucag guucaugggt t 21

<210> 614
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 614
cccaugaacc ugaaaacact t 21

<210> 615
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 615
guuuucaggu ucaugggugt t 21

<210> 616
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 616
cacccaugaa ccugaaaact t 21

<210> 617
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 617
uuuucagguu caugggugct t 21

<210> 618
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 618
gcacccauga accugaaaat t 21

<210> 619
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 619
uuucagguuc augggugcct t 21

<210> 620
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 620
ggcacccaug aaccugaaat t 21

<210> 621
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 621
aauugcugcu ggagaggcut t 21

<210> 622
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 622
agccucucca gcagcaauut t 21

<210> 623
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 623
uugcaucccu ugcuacugut t 21

<210> 624
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 624
acaguagcaa gggaugcaat t 21

<210> 625
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 625
gcuguagcug gguuugcugt t 21

<210> 626
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 626
cagcaaaccc agcuacagct t 21

<210> 627
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 627
uagaaguuga aauugcauct t 21

<210> 628
<211> 21
<212> DNA
<213> Artificial sequence

<220>
<223> Description of Combined DNA/RNA Molecule: Synthetic oligonucleotide

<220>
<223> Description of Artificial Sequence: Synthetic oligonucleotide

<400> 628
gaugcaauuu caacuucuat t 21

<210> 629
<211> 21
<212> DNA
<213> Artificial Sequence

<220>
<223> Description of Combined DNA/RNA Molecule: Synthetic oligonucleotide

<220>
<223> Description of Artificial Sequence: synthetic oligonucleotide

<400> 629
gaaguugaaa uugcauccct t 21

<210> 630
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 630
gggaugcaau uucaacuuct t 21

<210> 631
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 631
caucccuugc uacuguagat t 21

<210> 632
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 632
ucuacaguag caagggaugt t 21

<210> 633
<211> 21

<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 633
uaguaaaacc uggaguggat t 21

<210> 634
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 634
uccacuccag guuuuacuat t 21

<210> 635
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 635
uuuuuuguua gaauuuuugt t 21

<210> 636
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 636
caaaaauucu aacaaaaaat t 21

<210> 637
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 637
uacuauagaa guugaaauut t 21

<210> 638
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 638
aauuucaacu ucuauaguat t 21

<210> 639
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 639
ccuaguugcu acuguuucut t 21

<210> 640
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 640
agaaacagua gcaacuaggt t 21

<210> 641
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 641
cuaguugcua cuguuucugt t 21

<210> 642
<211> 21
<212> DNA
<213> Artificial Sequence

<220>
<223> Description of Combined DNA/RNA Molecule: Synthetic oligonucleotide

<220>
<223> Description of Artificial Sequence: synthetic oligonucleotide

<400> 642
cagaaacagu agcaacuagt t 21

<210> 643
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 643
guugcuacug uuucugaggt t 21

<210> 644
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 644
ccucagaaac aguagcaact t 21

<210> 645
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 645
uggagaggcu gcugcuacut t 21

<210> 646
<211> 21
<212> DNA
<213> Artificial Sequence

<220>
<223> Description of combined DNA/RNA Molecule: Synthetic oligonucleotide

<220>
<223> Description of Artificial sequence: Synthetic oligonucleotide

<400> 646
aguagcagca gccucuccat t 21

<210> 647
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 647
gagaggcugc ugcuacuaut t 21

<210> 648
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 648
auaguagcag cagccucuct t 21

<210> 649
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 649
agaggcugcu gcuacuauat t 21

<210> 650
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 650
uauaguagca gcagccucut t 21

<210> 651
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 651
gcugcugcua cuauagaagt t 21

<210> 652
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 652
cuucuauagu agcagcagct t 21

<210> 653
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 653
uuuuuugugu uuucagguut t 21

<210> 654
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 654
aaccugaaaa cacaaaaaat t 21

<210> 655
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 655
acuauagaag uugaaauugt t 21

<210> 656
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 656
caauuucaac uucuauagut t 21

<210> 657
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 657
uuaguaaaac cuggaguggt t 21

<210> 658
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 658
ccacuccagg uuuuacuaat t 21

<210> 659
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 659
gccuuacucc ugaaacauat t 21

<210> 660
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 660
uauguuucag gaguaaggct t 21

<210> 661
<211> 21
<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 661
agaaguugaa auugcaucct t 21

<210> 662
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 662
ggaugcaauu ucaacuucut t 21

<210> 663
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 663
ugccacagga uuuucaguat t 21

<210> 664
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 664
uacugaaaau ccuguggcat t 21

<210> 665
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 665
gcugccacag gauuuucagt t 21

<210> 666
<211> 21
<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 666
cugaaaaucc uguggcagct t 21

<210> 667
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 667
cagguucaug ggugccgcat t 21

<210> 668
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 668
ugcggcaccc augaaccugt t 21

<210> 669
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 669
aaauugcugc uggagaggct t 21

<210> 670
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 670
gccucuccag cagcaauuut t 21

<210> 671
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 671
auugcugcug gagaggcugt t 21

<210> 672
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 672
cagccucucc agcagcaaut t 21

<210> 673
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 673
ugaaauugca ucccuugcut t 21

<210> 674
<211> 21
<212> DNA
<213> Artificial Sequence

<220>
<223> Description of Combined DNA/RNA Molecule: Synthetic oligonucleotide

<220>
<223> Description of Artificial Sequence: synthetic oligonucleotide

<400> 674
agcaagggau gcaauuucat t 21

<210> 675
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 675
uuuuguuaga auuuuugcut t 21

<210> 676
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 676
agcaaaaauu cuaacaaaat t 21

<210> 677
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 677
uuuguuagaa uuuuugcugt t 21

<210> 678
<211> 21
<212> DNA
<213> Artificial Sequence
(-<220>

<223> Description of Combined DNA/RNA Molecule: Synthetic oligonucleotide

<220>
<223> Description of Artificial Sequence: Synthetic oligonucleotide

<400> 678
cagcaaaaau ucuaacaaat t 21

<210> 679
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 679
ggcugcugcu acuauagaat t 21

<210> 680
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 680
uucuauagua gcagcagcct t 21

<210> 681
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 681
uuguuagaau uuuugcuggt t 21

<210> 682
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 682
ccagcaaaaa uucuaacaat t 21

<210> 683
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 683
uuuuuuugug uuuucaggut t 21

<210> 684
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 684
accugaaaac acaaaaaaat t 21

<210> 685
<211> 21
<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 685
gaaacuacuu gggcaauagt t 21

<210> 686
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 686
cuauugccca aguaguuuct t 21

<210> 687
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 687
aauggauguu gccuuuacut t 21

<210> 688
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 688
aguaaaggca acauccauut t 21

<210> 689
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 689
ccucaaugga uguugccuut t 21

<210> 690
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 690
aaggcaacau ccauugaggt t 21

<210> 691
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 691
uugccuuuac uuuuagggut t 21

<210> 692
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 692
acccuaaaag uaaaggcaat t 21

<210> 693
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 693
agaaacuacu ugggcaauat t 21

<210> 694
<211> 21
<212> DNA
<213> Artificial Sequence

<220>
<223> Description of Combined DNA/RNA Molecule: Synthetic oligonucleotide

<220>
<223> Description of Artificial sequence: synthetic oligonucleotide

<400> 694
uauugcccaa guaguuucut t 21

<210> 695
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 695
aagaaacuac uugggcaaut t 21

<210> 696
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 696
auugcccaag uaguuucuut t 21

<210> 697
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 697
ugccuuuacu uuuaggguut t 21

<210> 698
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 698
aacccuaaaa guaaaggcat t 21

<210> 699
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 699
gccuuuacuu uuaggguugt t 21

<210> 700
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 700
caacccuaaa aguaaaggct t 21

<210> 701
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 701
cuuuacuuuu aggguuguat t 21

<210> 702
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 702
uacaacccua aaaguaaagt t 21

<210> 703
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 703
uuuacuuuua ggguuguact t 21

<210> 704
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 704
guacaacccu aaaaguaaat t 21

<210> 705
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 705
uacuuuuagg guuguacggt t 21

<210> 706
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 706
ccguacaacc cuaaaaguat t 21

<210> 707
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 707
ggaagaaacu acuugggcat t 21

<210> 708
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 708
ugcccaagua guuucuucct t 21

<210> 709
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 709
caauggaugu ugccuuuact t 21

<210> 710
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 710
guaaaggcaa cauccauugt t 21

<210> 711
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 711
ggauguugcc uuuacuuuut t 21

<210> 712
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 712
aaaaguaaag gcaacaucct t 21

<210> 713
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 713
uguugccuuu acuuuuaggt t 21

<210> 714
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 714
ccuaaaagua aaggcaacat t 21

<210> 715
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 715
gauguugccu uuacuuuuat t 21

<210> 716
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 716
uaaaaguaaa ggcaacauct t 21

<210> 717
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 717
ccagaagacu acuaugauat t 21

<210> 718
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 718
uaucauagua gucuucuggt t 21

<210> 719
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 719
uccagaagac uacuaugaut t 21

<210> 720
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 720
aucauaguag ucuucuggat t 21

<210> 721
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 721
uuuggaagaa acuacuuggt t 21

<210> 722
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 722
ccaaguaguu ucuuccaaat t 21

<210> 723
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 723
cuccucaaug gauguugcct t 21

<210> 724
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 724
ggcaacaucc auugaggagt t 21

<210> 725
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 725
ccaaaugugc aaucuggugt t 21

<210> 726
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 726
caccagauug cacauuuggt t 21

<210> 727
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 727
caaaugugca aucuggugat t 21

<210> 728
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 728
ucaccagauu gcacauuugt t 21

<210> 729
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 729
agaucugcuc cucaauggat t 21

<210> 730
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 730
uccauugagg agcagaucut t 21

<210> 731
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 731
cucaauggau guugccuuut t 21

<210> 732
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 732
aaaggcaaca uccauugagt t 21

<210> 733
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 733
gcucaaauuu uauauaagat t 21

<210> 734
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 734
ucuuauauaa aauuugagct t 21

<210> 735
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 735
agccugauuu ugguacaugt t 21

<210> 736
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 736
cauguaccaa aaucaggcut t 21

<210> 737
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 737
cucaaauuuu auauaagaat t 21

<210> 738
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 738
uucuuauaua aaauuugagt t 21

<210> 739
<211> 21
<212> DNA
<213> Artificial Sequence

<220>
<223> Description of Combined DNA/RNA Molecule: Synthetic oligonucleotide

<220>
<223> Description of Artificial Sequence: synthetic oligonucleotide

<400> 739
acaaagugcu gaauagggat t 21

<210> 740
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 740
ucccuauuca gcacuuugut t 21

<210> 741
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 741
cugauuuugg uacauggaat t 21

<210> 742
<211> 21
<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 742
uuccauguac caaaaucagt t 21

<210> 743
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 743
gauuuuggua cauggaauat t 21

<210> 744
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 744
uauuccaugu accaaaauct t 21

<210> 745
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 745
cucaucagcc ugauuuuggt t 21

<210> 746
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 746
ccaaaaucag gcugaugagt t 21

<210> 747
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 747
agcccacuug uguggauagt t 21

<210> 748
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 748
cuauccacac aagugggcut t 21

<210> 749
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 749
gugcugaaua gggaggaaut t 21

<210> 750
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 750
auuccucccu auucagcact t 21

<210> 751
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 751
aguaagggcg uggaggcuut t 21 <210> 752
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 752
aagccuccac gcccuuacut t 21

<210> 753
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 753
gugacuuaac ccaagaagct t 21

<210> 754
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 754
gcuucuuggg uuaagucact t 21

<210> 755

<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 755
uaguaagggc guggaggcut t 21

<210> 756
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 756
agccuccacg cccuuacuat t 21

<210> 757
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 757
ucaucagccu gauuuuggut t 21

<210> 758
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 758
accaaaauca ggcugaugat t 21

<210> 759
<211> 21
<212> DNA

<213> Artificial Sequence

<220>
<223> Description of combined DNA/RNA Molecule: Synthetic oligonucleotide

<220>
<223> Description of Artificial Sequence: Synthetic oligonucleotide

<400> 759
guagaagacc cuaaagacut t 21

<210> 760
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 760
agucuuuagg gucuucuact t 21

<210> 761
<211> 21
<212> DNA
<213> Artificial Sequence

<220>
<223> Description of combined DNA/RNA Molecule: Synthetic oligonucleotide

<220>
<223> Description of Artificial Sequence: synthetic oligonucleotide

<400> 761
agguagaaga cccuaaagat t 21

<210> 762
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 762
ucuuuagggu cuucuaccut t 21

<210> 763
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 763
aaaagguaga agacccuaat t 21

<210> 764
<211> 21
<212> DNA
<213> Artificial Sequence

<220>
<223> Description of Combined DNA/RNA Molecule: Synthetic oligonucleotide

<220>
<223> Description of Artificial Sequence: synthetic oligonucleotide

<400> 764
uuagggucuu cuaccuuuut t 21

<210> 765
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 765
aaagguagaa gacccuaaat t 21

<210> 766
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 766
uuuagggucu ucuaccuuut t 21

<210> 767
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 767
gauugugcag uggaaagaat t 21

<210> 768
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 768
uucuuuccac ugcacaauct t 21

<210> 769
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 769
ggauugugca guggaaagat t 21

<210> 770
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 770
ucuuuccacu gcacaaucct t 21

<210> 771
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 771
uguagacagc cauaugcagt t 21

<210> 772
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 772
cugcauaugg cugucuacat t 21

<210> 773
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 773
caugacuuua acccagaagt t 21

<210> 774
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 774
cuucuggguu aaagucaugt t 21

<210> 775
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 775
auagggagga auccauggat t 21

<210> 776
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 776
uccauggauu ccucccuaut t 21

<210> 777
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 777
cugaauaggg aggaauccat t 21

<210> 778
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 778
uggauuccuc ccuauucagt t 21

<210> 779
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 779
aaagugcuga auagggaggt t 21

<210> 780
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 780
ccucccuauu cagcacuuut t 21

<210> 781
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 781
ugacuuaacc caagaagcut t 21

<210> 782
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 782
agcuucuugg guuaagucat t 21

<210> 783
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 783
augacuuuaa cccagaagat t 21

<210> 784
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 784
ucuucugggu uaaagucaut t 21

<210> 785
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 785
gaagacccua aagacuuuct t 21

<210> 786
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 786
gaaagucuuu agggucuuct t 21

<210> 787
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 787
aaaaagcuca aauuuuauat t 21

<210> 788
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 788
uauaaaauuu gagcuuuuut t 21

<210> 789
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 789
aucagccuga uuuugguact t 21

<210> 790
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 790
guaccaaaau caggcugaut t 21

<210> 791
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 791
caucagccug auuuugguat t 21

<210> 792
<211> 21
<212> DNA
<213> Artificial Sequence

<220>
<223> Description of combined DNA/RNA molecule: Synthetic oligonucleotide

<220>
<223> Description of Artificial Sequence: Synthetic oligonucleotide

<400> 792
uaccaaaauc aggcugaugt t 21

<210> 793
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 793
auggacaaag ugcugaauat t 21

<210> 794
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 794
uauucagcac uuuguccaut t 21

<210> 795
<211> 21
<212> DNA
<213> Artificial Sequence

<220>
<223> Description of Combined DNA/RNA Molecule: Synthetic oligonucleotide

<220> .
<223> Description of Artificial Sequence: Synthetic oligonucleotide

<400> 795
uagaagaccc uaaagacuut t 21

<210> 796
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 796
aagucuuuag ggucuucuat t 21

<210> 797
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 797
aagguagaag acccuaaagt t 21

<210> 798
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 798
cuuuaggguc uucuaccuut t 21

<210> 799
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 799
cagcccacuu guguggauat t 21

<210> 800
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 800
uauccacaca agugggcugt t 21

<210> 801
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 801
uuuugguaca uggaauagut t 21

<210> 802
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 802
acuauuccau guaccaaaat t 21

<210> 803
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 803
ugaauaggga ggaauccaut t 21

<210> 804
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 804
auggauuccu cccuauucat t 21

<210> 805
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 805
gcugaauagg gaggaaucct t 21

<210> 806
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 806
ggauuccucc cuauucagct t 21

<210> 807
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 807
ugcugaauag ggaggaauct t 21

<210> 808
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 808
gauuccuccc uauucagcat t 21

<210> 809
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 809
uguguagaca gccauaugct t 21

<210> 810
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 810
gcauauggcu gucuacacat t 21

<210> 811
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 811
ugcuuugauu gcuucagact t 21

<210> 812
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 812
gucugaagca aucaaagcat t 21

<210> 813
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 813
uugcuuugau ugcuucagat t 21

<210> 814
<211> 21
<212> DNA
<213> Artificial sequence

<220>
<223> Description of combined DNA/RNA Molecule: Synthetic oligonucleotide

<220>
<223> Description of Artificial Sequence: Synthetic oligonucleotide

<400> 814
ucugaagcaa ucaaagcaat t 21

<210> 815
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 815
auugcuuuga uugcuucagt t 21

<210> 816
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 816
cugaagcaau caaagcaaut t 21

<210> 817
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 817
cuauugcuuu gauugcuuct t 21

<210> 818
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 818
gaagcaauca aagcaauagt t 21

<210> 819
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 819
auugcaagga auggccuaat t 21

<210> 820
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 820
uuaggccauu ccuugcaaut t 21

<210> 821
<211> 21
<212> DNA
<213> Artificial Sequence

<220>
<223> Description of Combined DNA/RNA-molecule: Synthetic oligonucleotide

<220>
<223> Description of Artificial Sequence: Synthetic oligonucleotide

<400> 821
auuuuccucc uaauucugat t 21

<210> 822
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 822
ucagaauuag gaggaaaaut t 21

<210> 823
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 823
gcucaucagc cugauuuugt t 21

<210> 824
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 824
caaaaucagg cugaugagct t 21

<210> 825
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 825
ugcucaucag ccugauuuut t 21

<210> 826
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 826
aaaaucaggc ugaugagcat t 21

<210> 827
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 827
aguugcucau cagccugaut t 21

<210> 828
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 828
aucaggcuga ugagcaacut t 21

<210> 829
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 829
aagggcgugg aggcuuuuut t 21

<210> 830
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 830
aaaaagccuc cacgcccuut t 21

<210> 831
<211> 21
<212> DNA
<213> Artificial sequence

<220>
<223> Description of Combined DNA/RNA Molecule: Synthetic oligonucleotide

<220>
<223> Description of Artificial Sequence: Synthetic oligonucleotide

<400> 831
guaagggcgu ggaggcuuut t 21

<210> 832
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 832
aaagccucca cgcccuuact t 21

<210> 833
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 833
gacccuaaag acuuuccugt t 21

<210> 834
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 834
caggaaaguc uuuaggguct t 21

<210> 835
<211> 21
<212> DNA
<213> Artificial Sequence

<220>
<223> Description of Combined DNA/RNA Molecule: Synthetic oligonucleotide

<220>
<223>.Description of Artificial Sequence: Synthetic oligonucleotide

<400> 835
aggagcauga cuuuaaccct t 21

<210> 836
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 836
ggguuaaagu caugcuccut t 21

<210> 837
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 837
ugugauuuuc cuccuaauut t 21

<210> 838
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 838
aauuaggagg aaaaucacat t 21

<210> 839
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 839
uugugauuuu ccuccuaaut t 21

<210> 840
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 840
auuaggagga aaaucacaat t 21

<210> 841
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 841
gacuuaaccc aagaagcuct t 21

<210> 842
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 842
gagcuucuug gguuaaguct t 21

<210> 843
<211> 21

<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 843
gcuucagaca augguuuggt t 21

<210> 844
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 844
ccaaaccauu gucugaagct t 21

<210> 845
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 845
uugcuucaga caaugguuut t 21

<210> 846
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 846
aaaccauugu cugaagcaat t 21

<210> 847
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 847
auugcuucag acaaugguut t 21

<210> 848
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 848
aaccauuguc ugaagcaaut t 21

<210> 849
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 849
uugauugcuu cagacaaugt t 21

<210> 850
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 850
cauugucuga agcaaucaat t 21

<210> 851
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 851
gacaaagugc ugaauagggt t 21

<210> 852
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 852
cccuauucag cacuuuguct t 21

<210> 853
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 853
aagaaaaagc ucaaauuuut t 21

<210> 854
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 854
aaaauuugag cuuuuucuut t 21

<210> 855
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 855
aaagaaaaag cucaaauuut t 21

<210> 856
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 856
aaauuugagc uuuuucuuut t 21

<210> 857
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 857
agaagacccu aaagacuuut t 21

<210> 858
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 858
aaagucuuua gggucuucut t 21

<210> 859
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 859
aaaaaaaagg uagaagacct t 21

<210> 860
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 860
ggucuucuac cuuuuuuuut t 21

<210> 861
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 861
gguagaagac ccuaaagact t 21

<210> 862
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 862
gucuuuaggg ucuucuacct t 21

<210> 863
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 863
aaaaaaggua gaagacccut t 21

<210> 864
<211> 21

<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 864
agggucuucu accuuuuuut t 21

<210> 865
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 865
aaaaagguag aagacccuat t 21

<210> 866
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 866
uagggucuuc uaccuuuuut t 21

<210> 867
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 867
uuaacccaag aagcucuuct t 21

<210> 868
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 868
gaagagcuuc uuggguuaat t 21

<210> 869
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 869
auuuugguac auggaauagt t 21

<210> 870
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 870
cuauuccaug uaccaaaaut t 21

<210> 871
<211> 21
<212> DNA
<213> Artificial Sequence

<220>
<223> Description of combined DNA/RNA Molecule: synthetic oligonucleotide

<220>
<223> Description of Artificial Sequence: Synthetic oligonucleotide

<400> 871
agugcugaau agggaggaat t 21

<210> 872
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 872
uuccucccua uucagcacut t 21

<210> 873
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 873
gaggccaggg aaauucccut t 21

<210> 874
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 874
agggaauuuc ccuggccuct t 21

<210> 875
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 875
agcucaaauu uuauauaagt t 21

<210> 876
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 876
cuuauauaaa auuugagcut t 21

<210> 877
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 877
cagggaaauu cccuuguuut t 21

<210> 878
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 878
aaacaaggga auuucccugt t 21

<210> 879
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 879
uugugcagug gaaagaaagt t 21

<210> 880
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 880
cuuucuuucc acugcacaat t 21

<210> 881
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 881
uacauggaau aguucagagt t 21

<210> 882
<211> 21
<212> DNA
<213> Artificial Sequence

<220>
<223> Description of combined DNA/RNA Molecule: Synthetic-oligonucleotide

<220>
<223> Description of Artificial Sequence: Synthetic oligonucleotide

<400> 882
cucugaacua uuccauguat t 21

<210> 883
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 883
guacauggaa uaguucagat t 21

<210> 884
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 884
ucugaacuau uccauguact t 21

<210> 885
<211> 21

<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 885
agggaaauuc ccuuguuuut t 21

<210> 886
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 886
aaaacaaggg aauuucccut t 21

<210> 887
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 887
ggaggccagg gaaauuccct t 21

<210> 888
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 888
gggaauuucc cuggccucct t 21

<210> 889
<211> 21
<212> DNA
<213> Artificial Sequence

<220>
<223> Description of Combined DNA/RNA Molecule: Synthetic oligonucleotide

<220>
<223> Description of Artificial Sequence: synthetic oligonucleotide

<400> 889
guguagacag ccauaugcat t 21

<210> 890
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 890
ugcauauggc ugucuacact t 21

<210> 891
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 891
guagacagcc auaugcagut t 21

<210> 892
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 892
acugcauaug gcugucuact t 21

<210> 893
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 893
gaagaccugu uuugccaugt t 21

<210> 894
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 894
cauggcaaaa caggucuuct t 21

<210> 895
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 895
agugggauga agaccuguut t 21

<210> 896
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 896
aacaggucuu caucccacut t 21

<210> 897
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 897
ugaagaccug uuuugccaut t 21

<210> 898
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 898
auggcaaaac aggucuucat t 21

<210> 899
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 899
ugggaugaag accuguuuut t 21

<210> 900
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 900
aaaacagguc uucaucccat t 21

<210> 901
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 901
cuuaacccaa gaagcucuut t 21

<210> 902
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 902
aagagcuucu uggguuaagt t 21

<210> 903
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 903
auugugcagu ggaaagaaat t 21

<210> 904
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 904
uuucuuucca cugcacaaut t 21

<210> 905
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 905
cuuuauugca aggaauggct t 21

<210> 906
<211> 21

<212> DNA
<213> Artificial Sequence

<220>
<223> Description of Combined DNA/RNA Molecule: Synthetic oligonucleotide

<220>
<223> Description of Artificial sequence: Synthetic oligonucleotide

<400> 906
gccauuccuu gcaauaaagt t 21

<210> 907
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 907
gagcaugacu uuaacccagt t 21

<210> 908
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 908
cuggguuaaa gucaugcuct t 21

<210> 909
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 909
gugauuuucc uccuaauuct t 21

<210> 910
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 910
gaauuaggag gaaaaucact t 21

<210> 911
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 911
ugauugcuuc agacaauggt t 21

<210> 912
<211> 21
<212> DNA
<213> Artificial Sequence

<220>
<223> Description of Combined DNA/RNA Molecule: synthetic oligonucleotide

<220>
<223> Description of Artificial Sequence: Synthetic oligonucleotide

<400> 912
ccauugucug aagcaaucat t 21

<210> 913
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 913
aagcucaaau uuuauauaat t 21

<210> 914
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 914
uuauauaaaa uuugagcuut t 21

<210> 915
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 915
cuggacaugg aucaagcact t 21

<210> 916
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 916
gugcuugauc cauguccagt t 21

<210> 917
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 917
ucaaauuuua uauaagaaat t 21

<210> 918
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 918
uuucuuauau aaaauuugat t 21

<210> 919
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 919
ugugcagugg aaagaaaggt t 21

<210> 920
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 920
ccuuucuuuc cacugcacat t 21

<210> 921
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 921
uugguacaug gaauaguuct t 21

<210> 922
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 922

gaacuauucc auguaccaat t 21

<210> 923
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 923
aagugggaug aagaccugut t 21

<210> 924
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 924
acaggucuuc aucccacuut t 21

<210> 925
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 925
ggaugaagac cuguuuugct t 21

<210> 926
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 926
gcaaaacagg ucuucaucct t 21

<210> 927
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 927
ucuggacaug gaucaagcat t 21

<210> 928
<211> 21
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 928
ugcuugaucc auguccagat t 21

<210> 929
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 929
uuugguacau ggaauaguut t 21

<210> 930
<211> 21
<212> DNA
<213> Artificial Sequence

<220>
<223> Description of combined DNA/RNA Molecule: Synthetic oligonucleotide

<220>
<223> Description of Artificial Sequence: Synthetic oligonucleotide

<400> 930
aacuauucca uguaccaaat t 21

<210> 931
<211> 1202

<212> DNA
<213> Artificial Sequence

<220>
<223> Description of Artificial Sequence: Synthetic polynucleotide

<400> 931

```
ctcgagactt ttagggttgt acgggactgt aacacctgct cttgaagcat atgaagatgg     60
ccccaacaaa aagaaaagga gaaaggaagg accccgtgca agttccaaaa cttcttataa    120
gaggaggagt agaagttcta gaagttaaaa ctggggttga ctcaattaca gaggtagaat    180
gctttttaac tccagaaatg ggtgacccag atgagcatct taggggtttt agtaagtcaa    240
tttctatatc agatacattt gaaagtgact ccccaaataa ggacatgctt ccttgttaca    300
gtgtggccag aattccacta cccaatctaa atgaggatct aacctgtgga aatatactaa    360
tgtgggaggc tgtgacctta aaaactgagg ttctaggggt gacaactttg atgaatgtgc    420
actctaatgg tcaagcaact catgacaatg gtgcaggaaa gccagtgcag ggcaccagct    480
ttcatttttt ttctgttggc ggggaggctt tagaattaca gggggtggtt tttaattaca    540
gaacaaagta cccagatgga acaatttttc caaagaatgc aacagtgcaa tctcaagtaa    600
tgaacacaga gcacaaggcg tacctagata agaacaaagc atatcctgtt gaatgttggg    660
ttcctgatcc caccagaaat gaaaacacaa gatattttgg gacactaaca ggaggagaaa    720
atgttcctcc agttcttcat ataacaaaca ctgccacaac agtgctgctt gatgaatttg    780
gtgttgggcc actttgcaaa ggtgacaact tgtatttgtc agctgttgat gtttgtggaa    840
tgtttactaa cagatctggt tcccagcagt ggagaggact gtccagatat tttaaggttc    900
agctcagaaa aaggagggtt aaaaacccct acccaatttc tttccttctt actgatttga    960
ttaacagaag gacccctaga gttgatgggc aacctatgta tggtatggat gctcaggtag   1020
aggaggttag agtttttgag gggacagagg aacttccagg ggacccagac atgatgagat   1080
atgttgacag atatggacag ttgcaaacaa agatgctgta atcaaaatcc tttattgtaa   1140
tatgcagtac attttaataa agtataacca gctttacttt acagttgcag tcatgcggcc   1200
gc                                                                  1202
```

<210> 932
<211> 2479
<212> DNA
<213> Artificial Sequence

<220>
<223> Description of Artificial Sequence: Synthetic polynucleotide

<400> 932

```
ctcgagccgc ctccaagctt actcagaagt agtaagggcg tggaggcttt ttaggaggcc     60
agggaaattc ccttgttttt cccttttttg cagtaatttt ttgctgcaaa aagctaaaat    120
ggacaaagtg ctgaataggg aggaatccat ggagcttatg gatttattag gccttgatag    180
gtctgcatgg gggaacattc ctgtcatgag aaaagcttat ctgaaaaaat gcaaagaact    240
ccaccctgat aaaggtgggg acgaagacaa gatgaagaga atgaattttt tatataaaaa    300
aatggaacaa ggtgtaaaag ttgctcatca gcctgatttt ggtacatgga atagttcaga    360
ggttggttgt gattttcctc ctaattctga taccctttat tgcaaggaat ggcctaactg    420
tgccactaat ccttcagtgc attgcccctg tttaatgtgc atgctaaaat taaggcatag    480
aaacagaaaa tttttaagaa gcagcccact tgtgtggata gattgctatt gctttgattg    540
cttcagacaa tggtttgggt gtgacttaac ccaagaagct cttcattgct gggagaaagt    600
```

tcttggagac accccctaca gggatctaaa gctttaagtg ccaacctatg gaacagatga 660
atgggaatcc tggtggaata catttaatga gaagtgggat gaagacctgt tttgccatga 720
agaaatgttt gccagtgatg atgaaaacac aggatcccaa cactctaccc cacctaaaaa 780
gaaaaaaaag gtagaagacc ctaaagactt tcctgtagat ctgcatgcat tcctcagtca 840
agctgtgttt agtaatagaa ctgttgcttc ttttgctgtg tataccacta aagaaaaagc 900
tcaaatttta tataagaaac ttatggaaaa atattctgta acttttataa gtagacatgg 960
ttttgggggt cataatattt tgtttttctt aacaccacat agacatagag tgtcagcaat 1020
taataactac tgtcaaaaac tatgtacctt tagtttttta atttgtaaag gtgtgaataa 1080
ggaatacttg ttttatagtg ccctgtgtag acagccatat gcagtagtgg aagaaagtat 1140
tcaggggggc cttaaggagc atgactttaa cccagaagaa ccagaagaaa ctaagcaggt 1200
ttcatggaaa ttagttacac agtatgcctt ggaaaccaag tgtgaggatg tttttttgct 1260
tatgggcatg tacttagact ttcaggaaaa cccacagcaa tgcaaaaaat gtgaaaaaaa 1320
ggatcagcca aatcacttta accatcatga aaaacactat tataatgccc aaatttttgc 1380
agatagcaaa aatcaaaaaa gcatttgcca gcaggctgtt gatactgtag cagccaaaca 1440
aagggttgac agcatccaca tgaccagaga agaaatgtta gttgaaaggt ttaatttctt 1500
gcttgataaa atggacttaa tttttggggc acatggcaat gctgttttag agcaatatat 1560
ggctggggtg gcctggattc attgcttgct gcctcaaatg gacactgtta tttatgactt 1620
tctaaaatgc attgtattaa acattccaaa aaaaaggtac tggctattca aggggccaat 1680
agacagtggc aaaactactt tagctgcagc tttacttgat ctctgtgggg gaaagtcatt 1740
aaatgttaat atgccattag aaagattaaa ctttgaatta ggagtgggta tagatcagtt 1800
tatggttgta tttgaggatg taaaaggcac tggtgcagag tcaagggatt taccttcagg 1860
gcatggcata agcaaccttg attgcttaag agattactta gatggaagtg taaaagttaa 1920
tttagagaga aaacaccaaa acaaaagaac acaggtgttt ccacctggaa ttgtaaccat 1980
gaatgaatat tcagtgccta gaactttaca ggccagattt gtaaggcaga tagattttag 2040
accaaaggcc tacctgagaa aatcactaag ctgctctgag tatttgctag aaaaaaggat 2100
tttgcaaagt ggtatgactt tgcttttgct tttaatctgg tttaggccag ttgctgactt 2160
tgcagctgcc attcatgaga ggattgtgca gtggaaagaa aggctggatt tagaaataag 2220
catgtataca ttttctacta tgaaagctaa tgttggtatg gggagaccca ttcttgactt 2280
tcctagagag gaagattctg aagcagaaga ctctggacat ggatcaagca ctgaatcaca 2340
atcacaatgc ttttcccagg tctcagaagc ctctggtgca gacacacagg aaaactgcac 2400
ttttcacatc tgtaaaggct ttcaatgttt caaaaaacca aagacccctc ccccaaaata 2460
actgcaactg tgcggccgc 2479

<210> 933
<211> 708
<212> DNA
<213> Artificial Sequence

<220>
<223> Description of Artificial Sequence: Synthetic polynucleotide

<400> 933

```
ctcgagcagc taacagccag taaacaaagc acaaggggaa gtggaaagca gccaagggaa     60
catgttttgc gagccagagc tgttttggct tgtcaccagc tggccatggt tcttcgccag    120
ctgtcacgta aggcttctgt gaaagttagt aaaacctgga gtggaactaa aaaaagagct    180
caaaggattt taattttttt gttagaattt ttgctggact tttgcacagg tgaagacagt    240
gtagacggga aaaaaagaca gagacacagt ggtttgactg agcagacata cagtgctttg    300
cctgaaccaa aagctacata ggtaagtaat gttttttttt gtgttttcag gttcatgggt    360
gccgcacttg cacttttggg ggacctagtt gctactgttt ctgaggctgc tgctgccaca    420
ggattttcag tagctgaaat tgctgctgga gaggctgctg ctactataga agttgaaatt    480
gcatcccttg ctactgtaga ggggattaca agtacctctg aggctatagc tgctataggc    540
cttactcctg aaacatatgc tgtaataact ggagctccgg gggctgtagc tgggtttgct    600
gcattggttc aaactgtaac tggtggtagt gctattgctc agttgggata tagattttt     660
gctgactggg atcataaagt ttcaacagtt gggctttttc gcggccgc                 708
```

<210> 934
<211> 738
<212> DNA
<213> Artificial Sequence

<220>
<223> Description of Artificial Sequence: Synthetic polynucleotide

<400> 934

```
ctcgagagca gccagctatg gctttacaat tatttaatcc agaagactac tatgatattt     60
tatttcctgg agtgaatgcc tttgttaaca atattcacta tttagatcct agacattggg    120
gcccgtcctt gttctccaca atctcccagg ctttttggaa tcttgttaga gatgatttgc    180
cagccttaac ctctcaggaa attcagagaa gaacccaaaa actatttgtt gaaagtttag    240
caaggttttt ggaagaaact acttgggcaa tagttaattc accagctaac ttatataatt    300
atatttcaga ctattattct agattgtctc cagttaggcc ctctatggta aggcaagttg    360
cccaaaggga gggaacctat atttcttttg gccactcata cacccaaagt atagatgatg    420
cagacagcat tcaagaagtt acccaaaggc tagatttaaa aaccccaaat gtgcaatctg    480
gtgaatttat agaaagaagt attgcaccag gaggtgcaaa tcaaagatct gctcctcaat    540
ggatgttgcc tttactttta gggttgtacg ggactgtaac acctgctctt gaagcatatg    600
aagatggccc caacaaaaag aaaaggagaa aggaaggacc ccgtgcaagt tccaaaactt    660
cttataagag gaggagtaga agttctagaa gttaaaactg gggttgactc aattacagag    720
gtagaatgct gcggccgc                                                  738
```